# EUROPEAN PATENT APPLICATION

(11) **EP 4 393 950 A1**
(43) Date of publication of application: **03.07.2024**
(21) Application number: 22860540.8
(22) Date of filing: 24.08.2022
(51) Int. Cl.: C07K 16/28, C07K 16/40, C07K 16/46, A61P 35/00

(54) **FAP/CD40 BINDING MOLECULE AND MEDICINAL USE THEREOF**

(30) Priority: 24.08.2021 CN 202110973560
(71) Applicant: Jiangsu Hengrui Pharmaceuticals Co., Ltd., Lianyungang, Jiangsu 222047 (CN); Shanghai Shengdi Pharmaceutical Co., Ltd, Shanghai 201210 (CN)
(72) Inventor: LIN, Yuan, Shanghai 201210 (CN); CHEN, Simeng, Shanghai 201210 (CN); WU, Tingting, Shanghai 201210 (CN); HU, Rongting, Shanghai 201210 (CN); LIAO, Cheng, Shanghai 201210 (CN)
(74) Representative: Potter Clarkson
(86) International application number: PCT/CN2022/114522
(87) International publication number: WO 2023/025194

(57) **Abstract**

An FAP/CD40 binding molecule and the medicinal use thereof. Specifically, provided are an FAP binding molecule, a CD40 binding molecule and an FAP/CD40 binding molecule, a method for preventing and treating diseases (such as tumors or cancers) using same, and the medicinal use thereof.

## Description

The present disclosure claims priority to the Chinese Patent Application (Application No. CN202110973560.5) filed on Aug. 24, 2021.

### TECHNICAL FIELD

The present disclosure relates to the field of biopharmaceutics, and particularly to the field of treating or interfering with diseases associated with the CD40/CD40L signaling pathway. Specifically, the present disclosure relates to a FAP/CD40-binding molecule, a pharmaceutical composition thereof, a method for treating disease, particularly tumors or cancer, using same, and related pharmaceutical use thereof.

### BACKGROUND

CD40 (TNFRSF5), a transmembrane phosphorylated glycoprotein, is a member of the tumor necrosis factor receptor superfamily (TNFRS). CD40 is expressed in many types of cells, including B cells, follicular dendritic cells (DCs), epithelial cells, monocytes, macrophages, smooth muscle cells, and tumor cells. Its ligand CD40L is mainly expressed in activated T cells, activated B cells, platelets, smooth muscle cells, etc. CD40L binding multimerizes CD40, generating downstream activation, growth and differentiation signals. CD40 signaling activates a variety of downstream signaling pathways, such as NF-xB, MAPK and STAT3 (Pype S, et al. J Biol Chem. 2000 Jun. 16; 275(24):1858693), and these pathways regulate gene expression by regulating the activation proteins c-Jun and ATF2 and the transcription factor Rel. Binding of CD40 to CD40L induces resting B cell proliferation, immunoglobulin switching and antibody secretion and plays important roles in the development of germinal centers in tissues and B cell survival. All of these are essential for humoral immune responses (Kehry M R. J Immunol 1996; 156:2345-2348). Binding of CD40L to the CD40 on DCs induces DC maturation. This is manifested in upregulated expression of the B7 family of costimulatory factors (CD80, CD86) and increased secretion of proinflammatory cytokines such as interleukin 12 (IL-12). The interaction between CD40 and CD40L provides a costimulatory signal for T cell activation and promotes DC cells' presentation of antigens to T cells.

With the clinical success of immune checkpoint inhibitor (ICI) therapies targeting CD40, CTLA-4 and PD-L1 in tumor treatment, immunotherapy has become the most anticipated research field among third-generation tumor therapies. Anti-tumor immunotherapy based on the mechanism that DC cells' antigen presentation to and activation of T cells are increased by activating CD40 has been shown to be clinically effective. However, there is systemic activation of CD40, which can lead to some target-specific toxicities such as cytokine storms, hepatotoxicity, hematologic toxicity, and venous thrombosis caused by peripheral CD40 activation. These toxicities limit the therapeutic windows of CD40 agonist antibodies. The clinical studies on Pfizer's CD40 agonists CP-870 and 893 and Medimmune's CD40L-Fc fusion protein MEDI5083 (a novel fusion protein from AstraZeneca that activates the CD40 signaling pathway, consisting of 3 CD40L molecules connected in tandem and IgG4-Fc) have been terminated. Thus, in the research and development of second-generation CD40 agonist antibodies, researchers aim to mediate CD40 activation through tumor-associated antigens (TAAs), such that CD40 is specifically activated within tumors while peripheral CD40 activation is reduced, thereby increasing the therapeutic windows of CD40 agonist antibodies.

Fibroblast activation protein (FAP) α is a tumor-associated antigen. FAP is lowly expressed in normal tissues of healthy adults and is selectively expressed in 93% of tumor tissues, with 30% being high expression, such as colon cancer, pancreatic cancer, breast cancer, gastric cancer, prostate cancer, bladder cancer, and oral squamous cell carcinoma. The present disclosure provides an anti-FAP antibody, an anti-CD40 antibody, and a bispecific antibody of the two. The bispecific antibody can mediate tumor-specific CD40 activation through FAP, have pro-maturation and activation effects on APCs (e.g., dendritic cells (DCs)), can eliminate hepatotoxicity, peripheral blood toxicity and other peripheral toxicities, and have an excellent window of administration and excellent druggability, thus providing a solution for clinical applications.

### SUMMARY

The present disclosure provides a CD40-binding molecule, a FAP-binding molecule and a FAP/CD40-binding molecule (e.g., an anti-FAP/CD40 bispecific antibody) with new structures; coding nucleic acids, vectors, host cells and pharmaceutical compositions thereof; a method for treating, ameliorating or preventing cell proliferative diseases (e.g., tumors or cancer) using same; and related pharmaceutical use thereof.

### CD40-Binding Molecule

The present disclosure provides a CD40-binding molecule, comprising at least one immunoglobulin single variable domain, and the immunoglobulin single variable domain comprises three complementarity-determining regions CDR1, CDR2, CDR3, wherein:
1) the CDR1 in the immunoglobulin single variable domain comprises or is the amino acid sequence set forth in SEQ ID NO: 39, and/or the CDR2 comprises or is the amino acid sequence set forth in SEQ ID NO: 40, and/or the CDR3 comprises or is the amino acid sequence set forth in SEQ ID NO: 41;
2) the amino acid sequences of the CDR1, CDR2 and CDR3 in the immunoglobulin single variable domain are set forth in SEQ ID NOs: 39, 40 and 41, respectively;
3) the amino acid sequences of the CDR1, CDR2 and CDR3 in the immunoglobulin single variable domain are set forth in SEQ ID NOs: 24, 25 and 26 or SEQ ID NO: 27, 28 and 29, respectively;
4) the amino acid sequence in the immunoglobulin single variable domain comprises or is set forth in any of SEQ ID NOs: 22, 23, and 32 to 38, or has at least 90%, at least 95%, at least 98%, or at least 99% sequence identity to any of SEQ ID NOs: 22, 23, and 32 to 38; and/or
5) the CDR1, CDR2 and CDR3 in the immunoglobulin single variable domain are the CDR1, CDR2 and CDR3 in SEQ ID NO: 22, or the CDR1, CDR2 and CDR3 in SEQ ID NO: 23, or the CDR1, CDR2 and CDR3 in SEQ ID NO: 32, or the CDR1, CDR2 and CDR3 in SEQ ID NO: 33, or the CDR1, CDR2 and CDR3 in SEQ ID NO: 34, or the CDR1, CDR2 and CDR3 in SEQ ID NO: 35, or the CDR1, CDR2 and CDR3 in SEQ ID NO: 36, or the CDR1, CDR2 and CDR3 in SEQ ID NO: 37, or the CDR1, CDR2 and CDR3 in SEQ ID NO: 38; the CDRs are defined according to the Kabat, IMGT, Chothia, AbM or Contact numbering scheme; in some specific embodiments, the CDRs are defined according to the Kabat numbering scheme.

In some embodiments, provided is a CD40-binding molecule, comprising any one or more (e.g., 1, 2, 3, 4, 5, 6, 7, 8, 9 or 10) of the aforementioned immunoglobulin single variable domains, and the immunoglobulin single variable domains may be identical or different.

In some embodiments, the aforementioned immunoglobulin single variable domains are nanobodies or VHHs.

In some embodiments, the aforementioned CD40-binding molecule further comprises a human immunoglobulin Fc region. In some specific embodiments, the Fc region is the Fc region of human IgG1, IgG2 or IgG4. In some specific embodiments, the Fc region of human IgG1 has a mutation that increases affinity for FcyRIIb or reduces ADCC effects, for example, S267E/L328F. Exemplary mutations that reduce ADCC effects are L234A/L235A on IgG1, V234A/G237A/P238S/H268A/V309L/A330S/P331S on IgG2, F234A/L235A on IgG4, S228P/F234A/L235A on IgG4, N297A on IgG1 IgG2, IgG3 or IgG4, V234A/G237A on IgG2, K214T/E233P/L234V/L235A/G236 deletion/A327G/P331A/D365E/L358M on IgG1, H268Q/V309L/A330S/P331S on IgG2, S267E/L328F on IgG1, L234F/L235E/D265A on IgG1, L234A/L235A/G237A/P238S/H268A/A330S/P331S on IgG1, S228P/F234A/L235A/G237A/P238S on IgG4, and S228P/F234A/L235A/G236 deletion/G237A/P238S on IgG4. A hybrid IgG2/4Fc domain may also be used, e.g., an Fc with residues 117-260 from IgG2 and residues 261-447 from IgG4. Exemplary mutations that increase affinity for FcyRIIb further include E233D/G237D/P238D/H268D/P271G/A330R on IgG1.

In some embodiments, the aforementioned Fc region may cause the anti-CD40 antibody or the antigen-binding fragment thereof to form a dimeric or multimeric molecule.

In some embodiments, the immunoglobulin single variable domain in the aforementioned CD40-binding molecule is linked to the Fc region, either directly or by a linker. The linker may be a non-functional amino acid sequence of 1-20 or more amino acids without a secondary or higher structure. For example, the linker is a flexible linker, for example, G₄S, GS, GAP, (G₄S)₂, (G₄S)₃, (G₄S)₄, (G₄S)₅ or ASGS.

In some embodiments, provided is a CD40-binding molecule, comprising any of the amino acid sequences set forth in SEQ ID NOs: 57 to 63 or an amino acid sequence having at least 80% identity thereto.

In some embodiments, the aforementioned CD40-binding molecule is linked, directly or indirectly, to at least one antibody that binds to another antigen, for example, forming a heterodimer.

In some embodiments, the aforementioned CD40-binding molecule has an activity selected from at least one of the following:
(a) binding to human CD40 or an epitope thereof with a K_{D} value of ≤10⁻⁷ M;
(b) increasing or promoting APC (e.g., dendritic cell (DC)) activation and/or proliferation; and
(c) inhibiting tumor growth and/or metastasis.

In some embodiments, the aforementioned CD40-binding molecule inhibits the binding of CD40 to CD40L or competes with CD40L for binding to CD40.

In some embodiments, the aforementioned CD40-binding molecules can inhibit tumor growth and/or metastasis by at least about 10%, e.g., at least about 20%, about 30%, about 40%, about 50%, about 60%, about 70%, or about 80%.

In some embodiments, the aforementioned CD40-binding molecule reduces the binding of CD40L to CD40 by at least 45%, at least 50%, at least 60%, at least 75%, at least 80%, at least 90%, or at least 95%.

In some embodiments, the aforementioned CD40-binding molecule binds to human CD40 with a K_{D} of 10⁻⁷ M, 10⁻⁸ M, 10⁻⁹ M, 10⁻¹⁰ M, 10⁻¹¹ M or lower.

In some embodiments, the aforementioned CD40-binding molecule is an anti-CD40 antibody or an antigen-binding fragment thereof.

In some embodiments, the aforementioned CD40-binding molecule is an agonist anti-CD40 antibody or an antigen-binding fragment thereof.

In some embodiments, the aforementioned anti-CD40 antibody is a camelid antibody, a chimeric antibody, a humanized antibody, or a fully human antibody. In some embodiments, the aforementioned anti-CD40 antibody is a nanobody or a VHH.

In some embodiments, the aforementioned anti-CD40 antibody or the antigen-binding fragment thereof includes, but is not limited to, linear antibodies, single-chain antibodies, nanobodies, peptibodies, domain antibodies and multispecific antibodies (bispecific antibodies, diabodies, triabodies and tetrabodies, tandem di-scFv, tandem tri-scFv).

In some embodiments, the aforementioned anti-CD40 antibody or the antigen-binding fragment thereof encompasses variants, for example, comprises one or more amino acid substitutions, e.g., conservative amino acid substitutions, as compared to any of SEQ ID NOs: 22, 23, and 32 to 38; for example, the variants comprise 1, 2, 3, 4, 5, 6, 7, 8, 9 or 10 conservative amino acid substitutions.

In some embodiments, the present disclosure provides an anti-CD40 antibody or an antigen-binding fragment thereof, obtained by affinity maturation of SEQ ID NO: 22 or 23, and the affinity-matured anti-CD40 antibody may have in one or more CDRs one or more changes that result in increased affinity for CD40 as compared to the parent anti-CD40 antibody.

In some embodiments, provided is an anti-CD40 antibody or an antigen-binding fragment thereof, which binds to, or competes for binding to the same epitope with, the aforementioned anti-CD40 antibody or the antigen-binding fragment thereof.

In some embodiments, provided is an anti-CD40 antibody or an antigen-binding fragment thereof, which blocks the binding of the aforementioned anti-CD40 antibody or the antigen-binding fragment thereof to CD40 (e.g., human CD40).

In some embodiments, provided is an anti-CD40 antibody or an antigen-binding fragment thereof, the binding of which to CD40 (e.g., human CD40) is blocked by the aforementioned anti-CD40 antibody or the antigen-binding fragment thereof.

In some embodiments, provided is a conjugate, comprising the aforementioned anti-CD40 antibody of the present disclosure or the antigen-binding fragment thereof.

In the present disclosure, "at least 90% (sequence) identity" encompasses at least 90%, at least 91%, at least 92%, at least 93%, at least 94%, at least 95%, at least 96%, at least 97%, at least 98%, at least 99%, or 100% (sequence) identity; "at least 80% (sequence) identity" encompasses at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, or 100% (sequence) identity.

### FAP-Binding Molecule

The present disclosure provides a FAP-binding molecule, comprising a heavy chain variable region and a light chain variable region, wherein the heavy chain variable region comprises a HCDR1, a HCDR2 and a HCDR3, and the light chain variable region comprises a LCDR1, a LCDR2 and a LCDR3, wherein:
1) the amino acid sequence of the HCDR1 is set forth in SEQ ID NO: 9, and/or the amino acid sequence of the HCDR2 is set forth in SEQ ID NO: 10, and/or the amino acid sequence of the HCDR3 is set forth in SEQ ID NO: 18; and/or the amino acid sequence of the LCDR1 is set forth in SEQ ID NO: 12, and/or the amino acid sequence of the LCDR2 is set forth in SEQ ID NO: 13, and/or the amino acid sequence of the LCDR3 is set forth in SEQ ID NO: 19;
2) the amino acid sequences of the HCDR1, HCDR2 and HCDR3 are set forth in SEQ ID NOs: 9, 10 and 18, respectively; and/or the amino acid sequences of the LCDR1, LCDR2 and LCDR3 are set forth in SEQ ID NOs: 12, 13 and 19, respectively;
3) the amino acid sequences of the HCDR1, HCDR2 and HCDR3 are set forth in SEQ ID NOs: 9, 10 and 11, respectively, and the amino acid sequences of the LCDR1, LCDR2 and LCDR3 are set forth in SEQ ID NOs: 12, 13 and 14, respectively;
   the amino acid sequences of the HCDR1, HCDR2 and HCDR3 are set forth in SEQ ID NOs: 9, 10 and 15, respectively, and the amino acid sequences of the LCDR1, LCDR2 and LCDR3 are set forth in SEQ ID NOs: 12, 13 and 14, respectively;
   the amino acid sequences of the HCDR1, HCDR2 and HCDR3 are set forth in SEQ ID NOs: 9, 10 and 15, respectively; the amino acid sequences of the LCDR1, LCDR2 and LCDR3 are set forth in SEQ ID NOs: 12, 13 and 16, respectively; or
   the amino acid sequences of the HCDR1, HCDR2 and HCDR3 are set forth in SEQ ID NOs: 9, 10 and 11, respectively; the amino acid sequences of the LCDR1, LCDR2 and LCDR3 are set forth in SEQ ID NOs: 12, 13 and 17, respectively;
4) the heavy chain variable region comprises the amino acid sequence set forth in SEQ ID NO: 1 or an amino acid sequence having at least 90% identity thereto, and the light chain variable region comprises the amino acid sequence set forth in SEQ ID NO: 2 or an amino acid sequence having at least 90% identity thereto;
   the heavy chain variable region comprises the amino acid sequence set forth in SEQ ID NO: 3 or an amino acid sequence having at least 90% identity thereto, and the light chain variable region comprises the amino acid sequence set forth in SEQ ID NO: 4 or an amino acid sequence having at least 90% identity thereto;
   the heavy chain variable region comprises the amino acid sequence set forth in SEQ ID NO: 5 or an amino acid sequence having at least 90% identity thereto, and the light chain variable region comprises the amino acid sequence set forth in SEQ ID NO: 6 or an amino acid sequence having at least 90% identity thereto; or
   the heavy chain variable region comprises the amino acid sequence set forth in SEQ ID NO: 7 or an amino acid sequence having at least 90% identity thereto, and the light chain variable region comprises the amino acid sequence set forth in SEQ ID NO: 8 or an amino acid sequence having at least 90% identity thereto;
5) the heavy chain variable region comprises the HCDR1, HCDR2 and HCDR3 in SEQ ID NO: 1, and the light chain variable region comprises the LCDR1, LCDR2 and LCDR3 in SEQ ID NO: 2;
   the heavy chain variable region comprises the HCDR1, HCDR2 and HCDR3 in SEQ ID NO: 3, and the light chain variable region comprises the LCDR1, LCDR2 and LCDR3 in SEQ ID NO: 4;
   the heavy chain variable region comprises the HCDR1, HCDR2 and HCDR3 in SEQ ID NO: 5, and the light chain variable region comprises the LCDR1, LCDR2 and LCDR3 in SEQ ID NO: 6; or
   the heavy chain variable region comprises the HCDR1, HCDR2 and HCDR3 in SEQ ID NO: 7, and the light chain variable region comprises the LCDR1, LCDR2 and LCDR3 in SEQ ID NO: 8, wherein the CDRs are defined according to the Kabat, IMGT, Chothia, AbM or Contact numbering scheme, e.g., the Kabat numbering scheme.

In some embodiments, provided is a FAP-binding molecule (e.g., an anti-FAP antibody or an antigen-binding fragment thereof), comprising any of or a combination of several of the aforementioned HCDR1, HCDR2, HCDR3, LCDR1, LCDR2 and LCDR3.

In some embodiments, the aforementioned FAP-binding molecule further comprises a human immunoglobulin Fc region. In some specific embodiments, the Fc region is the Fc region of human IgG1, IgG2 or IgG4. In some embodiments, the Fc of human IgG1 has a mutation that removes or reduces Fc effector function, e.g., N297A or D265A/N297A on IgG1. Other exemplary mutations that remove or reduce Fc effector function include L234A/L235A, L234A/L235A/P329G, L234E, L234F, L234E/L235F or L234E/L235F/P329G on IgG1, V234A/G237A/P238S/H268A/V309L/A330S/P331S on IgG2, F234A/L235A on IgG4, S228P/F234A/L235A on IgG4, N297A on IgG2 or IgG4, V234A/G237A on IgG2, K214T/E233P/L234V/L235A/G236 deletion/A327G/P331A/D365E/L358M on IgG1, H268Q/V309L/A330S/P331S on IgG2, S267E/L328F on IgG1, L234F/L235E/D265A on IgG1, L234A/L235A/G237A/P238S/H268A/A330S/P331S on IgG1, and S228P/F234A/L235A/G237A/P238S on IgG4.

In some embodiments, provided is a FAP-binding molecule (e.g., an anti-FAP antibody or an antigen-binding fragment thereof), comprising a heavy chain (HC) and a light chain (LC), wherein:
the heavy chain is the amino acid sequence set forth in SEQ ID NO: 49 or an amino acid sequence having at least 80% identity thereto, and the light chain is the amino acid sequence set forth in SEQ ID NO: 50 or an amino acid sequence having at least 80% identity thereto;
the heavy chain is the amino acid sequence set forth in SEQ ID NO: 51 or an amino acid sequence having at least 80% identity thereto, and the light chain is the amino acid sequence set forth in SEQ ID NO: 52 or an amino acid sequence having at least 80% identity thereto;
the heavy chain is the amino acid sequence set forth in SEQ ID NO: 53 or an amino acid sequence having at least 80% identity thereto, and the light chain is the amino acid sequence set forth in SEQ ID NO: 54 or an amino acid sequence having at least 80% identity thereto; or
the heavy chain is the amino acid sequence set forth in SEQ ID NO: 55 or an amino acid sequence having at least 80% identity thereto, and the light chain is the amino acid sequence set forth in SEQ ID NO: 56 or an amino acid sequence having at least 80% identity thereto.

In some embodiments, the heavy chain variable region of the aforementioned FAP-binding molecule has 0 to 10 (1, 2, 3, 4, 5, 6, 7, 8, 9 or 10) amino acid changes; the light chain variable region has 0 to 10 (1, 2, 3, 4, 5, 6, 7, 8, 9 or 10) amino acid changes. In some specific embodiments, the amino acid changes are conservative replacements, substitutions or modifications, and/or deletions or additions that do not affect function.

In some embodiments, the aforementioned FAP-binding molecule has an activity selected from at least one of the following:
(a) binding to human FAP or an epitope thereof with a K_{D} value of ≤10⁻⁷;
(b) increasing or promoting APC (e.g., dendritic cell (DC)) activation and/or proliferation; and
(c) inhibiting tumor growth and/or metastasis.

In some embodiments, the aforementioned FAP-binding molecule binds to FAP (e.g., human FAP) or a fragment thereof with a K_{D} of 10⁻⁷ M, 10⁻⁸ M, 10⁻⁹ M, 10⁻¹⁰ M, 10⁻¹¹ M or lower.

In some embodiments, the aforementioned FAP-binding molecule is an anti-FAP antibody or an antigen-binding fragment thereof; in some specific embodiments, the aforementioned FAP-binding molecule is a chimeric antibody, a humanized antibody or a fully human antibody, or an antigen-binding fragment thereof.

In some embodiments, the antigen-binding fragment of the aforementioned anti-FAP antibody includes, but is not limited to, Fab, Fv, sFv, Fab', F(ab')₂, linear antibodies, single-chain antibodies, scFv, sdAb, sdFv, nanobodies, peptibodies, domain antibodies and multispecific antibodies (bispecific antibodies, diabodies, triabodies and tetrabodies, tandem di-scFv, tandem tri-scFv), and is, for example, an scFv, Fv, Fab or Fab' fragment.

In some embodiments, provided is an anti-FAP antibody or an antigen-binding fragment thereof, which binds to, or competes for binding to the same FAP (e.g., human FAP) epitope with, the aforementioned anti-FAP antibody or the antigen-binding fragment thereof.

In some embodiments, provided is an anti-FAP antibody or an antigen-binding fragment thereof, which blocks the binding of the aforementioned anti-FAP antibody or the antigen-binding fragment thereof to FAP (e.g., human FAP).

In some embodiments, provided is an anti-FAP antibody or an antigen-binding fragment thereof, the binding of which to FAP (e.g., human FAP) is blocked by the aforementioned anti-FAP antibody or the antigen-binding fragment thereof.

In some embodiments, provided is a conjugate, comprising the aforementioned anti-FAP antibody or the antigen-binding fragment thereof.

### FAP/CD40-Binding Molecule

The present disclosure provides a FAP/CD40-binding molecule, comprising a first antigen-binding domain that specifically binds to FAP and a second antigen-binding domain that specifically binds to CD40, wherein the second antigen-binding domain that specifically binds to CD40 comprises at least one immunoglobulin single variable domain.

In some embodiments, the FAP/CD40-binding molecule comprises one immunoglobulin single variable domain that specifically binds to CD40. In some other embodiments, the FAP/CD40-binding molecule comprises 2, 3, 4, 5, 6, 7, 8, 9, 10 or more immunoglobulin single variable domains that specifically bind to CD40, and the immunoglobulin single variable domains may be identical or different.

In some embodiments, the FAP/CD40-binding molecule comprises at least one (e.g., 2, 3 or 4) antigen-binding domain that specifically binds to FAP.

In some embodiments, in the aforementioned FAP/CD40-binding molecule, the immunoglobulin single variable domain that specifically binds to CD40 comprises three complementarity-determining regions CDR1, CDR2 and CDR3, wherein the amino acid sequences of the CDR1, CDR2 and CDR3 are set forth in SEQ ID NOs: 39, 40 and 41, respectively. In some specific embodiments, the amino acid sequences of the CDR1, CDR2 and CDR3 are set forth in SEQ ID NOs: 24, 25 and 26, respectively; or the amino acid sequences of the CDR1, CDR2 and CDR3 are set forth in SEQ ID NOs: 27, 28 and 29, respectively.

In some embodiments, provided is a FAP/CD40-binding molecule, comprising a first antigen-binding domain that specifically binds to FAP and a second antigen-binding domain that specifically binds to CD40, wherein the first antigen-binding domain that specifically binds to FAP comprises a heavy chain variable region and a light chain variable region; the heavy chain variable region comprises a HCDR1, a HCDR2 and a HCDR3, and the light chain variable region comprises a LCDR1, a LCDR2 and a LCDR3, wherein:
the amino acid sequences of the HCDR1, HCDR2 and HCDR3 are set forth in SEQ ID NOs: 9, 10 and 18, respectively; the amino acid sequences of the LCDR1, LCDR2 and LCDR3 are set forth in SEQ ID NOs: 12, 13 and 19, respectively.

In some specific embodiments, in the aforementioned first antigen-binding domain that specifically binds to FAP:
the amino acid sequences of the HCDR1, HCDR2 and HCDR3 are set forth in SEQ ID NOs: 9, 10 and 11, respectively; the amino acid sequences of the LCDR1, LCDR2 and LCDR3 are set forth in SEQ ID NOs: 12, 13 and 14, respectively;
the amino acid sequences of the HCDR1, HCDR2 and HCDR3 are set forth in SEQ ID NOs: 9, 10 and 15, respectively; the amino acid sequences of the LCDR1, LCDR2 and LCDR3 are set forth in SEQ ID NOs: 12, 13 and 14, respectively;
the amino acid sequences of the HCDR1, HCDR2 and HCDR3 are set forth in SEQ ID NOs: 9, 10 and 15, respectively; the amino acid sequences of the LCDR1, LCDR2 and LCDR3 are set forth in SEQ ID NOs: 12, 13 and 16, respectively; or
the amino acid sequences of the HCDR1, HCDR2 and HCDR3 are set forth in SEQ ID NOs: 9, 10 and 11, respectively; the amino acid sequences of the LCDR1, LCDR2 and LCDR3 are set forth in SEQ ID NOs: 12, 13 and 17, respectively.

In some embodiments, in the aforementioned FAP/CD40-binding molecule, the heavy chain variable region of the first antigen-binding domain that specifically binds to FAP comprises the amino acid sequence set forth in SEQ ID NO: 1 or an amino acid sequence having at least 80% identity thereto, and the light chain variable region comprises the amino acid sequence set forth in SEQ ID NO: 2 or an amino acid sequence having at least 80% identity thereto;
the heavy chain variable region comprises the amino acid sequence set forth in SEQ ID NO: 3 or an amino acid sequence having at least 80% identity thereto, and the light chain variable region comprises the amino acid sequence set forth in SEQ ID NO: 4 or an amino acid sequence having at least 80% identity thereto;
the heavy chain variable region comprises the amino acid sequence set forth in SEQ ID NO: 5 or an amino acid sequence having at least 80% identity thereto, and the light chain variable region comprises the amino acid sequence set forth in SEQ ID NO: 6 or an amino acid sequence having at least 80% identity thereto; or
the heavy chain variable region comprises the amino acid sequence set forth in SEQ ID NO: 7 or an amino acid sequence having at least 80% identity thereto, and the light chain variable region comprises the amino acid sequence set forth in SEQ ID NO: 8 or an amino acid sequence having at least 80% identity thereto.

In some embodiments, in the aforementioned FAP/CD40-binding molecule, the first antigen-binding domain that specifically binds to FAP comprises a heavy chain (HC) and a light chain (LC);
for example, the heavy chain is of the IgG1 or IgG4 isotype, and the light chain is of the Kappa isotype;
in some specific embodiments, the heavy chain is the amino acid sequence set forth in SEQ ID NO: 49 or an amino acid sequence having at least 80% identity thereto, and the light chain is the amino acid sequence set forth in SEQ ID NO: 50 or an amino acid sequence having at least 80% identity thereto;
the heavy chain is the amino acid sequence set forth in SEQ ID NO: 51 or an amino acid sequence having at least 80% identity thereto, and the light chain is the amino acid sequence set forth in SEQ ID NO: 52 or an amino acid sequence having at least 80% identity thereto;
the heavy chain is the amino acid sequence set forth in SEQ ID NO: 53 or an amino acid sequence having at least 80% identity thereto, and the light chain is the amino acid sequence set forth in SEQ ID NO: 54 or an amino acid sequence having at least 80% identity thereto; or
the heavy chain is the amino acid sequence set forth in SEQ ID NO: 55 or an amino acid sequence having at least 80% identity thereto, and the light chain is the amino acid sequence set forth in SEQ ID NO: 56 or an amino acid sequence having at least 80% identity thereto.

In some embodiments, in the aforementioned FAP/CD40-binding molecule, the first antigen-binding domain that specifically binds to FAP comprises a heavy chain variable region and a light chain variable region, wherein:
the immunoglobulin single variable domain of the second antigen-binding domain that specifically binds to CD40 is located at the N-terminus of the heavy chain variable region of the first antigen-binding domain that specifically binds to FAP;
the immunoglobulin single variable domain of the second antigen-binding domain that specifically binds to CD40 is located at the C-terminus of the heavy chain variable region of the first antigen-binding domain that specifically binds to FAP;
the immunoglobulin single variable domain of the second antigen-binding domain that specifically binds to CD40 is located at the N-terminus of the light chain variable region of the first antigen-binding domain that specifically binds to FAP; and/or
the immunoglobulin single variable domain of the second antigen-binding domain that specifically binds to CD40 is located at the C-terminus of the light chain variable region of the first antigen-binding domain that specifically binds to FAP.

In some embodiments, the immunoglobulin single variable domain of the aforementioned second antigen-binding domain that specifically binds to CD40 is linked, either directly or by a linker, to the first antigen-binding domain that specifically binds to FAP; for example, the linker has an amino acid sequence represented by (G₄S)ₓ, wherein x is independently selected from an integer from 1 to 20 (e.g., 1, 2, 3, 4, 5, 6, 7, 8, 9 or 10); for example, the linker is an amino acid sequence represented by (G₄S)₂, (G₄S)₃ or (G₄S)₄.

In some embodiments, in the aforementioned FAP/CD40-binding molecule, the second antigen-binding domain that specifically binds to CD40 is multivalent (for example, one of the aforementioned FAP/CD40-binding molecules comprises 1, 2, 3, 4, 5, 6, 7, 8, 9 or 10 second antigen-binding domains that specifically bind to CD40). In some specific embodiments, the second antigen-binding domain that specifically binds to CD40 is divalent, tetravalent, or hexavalent. In some specific embodiments, the second antigen-binding domain that specifically binds to CD40 comprises 2, 3, 4, 5 or 6 said immunoglobulin single variable domains.

In some embodiments, provided is a FAP/CD40-binding molecule, comprising a first polypeptide chain and a second polypeptide chain, wherein the first polypeptide chain comprises any of the amino acid sequences set forth in SEQ ID NOs: 42 and 44-48 or an amino acid sequence having at least 80% identity thereto, and the second polypeptide chain comprises the amino acid sequence set forth in SEQ ID NO: 43 or an amino acid sequence having at least 80% identity thereto. In some specific embodiments, one FAP/CD40-binding molecule comprises two first polypeptide chains and two second polypeptide chains; in some specific embodiments, the two first polypeptide chains are identical and the two second polypeptide chains are identical.

In some embodiments, the aforementioned FAP/CD40-binding molecule can inhibit tumor growth (e.g., volume increase or tumor weight increase) and/or metastasis (e.g., multi-organ or multi-tissue metastasis or distant metastasis) by at least about 10%, e.g., at least about 20%, about 30%, about 40%, about 50%, about 60%, about 70%, about 80% or about 90%.

In some embodiments, the aforementioned FAP/CD40-binding molecule is an anti-FAP/CD40 bispecific antibody or an antigen-binding fragment thereof, wherein the antigen-binding fragment includes, but is not limited to, Fab, Fv, sFv, Fab', F(ab')₂, linear antibodies, single-chain antibodies, scFv, sdAb, sdFv, nanobodies, peptibodies, domain antibodies and multispecific antibodies (bispecific antibodies, diabodies, triabodies and tetrabodies, tandem di-scFv, tandem tri-scFv), and is, for example, an scFv, Fv, Fab or Fab' fragment.

In some embodiments, the anti-FAP/CD40 bispecific antibody comprises the immunoglobulin single variable domain in the aforementioned second antigen-binding domain that specifically binds to CD40, as provided by the aforementioned disclosure, and the heavy chain variable region (VH) and the light chain variable region (VL) in the first antigen-binding domain that specifically binds to FAP, as provided by the aforementioned disclosure.

In some embodiments, in the anti-FAP/CD40 bispecific antibody:
the first antigen-binding domain that specifically binds to FAP is a first antibody, comprising a heavy chain (HC) and a light chain (LC); and
the second antigen-binding domain that specifically binds to CD40 is a second antibody, which is a VHH, having the CDR1, CDR2 and CDR3 in the aforementioned CD40-binding molecule provided by the present disclosure.

In some specific embodiments, the VHH, as a second antibody, is located at the N-terminus and/or the C-terminus of the heavy or light chain of the first antibody.

In some specific embodiments, the anti-FAP/CD40 bispecific antibody comprises 1 first antibody and 1, 2, 3, 4, 5, 6, 7, 8, 9 or 10 (e.g., 2, 4 or 6) VHH second antibodies; the first antibody comprises two HCs and two LCs, wherein the VH of one HC of the first antibody forms an antigen-binding site with the VL of one LC, and the VH of the other HC forms an antigen-binding site with the VL of the other LC.

In some specific embodiments, the first antibody of the anti-FAP/CD40 bispecific antibody or the antigen-binding fragment thereof may be linked with 1, 2, 3, 4, 5, 6, 7 or 8 VHH second antibodies, and the VHH second antibodies may be identical or different, may all be linked to the N-terminus of the heavy chain of the first antibody, or may all be linked to the C-terminus of the heavy chan of the first antibody, or may all be linked to the N-terminus of the light chain of the first antibody, or may all be linked to the C-terminus of the light chain of the first antibody, or may be linked to any combination of the N-terminus of the heavy chain, the C-terminus of the heavy chain, the N-terminus of the light chain and the C-terminus of the light chain. In some specific embodiments, the anti-FAP/CD40 bispecific antibody comprises a first polypeptide chain and a second polypeptide chain, wherein:
(i) the first polypeptide chain is, from N-terminus to C-terminus: [heavy chain of first antibody]-linker 1-[second antibody]; the second polypeptide chain is the light chain of the first antibody;
(ii) the first polypeptide chain is, from N-terminus to C-terminus: [heavy chain of first antibody]-linker 1-[second antibody]i-linker 2-[second antibody]₂; the second polypeptide chain is the light chain of the first antibody;
(iii) the first polypeptide chain is, from N-terminus to C-terminus: [heavy chain of first antibody]-linker 1-[second antibody]i-linker 2-[second antibody]₂-linker 3-[second antibody]₃; the second polypeptide chain is the light chain of the first antibody;
(iv) the first polypeptide chain is, from N-terminus to C-terminus: [second antibody]-linker 1-[heavy chain of first antibody]; the second polypeptide chain is the light chain of the first antibody;
(v) the first polypeptide chain is, from N-terminus to C-terminus: [second antibody]₂-linker 2-[second antibody]i-linker 1-[heavy chain of first antibody]; the second polypeptide chain is the light chain of the first antibody;
(vi) the first polypeptide chain is, from N-terminus to C-terminus: [second antibody]₃-linker 3-[second antibody]₂-linker 2-[second antibody]i-linker 1-[heavy chain of first antibody]; the second polypeptide chain is the light chain of the first antibody;
(vii) the first polypeptide chain is, from N-terminus to C-terminus: [second antibody] i-linker 1-[heavy chain of first antibody]-linker 2-[second antibody]₂; the second polypeptide chain is the light chain of the first antibody;
(viii) the first polypeptide chain is, from N-terminus to C-terminus: [second antibody]₁-linker 1-[second antibody]₂-linker 2-[heavy chain of first antibody]-linker 3-[second antibody]₃; the second polypeptide chain is the light chain of the first antibody;
(ix) the first polypeptide chain is, from N-terminus to C-terminus: [second antibody] i-linker 1-[heavy chain of first antibody]-linker 2-[second antibody]₂-linker 3-[second antibody]₃; the second polypeptide chain is the light chain of the first antibody;
wherein the [secondary antibody]₁, [secondary antibody]₂ and [secondary antibody]₃ may be identical or different.

In some embodiments, the VHH second antibodies in the aforementioned anti-FAP/CD40 bispecific antibody are linked to the first antibody, either directly or by linkers. The linkers are selected from the group consisting of amino acid sequences represented by (GₘSₙ)ₓ, (GGNGT)ₓ and (YGNGT)ₓ, wherein m and n are each independently selected from integers from 1 to 8 (e.g., 1, 2, 3, 4, 5, 6, 7 or 8), and x is independently selected from an integer from 1 to 20 (e.g., 1, 2, 3, 4, 5, 6, 7, 8, 9, 10, 11, 12, 13, 14, 15, 16, 17, 18, 19 or 20). For example, the linkers are amino acid sequences represented by G₄S, (G₄S)₂, (G₄S)₃, (G₄S)₄, (G₄S)₅, or (G₄S)₆. In some embodiments, linker 1, linker 2 and linker 3 may be identical or different.

In some embodiments, the heavy chain of the first antibody of the anti-FAP/CD40 bispecific antibody comprises a heavy chain variable region (VH) and a heavy chain constant region (CH), and the light chain comprises a light chain variable region (VL) and a light chain constant region (CL). The first antibody may be a full-length antibody. In some embodiments, the heavy chain of the first antibody of the anti-FAP/CD40 bispecific antibody is of an IgG isotype (e.g., IgG1, IgG2, IgG3 or IgG4), e.g., the IgG1 isotype; and/or the light chain of the first antibody is of the Kappa isotype.

In some embodiments, the two HCs of the anti-FAP/CD40 bispecific antibody comprise the same CDRs and/or the two LCs comprise the same CDRs. In some specific embodiments, the two HCs of the first antibody comprise the same VH and/or the two LCs comprise the same VL. In some specific embodiments, the two HCs of the first antibody have identical amino acid sequences and/or the two LCs have identical amino acid sequences.

In some embodiments, two VHH second antibodies of the anti-FAP/CD40 bispecific antibody have identical or different amino acid sequences. For example, the two VHH second antibodies have identical amino acid sequences.

In some embodiments, the anti-FAP/CD40 bispecific antibody comprises two first polypeptide chains and two second polypeptide chains, wherein for each polypeptide chain: a) each of the first polypeptide chains independently comprises a VHH second antibody and the heavy chain (HC) of the first antibody; and b) each of the second polypeptide chains independently comprises the light chain (LC) of the first antibody; wherein the VHH is linked to the N-terminus and/or the C-terminus of the HC of the second antibody by a linker;
or, i) each of the first polypeptide chains independently comprises the heavy chain (HC) of the first antibody; and ii) each of the second polypeptide chains independently comprises a VHH second antibody and the light chain (LC) of the first antibody; wherein the VHH is linked to the N-terminus and/or the C-terminus of the LC of the first antibody either directly or by a linker.

In some specific embodiments, the anti-FAP/CD40 bispecific antibody comprises two identical first polypeptide chains and two identical second polypeptide chains. In some embodiments, an antibody that competes for binding to the same epitope with the anti-FAP/CD40 bispecific antibody of the present disclosure is provided.

In some embodiments, a mutation is introduced into the Fc region of the FAP/CD40-binding molecule or the anti-FAP/CD40 bispecific antibody of the present disclosure. The mutation is, for example, a mutation that removes or reduces IgG Fc effector function, including but not limited to, N297A or D265A/N297A on IgG1, or L234A/L235A, L234A/L235A/P329G, L234E, L234F, L234E/L235F or L234E/L235F/P329G on IgG1, V234A/G237A/P238S/H268A/V309L/A330S/P331S on IgG2, F234A/L235A on IgG4, S228P/F234A/L235A on IgG4, N297A on IgG2 or IgG4, V234A/G237A on IgG2, K214T/E233P/L234V/L235A/G236 deletion/A327G/P331A/D365E/L358M on IgG1, H268Q/V309L/A330S/P331S on IgG2, S267E/L328F on IgG1, L234F/L235E/D265A on IgG1, L234A/L235A/G237A/P238S/H268A/A330S/P331S on IgG1, and S228P/F234A/L235A/G237A/P238S on IgG4.

In the context of the mutations contained in the Fc region herein, "/" represents "and"; for example, "D265A/N297A" represents "D265A and N297A"; that is, the Fc comprises the mutations D265A and N297A; the amino acid positions of the mutations are numbered according to the EU numbering scheme.

In some embodiments, the aforementioned FAP/CD40-binding molecule or anti-FAP/CD40 bispecific antibody provided by the present disclosure has one or more of the following characteristics:
(a) binding to human FAP or an epitope thereof with a K_{D} value of ≤10⁻⁷;
(b) binding to human CD40 or an epitope thereof with a K_{D} value of ≤10⁻⁷;
(c) inducing the immune stimulation of CD40-expressing antigen-presenting cells (APCs);
(d) increasing APC (e.g., dendritic cell) activation, and/or promoting APC (e.g., dendritic cell) proliferation;
(e) stimulating tumor-specific T-cell responses;
(f) causing or promoting tumor cell apoptosis; and/or
(g) inhibiting tumor growth and/or metastasis.

In addition, the anti-CD40 antibody, the anti-FAP antibody and the FAP/CD40-binding molecule of the present disclosure are all resistant to heat treatment or have relatively high stability. For example, no significant aggregation or degradation is seen after 30 days of treatment at 40 °C, and they are stable at least at 60 °C.

### Polynucleotide

The present disclosure provides polynucleotides that encode the CD40-binding molecule, the FAP-binding molecule, the FAP/CD40-binding molecule, and the anti-FAP/CD40 bispecific antibody or the antigen-binding fragment thereof of the present disclosure. The nucleic acids of the present disclosure may be RNAs, DNAs or cDNAs. In some embodiments, the nucleic acids of the present disclosure are substantially isolated nucleic acids.

The nucleic acids of the present disclosure may also be in the form of a vector, and they may be present in the vector and/or may be part of the vector. The vector is, for example, a plasmid, cosmid, YAC or viral vector. The vector may especially be an expression vector, i.e., a vector that provides for the *in-vitro* and/or *in-vivo* (i.e., in suitable host cells, host organisms and/or expression systems) expression of the CD40-binding molecule. The expression vector generally comprises at least one of the nucleic acids of the present disclosure, which is operably linked to one or more suitable expression regulatory elements (e.g., promoters, enhancers and terminators). The selection of the elements and their sequences for expression in a particular host is within the knowledge of those skilled in the art. Regulatory elements and other elements useful or necessary for the expression of the CD40-binding molecule, the FAP-binding molecule, the FAP/CD40-binding molecule, or the anti-FAP/CD40 bispecific antibody or the antigen-binding fragment thereof of the present disclosure are, for example, promoters, enhancers, terminators, integrators, selection markers, leader sequences, and reporter genes.

The nucleic acids of the present disclosure may be prepared or obtained by known means (e.g., by automatic DNA synthesis and/or recombinant DNA techniques) based on information on the amino acid sequence of the polypeptide of the present disclosure, and/or may be isolated from a suitable natural source.

### Host Cell

The present disclosure provides a recombinant host cell that expresses or is capable of expressing one or more of the CD40-binding molecules, the FAP-binding molecules, the FAP/CD40-binding molecules, the anti-FAP/CD40 bispecific antibodies or the antigen-binding fragments thereof of the present disclosure, and/or comprises the nucleic acids or vectors of the present disclosure.

In some embodiments, the host cell is a bacterial cell, a fungal cell or a mammalian cell. Bacterial cells include, for example, cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Fungal cells include, for example, cells of the species *Trichoderma, Neurospora,* and *Aspergillus;* or cells of the species *Saccharomyces* (e.g., *Saccharomyces cerevisiae*)*, Schizosaccharomyces* (e.g., *Schizosaccharomyces pombe*)*, Pichia* (*Pichia pastoris* and *Pichia methanolica*)*,* and *Hansenula.*

Mammalian cells include, for example, HEK293 cells, CHO cells, BHK cells, HeLa cells, COS cells, and the like.

However, amphibian cells, insect cells, plant cells, and any other cells used in the art for expressing heterologous proteins may also be used in the present disclosure.

The cells of the present disclosure are unable to develop into complete plants or individual animals.

### Production or Preparation Method

The present disclosure provides a method for preparing the CD40-binding molecule, the FAP-binding molecule, the FAP/CD40-binding molecule, or the anti-FAP/CD40 bispecific antibody or the antigen-binding fragment thereof of the present disclosure, the methods generally comprising the following steps:
- culturing the host cell of the present disclosure under conditions that allow for expression of the CD40-binding molecule, the FAP-binding molecule, the FAP/CD40-binding molecule, or the anti-FAP/CD40 bispecific antibody or the antigen-binding fragment thereof of the present disclosure; and
- isolating the protein of interest expressed by the host cell from the culture; and
- optionally, further purifying and/or modifying the protein of interest of the present disclosure.

The CD40-binding molecule, the FAP-binding molecule, the FAP/CD40-binding molecule, or the anti-FAP/CD40 bispecific antibody or the antigen-binding fragment thereof of the present disclosure may be produced intracellularly (e.g., in the cytoplasm, in the periplasm, or in the inclusion bodies) in the cell described above, and then is isolated from the host cell and optionally further purified; or may be produced extracellularly (e.g., in the medium in which the host cell is cultured), and then is isolated from the medium and optionally further purified.

Methods and reagents for recombinant production of polypeptides, e.g., specific suitable expression vectors, transformation or transfection methods, selection labels, methods for inducing protein expression, culture conditions, and the like, are known in the art. Similarly, techniques for isolating and purifying proteins of interest suitable for use in the preparation of the binding molecules or antibodies of the present disclosure are well known to those skilled in the art. Methods for producing and purifying antibodies are well known in the prior art and can be found in, for example, "Antibodies: A Laboratory Manual", Cold Spring Harbor Press (chapters 5-8 and 15). The engineered antibodies of the present disclosure may also be prepared and purified by conventional methods. For example, cDNA sequences encoding the heavy and light chains can be cloned and recombined into an expression vector. Recombinant immunoglobulin expression vectors can be used to stably transfect cells. Mammalian expression systems may result in glycosylation of antibodies, particularly at the highly conserved N-terminus of the Fc region. Stable clones are obtained by expression of antibodies specifically binding to the human-derived antigen. Positive clones are expanded in a serum-free medium of a bioreactor to produce antibodies. The culture medium with the secreted antibody can be purified and collected by conventional techniques. The antibodies can be filtered and concentrated by conventional methods. Soluble mixtures and multimers can also be removed by conventional methods, such as molecular sieves and ion exchange. The resulting products should be immediately frozen, e.g., at -70 °C, or lyophilized. However, the CD40-binding molecule, the FAP-binding molecule, the FAP/CD40-binding molecule, or the anti-FAP/CD40 bispecific antibody or the antigen-binding fragment thereof of the present disclosure may also be obtained by other methods known in the art of producing proteins, such as chemical synthesis, including solid-phase or liquid-phase synthesis.

### Composition

The present disclosure provides a composition (e.g., a pharmaceutical composition), comprising a prophylactically or therapeutically effective amount of the CD40-binding molecule, the FAP-binding molecule, the FAP/CD40-binding molecule, the anti-FAP/CD40 bispecific antibody or the antigen-binding fragment thereof, or the coding polynucleotide described above in the present disclosure, and one or more pharmaceutically acceptable carriers, diluents, buffers or excipients.

In some specific embodiments, a unit dose of the pharmaceutical composition may comprise 0.01 to 99 wt% of the CD40-binding molecule, the FAP/CD40-binding molecule, or the anti-FAP/CD40 bispecific antibody or the antigen-binding fragment thereof. In some other specific embodiments, a unit dose of the pharmaceutical composition comprises the CD40-binding molecule, the FAP/CD40-binding molecule or the anti-FAP/CD40 bispecific antibody in the amount of 0.1-2000 mg; and in some specific embodiments, 1-1000 mg.

### Kit

The present disclosure provides a kit, comprising the CD40-binding molecule, the FAP-binding molecule, the FAP/CD40-binding molecule, the anti-FAP/CD40 bispecific antibody or the antigen-binding fragment thereof, and/or the coding polynucleotide of the present disclosure. In some embodiments, further provided are a diagnostic reagent comprising the polynucleotide described above and related diagnostic use.

### Method for Treating Disease and Pharmaceutical Use

The present disclosure provides use of the CD40-binding molecule, the FAP-binding molecule, the FAP/CD40-binding molecule, the anti-FAP/CD40 bispecific antibody or the antigen-binding fragment thereof, the coding polynucleotide, or the pharmaceutical composition of the present disclosure in the prevention and/or treatment of a disease and a method for preventing and/or treating disease using same, wherein the disease may or may not be associated with the CD40 signaling pathway. In some embodiments, the present disclosure provides a method for preventing and/or treating a CD40-related disease, the method comprising administering to a subject a prophylactically and/or therapeutically effective amount of the CD40-binding molecule of the present disclosure, or a pharmaceutical composition comprising the CD40-binding molecule of the present disclosure. The present disclosure further provides use of the CD40-binding molecule of the present disclosure in the preparation of a medicament for preventing and/or treating CD40 or CD40L-related diseases or CD40 or CD40L-related disorders.

The CD40-binding molecule, the FAP-binding molecule, the FAP/CD40-binding molecule, the anti-FAP/CD40 bispecific antibody or the antigen-binding fragment thereof, the coding polynucleotide, or the pharmaceutical composition of the present disclosure may be used alone or in combination with other anti-cancer or tumor therapeutic approaches (e.g., other immunogenic agents, standard cancer therapies, or other antibody molecules) to inhibit cancer or tumor growth.

In some embodiments, the present disclosure provides a method for preventing and/or treating a cancer or tumor, comprising administering to a patient or subject a prophylactically and/or therapeutically effective amount of the CD40-binding molecule, the FAP-binding molecule, the FAP/CD40-binding molecule, the anti-FAP/CD40 bispecific antibody or the antigen-binding fragment thereof, the coding polynucleotide, or the pharmaceutical composition of the present disclosure to inhibit tumor cell growth in the patient or subject. In some specific embodiments, the cancer is preferably, but is not limited to, a cancer that is responsive to immunotherapy.

In the above methods or use, non-limiting examples of the cancer or tumor include lung cancer, ovarian cancer, colon cancer, rectal cancer, melanoma (e.g., metastatic malignant melanoma), kidney cancer, bladder cancer, breast cancer, liver cancer, lymphoma, hematologic malignancies, head and neck cancer, glioma, gastric cancer, nasopharyngeal cancer, laryngeal cancer, cervical cancer, uterine body tumors, and osteosarcoma. Examples of other cancers that can be treated using the method of the present disclosure include: bone cancer, pancreatic cancer, skin cancer, prostate cancer, skin or intraocular malignant melanoma, uterine cancer, anal cancer, testicular cancer, fallopian tube cancer, endometrial cancer, vaginal cancer, vulvar cancer, Hodgkin's disease, non-Hodgkin's lymphoma, esophageal cancer, small intestine cancer, endocrine system cancer, thyroid cancer, parathyroid cancer, adrenal cancer, soft tissue sarcoma, urethral cancer, penile cancer, chronic or acute leukemia, including acute myeloid leukemia, chronic myeloid leukemia, acute lymphocytic leukemia, and chronic lymphocytic leukemia, childhood solid tumors, lymphocytic lymphoma, bladder cancer, kidney or ureteral cancer, renal pelvis cancer, central nervous system (CNS) tumor, primary CNS lymphoma, tumor angiogenesis, spinal tumor, brainstem neuroglioma, pituitary adenoma, Kaposi's sarcoma, epidermal carcinoma, squamous cell carcinoma, T cell lymphoma, and environmentally induced cancers, including asbestos-induced cancers, and combinations of the cancers. In some embodiments, the tumor or cancer described above is metastatic and/or advanced.

In addition, the present disclosure further provides a method for preventing and/or treating an infectious disease in a subject or patient, comprising administering to the subject or patient the CD40-binding molecule, the FAP-binding molecule, the FAP/CD40-binding molecule, the anti-FAP/CD40 bispecific antibody or the antigen-binding fragment thereof, the coding polynucleotide, or the pharmaceutical composition of the present disclosure to provide for the prevention and/or treatment of the infectious disease in the subject. In a way similar to the use for the cancer or tumor described above, the CD40-binding molecule may be used alone or in combination with vaccines to stimulate immune responses to pathogens, toxins and autoantigens. Examples of pathogens to which the treatment method can be particularly applied include pathogens for which no effective vaccine is currently available, or pathogens for which conventional vaccines are not fully effective. The pathogens include, but are not limited to, HIV, hepatitis viruses (A, B and C), influenza viruses, herpes viruses, giardia, malaria, leishmania, Staphylococcus aureus, and Pseudomonas aeruginosa.

### Detection

The present disclosure provides a composition for detecting CD40 or FAP, the composition comprising the CD40-binding molecule or the FAP-binding molecule of the present disclosure. The present disclosure further provides a method, system or device for detecting CD40 or FAP *in vivo* or *in vitro,* comprising treating a sample with the CD40-binding molecule or the FAP-binding molecule of the present disclosure.

In some embodiments, the method, system or device for *in vitro* detection may, for example, comprise:
(1) contacting the sample with an antibody that binds to CD40 or FAP;
(2) detecting a complex formed between the antibody that binds to CD40 or FAP and the sample; and/or
(3) contacting a reference sample (e.g., a control sample) with the antibody; and
(4) determining the extent of formation of the complex by comparison with the reference sample. A change (e.g., a statistically significant change) in the formation of the complex in the sample or subject as compared to a control sample or subject indicates the presence of CD40 or FAP in the sample.

In some other embodiments, the method, system or device for *in vivo* detection may comprise:
(1) administering to a subject the CD40-binding molecule or the FAP-binding molecule; and
(2) detecting complex formation between the CD40-binding molecule or the FAP-binding molecule and the subject.

The detection may include determining the location or time at which the complex is formed. The CD40-binding molecule or the FAP-binding molecule may be labeled with a detectable substance, so that the detection can be achieved by detecting the label. Suitable detectable substances include various enzymes, prosthetic groups, fluorescent substances, luminescent substances, and radioactive substances. Complex formation between the CD40-binding molecule and CD40, or the FAP-binding molecule and FAP, can be detected by measuring or visualizing the antibody that binds to or does not bind to CD40 or FAP. Conventional detection assays may be used, such as enzyme-linked immunosorbent assay (ELISA), radioimmunoassay (RIA) or tissue immunohistochemistry. For detection purposes, the CD40-binding molecule or the FAP-binding molecule of the present disclosure may be labeled with a fluorophore chromophore.

In some embodiments, a kit is further provided, the kit comprising the CD40-binding molecule or the FAP-binding molecule, and may further comprise instructions for diagnostic use. The kit may further comprise at least one additional reagent, such as a label or an additional diagnostic agent. For *in-vivo* use, the CD40-binding molecule or the FAP-binding molecule may be formulated into a pharmaceutical composition.

### Definitions

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains. Unless the context clearly requires otherwise, throughout the description and the claims, the words "comprise", "have", "include", and the like are to be construed in an inclusive sense as opposed to an exclusive or exhaustive sense; that is to say, in the sense of "including, but not limited to". "Optional" or "optionally" means that the event or circumstance subsequently described may, but does not necessarily, occur, and that the description includes instances where the event or circumstance occurs or does not occur. The three-letter and single-letter codes for amino acids used herein are as described in J. biol. chem, 243, p3558 (1968).

"CD40" and "CD40 antigen" refer to an approximately 48 kD glycoprotein expressed on the surface of normal and neoplastic B cells, which acts as a receptor for signals involved in cellular proliferation and differentiation (Ledbetter et al., 1987, J. Immunol. 138:788-785). A cell that endogenously expresses CD40 is any cell characterized by the surface expression of CD40, including but not limited to, normal and neoplastic B cells, interdigitating cells, basal epithelial cells, carcinoma cells, macrophages, endothelial cells, follicular dendritic cells, tonsil cells, and bone marrow-derived plasma cells. In the present disclosure, "CD40" refers to any native CD40 derived from any vertebrate, including mammals such as primates (e.g., humans) and rodents (e.g., mice and rats), unless otherwise indicated. A cDNA molecule encoding CD40 has been isolated from a library prepared from the Burkitt lymphoma cell line Raji (Stamenkovic et al., 1989, EMBO J. 8:1403). Sequence information can be found in Table 8 of the present disclosure. In the present disclosure, "CD40" encompasses full-length unprocessed CD40 as well as any form of CD40 that results from processing in a cell, and further encompasses naturally occurring variants of CD40, such as splice variants or allelic variants. In one embodiment, the CD40-binding molecule of the present disclosure is capable of specifically binding to human, mouse and/or cynomolgus monkey CD40.

"Fibroblast activation protein (FAP)" and "FAP antigen", also known as prolyl endopeptidase FAP or seprase (EC 3.4.21), refer to any native FAP derived from any vertebrate, including mammals, such as primates (e.g., humans), non-human primates (e.g., cynomolgus monkeys) and rodents (e.g., mice and rats), unless otherwise indicated. In the present disclosure, "FAP" encompasses full-length unprocessed FAP as well as any form of FAP that results from processing in a cell, and further encompasses naturally occurring variants of FAP, such as splice variants or allelic variants. In one embodiment, the FAP-binding molecule of the present disclosure is capable of specifically binding to human, mouse and/or cynomolgus monkey FAP. The amino acid sequence of human FAP is shown under UniProt (www.uniprot.org) accession No. Q12884 (version 149, SEQ ID NO: 2), or NCBI (www.ncbi.nlm.nih.gov/) RefSeq NP_004451.2, or GeneBank accession No. AAC51668. The extracellular domain (ECD) of human FAP extends from amino acid position 26 to 760. Amino acid sequences such as the ECD of His-tagged human FAP are shown in Table 2 of the present disclosure. The amino acid sequence of mouse FAP is shown under UniProt accession No. P97321 (version 126, SEQ ID NO: 143), or NCBI RefSeq NP_032012.1. The extracellular domain (ECD) of mouse FAP extends from amino acid position 26 to 761. In some embodiments, the FAP-binding molecule of the present disclosure binds to the extracellular domain of FAP.

"Antibody" is used in the broadest sense and encompasses a variety of antibody structures, including, but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) so long as they exhibit the desired antigen-binding activity. An antibody may refer to an immunoglobulin, a tetrapeptide chain structure formed by linking two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, or isotypes of immunoglobulins, namely IgM, IgD, IgG, IgA and IgE, with their corresponding heavy chains being µ chain, δ chain, γ chain, α chain and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3 and IgG4. Light chains are divided into κ or λ chains according to differences in the constant regions. Each of the five classes of Ig may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDR regions and 4 FR regions arranged from the amino terminus to the carboxyl terminus in the following order: FR1, CDR1, FR2, CDR2, FR3, CDR3, and FR4. The 3 CDR regions of the light chain refer to LCDR1, LCDR2, and LCDR3; and the 3 CDR regions of the heavy chain refer to HCDR1, HCDR2, and HCDR3.

"Bispecific antibody" encompasses antibodies (including antibodies or antigen-binding fragments thereof, such as single-chain antibodies) capable of specifically binding to two different antigens or at least two different epitopes of the same antigen. Typical structural models of bispecific antibodies include bispecific antibodies such as KiH, CrossMAb, Triomab quadroma, FcΔAdp, ART-Ig, BiMAb, Biclonics, BEAT, DuoBody, Azymetric, XmAb, 2:1 TCBs, 1Fab-IgG TDB, FynomAb, two-in-one/DAF, scFv-Fab-IgG, DART-Fc, LP-DART, CODV-Fab-TL, HLE-BiTE, F(ab)2-CrossMAb, IgG-(scFv)2, Bs4Ab, DVD-Ig, Tetravalent-DART-Fc, (scFv)4-Fc, CODV-Ig, mAb2, and F(ab)4-CrossMAb (see Aran F. Labrijn et al., Nature Reviews Drug Discovery, volume 18, pages 585-608 (2019); Chen S1 et al., J Immunol Res., Feb. 11, 2019; 2019: 4516041).

For the determination or definition of "CDRs", the deterministic depiction of CDRs and identification of residues comprising antigen-binding sites of the antibody can be accomplished by resolving the structure of the antibody and/or resolving the structure of the antibody-ligand complex. This can be accomplished by any of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition. The Kabat numbering scheme is a standard for numbering residues in antibodies and is generally used to identify CDR regions (see, e.g., Johnson & Wu, 2000, Nucleic Acids Res., 28:214-8). The Chothia numbering scheme is similar to the Kabat numbering scheme, except that it takes into account the position of certain structural loop regions. (see, e.g., Chothia et al., 1986, J. Mol. Biol., 196:901-17; Chothia et al., 1989, Nature, 342:877-83). The AbM numbering scheme adopts a computer program integration suite for modeling antibody structures manufactured by Oxford Molecular Group (see, e.g., Martin et al., 1989, Proc Natl Acad Sci (USA), 86:9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198). The contact definition is based on the analysis of the available complex crystal structures (see, e.g., MacCallum et al., 1996, J. Mol. Biol., 5:732-45). In the conformational definition, the positions of the CDRs can be identified as residues that contribute enthalpy to the antigen binding (see, e.g., Makabe et al., 2008, Journal of Biological Chemistry, 283:1156-1166). In addition, other CDR boundary definitions may not strictly follow one of the above methods, but still overlap with at least a portion of the Kabat CDRs, although they may be shortened or lengthened based on predictions or experimental results that a particular residue or a particular group of residues do not significantly affect the antigen binding. As used in the present disclosure, a CDR may refer to a CDR defined by any method known in the art, including combinations of methods. The correspondence between the various numbering schemes is well known to those skilled in the art, and examples are shown in Table 1 below.

**Table 1. Relationships among CDR numbering schemes**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

The CDR amino acid residues of the VL and VH regions of the antibody of the present disclosure accord with the known Kabat numbering scheme in terms of number and positions.

"Domain" of a polypeptide or protein refers to a folded protein structure that is capable of maintaining its tertiary structure independently of the rest of the protein. In general, a domain is responsible for a single functional property of a protein, and in many cases may be added, removed or transferred to other proteins without loss of functions of the rest of the protein and/or the domain.

"Immunoglobulin domain" refers to a globular region of an antibody chain (e.g., a chain of a conventional antibody with a tetrapeptide chain structure or a heavy-chain antibody) or a polypeptide essentially consisting of such globular regions. The immunoglobulin domain is characterized in that it retains the immunoglobulin fold characteristic of an antibody molecule, and it consists of a 2-layer sandwich of about 7 antiparallel β-strands arranged in two β-sheets, optionally stabilized by a conserved disulfide bond.

"Immunoglobulin variable domain" refers to an immunoglobulin domain essentially consisting of four "framework regions" referred to in the art and hereinafter as "framework region 1" or "FR1", "framework region 2" or "FR2", "framework region 3" or "FR3", and "framework region 4" or "FR4", wherein the framework regions are interrupted by three "complementarity-determining regions" or "CDRs" referred to in the art and hereinafter as "complementarity-determining region 1" or "CDR1", "complementarity-determining region 2" or "CDR2" and "complementarity-determining region 3" or "CDR3". Thus, the general structure or sequence of an immunoglobulin variable domain can be expressed as follows: FR1-CDR1-FR2-CDR2-FR3-CDR3-FR4. Immunoglobulin variable domains possess specificity for an antigen by virtue of having an antigen-binding site.

"Immunoglobulin variable domain" refers to an immunoglobulin domain essentially consisting of four "framework regions" referred to in the art and hereinafter as "framework region 1" or "FR1", "framework region 2" or "FR2", "framework region 3" or "FR3", and "framework region 4" or "FR4", wherein the framework regions are interrupted by three "complementarity-determining regions" or "CDRs" referred to in the art and hereinafter as "complementarity-determining region 1" or "CDR1", "complementarity-determining region 2" or "CDR2" and "complementarity-determining region 3" or "CDR3". Thus, the general structure or sequence of an immunoglobulin variable domain can be expressed as follows: FR1-CDR1-FR2-CDR2-FR3-CDR3-FR4. Immunoglobulin variable domains possess specificity for an antigen by virtue of having an antigen-binding site.

"Antibody framework (FR)" refers to a portion of a variable domain, which serves as a framework for the antigen-binding loops (CDRs) of the variable domain.

"Immunoglobulin single variable domain" is generally used to refer to an immunoglobulin variable domain (which may be a heavy or light chain domain, including a VH, VHH or VL domain) that can form a functional antigen-binding site without interacting with other variable domains (e.g., without VH/VL interactions as are required between the VH and VL domains of conventional four-chain monoclonal antibodies). Examples of "immunoglobulin single variable domains" include nanobodies (including a VHH, humanized VHH and/or camelized VH, e.g. a camelized human VH), IgNAR, domains, (single-domain) antibodies as VH domains or derived from VH domains (such as dAbs^{™}) and (single-domain) antibodies as VL domains or derived from VL domains (such as dAbs^{™}). Immunoglobulin single variable domains based on and/or derived from heavy chain variable domains (such as VH or VHH domains) are generally preferred. A specific example of an immunoglobulin single variable domain is a "VHH domain" (or abbreviated as "VHH") as defined below.

"VHH domain", also known as heavy chain single-domain antibody, VHH, V_{H}H domain, VHH antibody fragment, VHH antibody or nanobody, is a variable domain of an antigen-binding immunoglobulin known as a "heavy-chain antibody" (i.e., "an antibody devoid of light chains") (Hamers-Casterman C, Atarhouch T, Muyldermans S, Robinson G, Hamers C, Songa EB, Bendahman N, Hamers R., "Naturally occurring antibodies devoid of light chains"; Nature 363, 446-448 (1993)). The term "VHH domain" is used to distinguish the variable domain from the heavy chain variable domain (which is referred to in the present disclosure as a "VH domain") and the light chain variable domain (which is referred to in the present disclosure as a "VL domain") present in conventional antibodies with a tetrapeptide chain structure. VHH domains specifically bind to an epitope without the need for an additional antigen-binding domain (as opposed to the VH or VL domain in conventional antibodies with a tetrapeptide chain structure, in which case the epitope is recognized by the VL domain together with the VH domain). The VHH domain is a small, stable and efficient antigen recognition unit formed by a single immunoglobulin domain. The terms "heavy-chain single-domain antibody", "VHH domain", "VHH", "V_{H}H domain", "VHH antibody fragment", "VHH antibody", "Nanobody^{®}" and "Nanobody^{®} domain" ("Nanobody" is a trademark of Ablynx N.V, Ghent, Belgium) are used interchangeably. "VHH domains" include, but are not limited to, natural antibodies produced by camelids, antibodies produced by camelids and then humanized, or antibodies obtained by screening with phage display techniques. The total number of amino acid residues in a VHH domain will usually be in the range of 110 to 120, often between 112 and 115. However, it should be noted that smaller and longer sequences may also be suitable for the purposes described in the present disclosure. Methods for obtaining VHHs that bind to a particular antigen or epitope have been previously disclosed in the following documents: R. van der Linden et al., Journal of Immunological Methods, 240 (2000) 185-195; Li et al., J Biol Chem., 287 (2012)13713-13721; Deffar et al., African Journal of Biotechnology Vol. 8 (12), pp.2645-2652, 17 June, 2009 and WO94/04678.

As is well known in the art for VH domains and for VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, generally, the numbering according to Kabat may or may not correspond to the actual numbering of the amino acid residues in the actual sequence. Other numbering systems or numbering schemes include Chothia, IMGT and AbM.

"Humanized antibody", also known as CDR-grafted antibody, refers to an antibody produced by grafting non-human CDR sequences into the framework of variable regions of a human antibody. Such an antibody can overcome the strong immune response induced by the chimeric antibody because of carrying a large number of non-human protein components. To avoid the decrease in activity caused by the decrease in immunogenicity, the variable regions of a fully human antibody can be subjected to minimum reverse mutation to maintain activity. Examples of "humanization" include "humanization" of VHH domains derived from camelidae by replacing one or more amino acid residues in the amino acid sequence of the original VHH sequence with one or more amino acid residues present at the corresponding positions in a VH domain of a human conventional antibody with a tetrapeptide chain structure (also referred to in the present disclosure as "sequence optimization"; in addition to humanization, "sequence optimization" may also encompass other modifications to the sequence by one or more mutations providing improved properties of the VHH, such as removal of potential post-translational modification sites). The humanized VHH domain may contain one or more fully human framework region sequences. In addition, in order to avoid the decrease in activity caused by the decrease in immunogenicity, the framework region sequence in the human antibody variable region can be subjected to minimum reverse mutation or back mutation to maintain activity.

"Fully human antibody" includes antibodies having variable and constant regions derived from human germline immunoglobulin sequences. The fully human antibody of the present disclosure may include amino acid residues not encoded by human germline immunoglobulin sequences (e.g., mutations introduced by random or site-specific mutagenesis *in vitro* or by somatic mutations *in vivo*)*.* "Fully human antibody" does not include "humanized antibodies".

Typically, the CD40-binding molecule or the FAP-binding molecule of the present disclosure will bind to the antigen to be bound (i.e., CD40 or FAP) with a dissociation constant (K_{D}) of preferably 10⁻⁷ to 10⁻¹⁰ mol/L (M), more preferably 10⁻⁸ to 10⁻¹⁰ mol/L, even more preferably 10⁻⁹ to 10⁻¹⁰ or less, and/or with an association constant (KA) of at least 10⁻⁷ M, preferably at least 10⁻⁸ M, more preferably at least 10⁻⁹ M or more preferably at least 10⁻¹⁰ M, as measured in a Biacore, KinExA or Fortibio assay. Any K_{D} value greater than 10⁻⁴M is generally considered to indicate non-specific binding. Specific binding of an antigen-binding protein to an antigen or epitope can be measured in any suitable manner known, including, for example, surface plasmon resonance (SPR) assays, Scatchard assay, and/or competitive binding assay (e.g., radioimmunoassay (RIA), enzyme immunoassay (EIA), and sandwich competitive assay) described in the present disclosure.

"Compete", when used in a case where antigen-binding proteins (e.g., neutralizing antigen-binding proteins or neutralizing antibodies) compete for the same epitope, refers to the competition between the antigen-binding proteins, which is measured by the following assays in which a test antigen-binding protein (e.g., an antibody or an immunologically functional fragment thereof) prevents or inhibits (e.g., reduces) specific binding of a reference antigen-binding protein (e.g., a ligand or a reference antibody) to a common antigen (e.g., CD40 or a fragment thereof). Numerous types of competitive binding assays are available for determining whether an antigen-binding protein competes with another, such as solid phase direct or indirect radioimmunoassay (RIA), solid phase direct or indirect enzyme immunoassay (EIA), and sandwich competition assay (see, e.g., Stahli et al., 1983, Methods in Enzymology 9: 242-253); solid phase direct biotin-avidin EIA (see, e.g., Kirkland et al., 1986, J. Immunol. 137: 3614-3619), solid phase direct labeled assay, and solid phase direct labeled sandwich assay (see, e.g., Harlow and Lane, 1988, Antibodies: A Laboratory Manual, Cold Spring Harbor Press); solid phase direct labeled RIA with I-125 label (see, e.g., Morel et al., 1988, Molec. Immunol. 25: 7-15); solid phase direct biotin-avidin EIA (see, e.g., Cheung, et al., 1990, Virology 176: 546-552) and direct labeled RIA (Moldenhauer et al., 1990, Scand. J. Immunol. 32: 77-82). Generally, the assay relates to the use of a purified antigen (which is on a solid surface or cell surface) that binds to an unlabeled detection antigen-binding protein and a labeled reference antigen-binding protein. Competitive inhibition is measured by measuring the amount of label bound to the solid surface or the cell in the presence of the test antigen-binding protein. Generally, the test antigen-binding protein exists in an excessive amount. Antigen-binding proteins identified by competitive assays (competitive antigen-binding proteins) include: antigen-binding proteins that bind to the same epitope as the reference antigen-binding protein; and antigen-binding proteins that bind to an epitope that is sufficiently close to the epitope to which the reference antigen-binding protein binds, and the two epitopes spatially hinder each other from binding. Generally, when the competitive antigen-binding protein exists in an excessive amount, the specific binding of the reference antigen-binding protein to the common antigen will be inhibited (e.g., reduced) by at least 40%-45%, 45%-50%, 50%-55%, 55%-60%, 60%-65%, 65%-70%, 70%-75% or 75% or more. In certain instances, the binding is inhibited by at least 80%-85%, 85%-90%, 90%-95%, 95%-97% or 97% or more.

Antibodies can be competitively screened for binding to the same epitope using conventional techniques known to those skilled in the art. For example, competition and cross-competition studies can be performed to obtain antibodies that compete or cross-compete with one another for binding to an antigen. A high-throughput method for obtaining antibodies that bind to the same epitope based on their cross-competition is described in International Patent Publication No. WO03/48731. Therefore, antibodies that compete for binding to the same epitope on CD40 or FAP with the antibody molecules of the present disclosure can be obtained by conventional techniques known to those skilled in the art.

"CD40-binding molecule" refers to any protein capable of specifically binding to CD40 or any molecule comprising the protein. The CD40-binding molecule may include antibodies against CD40 as defined in the present disclosure or conjugates thereof. The CD40-binding molecule further encompasses immunoglobulin superfamily antibodies (IgSF) or CDR-grafted molecules. The "CD40-binding molecule" of the present disclosure may comprise at least one immunoglobulin single variable domain (such as a VHH) that binds to CD40. In some embodiments, the "CD40-binding molecule" may comprise 2, 3, 4 or more immunoglobulin single variable domains (such as VHHs) that bind to CD40. The CD40-binding molecule of the present disclosure may further comprise, in addition to the immunoglobulin single variable domain of CD40, a linker and/or a moiety with effector function, such as a half-life extending moiety (e.g., an immunoglobulin single variable domain that binds to serum albumin), and/or a fusion partner (such as serum albumin) and/or a conjugated polymer (such as PEG) and/or an Fc region. In some embodiments, the "CD40-binding molecule" of the present disclosure further encompasses bispecific/multi-specific antibodies comprising immunoglobulins that bind to different antigens (e.g., a first antibody that binds to a first antigen (e.g., CD40) and a second antibody that binds to a second antigen (e.g., FAP), optionally a third antibody that binds to a third antigen, and further optionally a fourth antibody that binds to a fourth antigen).

"FAP-binding molecule" refers to any protein capable of specifically binding to FAP or any molecule comprising the protein. The FAP-binding molecule may include antibodies against FAP as defined in the present disclosure or conjugates thereof.

"FAP/CD40-binding protein" refers to any protein capable of specifically binding to CD40 and FAP or any molecule comprising the protein. The FAP/CD40-binding molecule may include antibodies against CD40 and FAP as defined in the present disclosure or conjugates thereof.

"Bind to CD40" or "bind CD40" refers to being able to interact with CD40 or an epitope thereof, wherein the CD40 or the epitope thereof may be derived from humans. "Bind to FAP" or "bind FAP" refers to being able to interact with FAP or an epitope thereof, wherein the FAP or the epitope thereof may be derived from humans. The "antigen-binding site" in the present disclosure refers to a discontinuous three-dimensional spatial site on an antigen that is recognized by the antibody of the present disclosure.

"Antigen" refers to a molecule used for immunization of an immunocompetent vertebrate to produce an antibody that recognizes the antigen or to screen an expression library (e.g., particularly phage, yeast or ribosome display library). Herein, the antigen is defined in a broader sense, and includes a target molecule that is specifically recognized by the antibody, and a portion or a mimic of a molecule used in an immunization process for producing the antibody or in library screening for selecting the antibody. For example, for the antibody of the present disclosure that binds to human CD40, monomers and multimers (e.g., dimers, trimers, etc.) of human CD40, and truncated variants and other variants of human CD40 are all referred to as antigens.

"Epitope" refers to a site on an antigen to which an immunoglobulin or an antibody binds. An epitope may be formed from contiguous amino acids, or non-contiguous amino acids juxtaposed by tertiary folding of the protein. An epitope formed from contiguous amino acids is generally retained after exposure to a denaturing solvent, while an epitope formed by tertiary folding is generally lost after a denaturing solvent treatment. An epitope generally comprises, for example, at least 3-15 amino acids in a unique spatial conformation. Methods for determining what epitope is bound by a given antibody are well known in the art and include an immunoblotting assay, an immunoprecipitation assay, and the like. Methods for determining the spatial conformation of an epitope include techniques in the art and techniques described in the present disclosure, such as X-ray crystallography and two-dimensional nuclear magnetic resonance.

"Specific binding" or "selective binding" refers to binding of an antibody to an epitope on a predetermined antigen. For example, an antibody binds to a predetermined antigen or epitope thereof with an equilibrium dissociation constant (K_{D}) of about less than 10⁻⁷ M or even less and with an affinity that is at least twice as high as its affinity for binding to a non-specific antigen other than the predetermined antigen or the epitope thereof (or non-specific antigens other than closely related antigens, e.g., BSA, etc.), when measured by surface plasmon resonance (SPR) techniques in an instrument using human CD40 or an epitope thereof as an analyte and the antibody as a ligand. "Antigen-recognizing antibody" is used interchangeably in the present disclosure with "specifically bound antibody".

"Binding affinity" or "affinity" is used in the present disclosure as a measure of the strength of a non-covalent interaction between two molecules (e.g., an antibody or a portion thereof and an antigen). The binding affinity between two molecules can be quantified by determining the dissociation constant (KD). KD can be determined by measuring the kinetics of complex formation and dissociation using, for example, the surface plasmon resonance (SPR) method (Biacore). The rate constants corresponding to the association and dissociation of a monovalent complex are referred to as the association rate constant ka (or kon) and the dissociation rate constant kd (or koff), respectively. K_{D} is related to ka and kd by the equation K_{D} = kd/ka. The value of the dissociation constant can be determined directly by well-known methods and can be calculated by methods such as those described by Caceci et al (1984, Byte 9:340-362) even for complex mixtures. For example, K_{D} can be determined by using a dual filtration nitrocellulose filter binding assay such as that disclosed by Wong & Lohman (1993, Proc. Natl. Acad. Sci. USA 90:5428-5432). Other standard assays for evaluating the binding ability of an antibody to a target antigen are known in the art and include, for example, ELISA, western blot, RIA and flow cytometry, as well as other assays exemplified elsewhere in the present disclosure. The binding kinetics and binding affinity of the antibody can also be evaluated by standard assays known in the art, such as surface plasmon resonance (SPR), for example, by using the Biacore^{™} system or KinExA. The binding affinities associated with different molecular interactions, e.g., the binding affinities of different antibodies for a given antigen, can be compared by comparing the K_{D} values of antibody/antigen complexes. Similarly, the specificity of an interaction can be evaluated by determining and comparing the K_{D} value for the interaction of interest (e.g., a specific interaction between an antibody and an antigen) with the K_{D} value for an interaction not of interest (e.g., a control antibody known not to bind to CD40).

"Conservative substitution" refers to a substitution to another amino acid residue having similar properties to the original amino acid residue. For example, lysine, arginine and histidine have similar properties in that they have basic side chains, and aspartic acid and glutamic acid have similar properties in that they have acidic side chains. In addition, glycine, asparagine, glutamine, serine, threonine, tyrosine, cysteine and tryptophan have similar properties in that they have uncharged polar side chains, and alanine, valine, leucine, threonine, isoleucine, proline, phenylalanine and methionine have similar properties in that they have nonpolar side chains. In addition, tyrosine, phenylalanine, tryptophan and histidine have similar properties in that they have aromatic side chains. Thus, it will be apparent to those skilled in the art that even when an amino acid residue in a group exhibiting similar properties as described above is replaced, it will not exhibit a particular change in properties.

"Homology", "identity" or "sequence identity" refers to sequence similarity between two polynucleotide sequences or between two polypeptides. When positions in two compared sequences are occupied by identical nucleotides or amino acid monomers, e.g., if the position of each of two DNA molecules is occupied by an identical nucleotide, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. Generally, when two sequences are aligned, comparison is performed to obtain the maximum homology percentage.

"Host cell" includes individual cells or cell cultures which may be or have been the recipient of a vector for incorporation of a polynucleotide insert. The host cell includes progeny of a single host cell, and the progeny may not necessarily be completely identical (in morphology or genomic DNA complement) to the original parent cell due to natural, accidental or deliberate mutations. The host cell includes cells transfected and/or transformed *in vivo* with polynucleotides of the present disclosure. "Cell", "cell line", and "cell culture" are used interchangeably, and all such designations include their progenies. It should also be understood that all progenies may not be precisely identical in DNA content due to intentional or unintentional mutations. Mutant progeny with identical function or biological activity as screened in the original transformed cells is included.

The terms "inhibition" and "blocking" are used interchangeably and encompass both partial and complete inhibition/blocking. "Inhibition of growth" (e.g., involving cells) is intended to include any measurable reduction in cell growth.

"Agonistic activity", "agonist activity" or "agonism" refers to the function of a substance as an agonist. The binding of an agonist to a cell receptor initiates a reaction or activity that is similar to or the same as that initiated by the receptor's natural ligand. For example, a CD40 agonist or CD40 agonist antibody can induce any or all of the following responses: cell proliferation and/or differentiation; up-regulation of intercellular adhesion via such molecules as ICAM-1, E-selectin and VCAM; secretion of pro-inflammatory cytokines such as IL-1, IL-6, IL-8, IL-12 and TNF; signal transduction through the CD40 receptor by such pathways as TRAF (e.g., TRAF2 and/or TRAF3), MAP kinases such as NIK (NF-κB inducing kinase), 1-κB kinases (IKKα/β), transcription factor NF-κB, Ras and the MEK/ERK pathway, the PI3K/Akt pathway, and the P38 MAPK pathway; transduction of anti-apoptotic signals by such molecules as XIAP, Mcl-1 and BCLx; B and/or T cell memory generation: B cell antibody production; B cell isotype switching; up-regulation of cell surface expression of MHC class II and CD80/86, and the like.

"CD40-related disease" or "CD40-related condition" refers to a condition in which cells expressing CD40 are modified or eliminated. These cells include CD40-expressing cells demonstrating abnormal proliferation or CD40-expressing cells that are associated with cancerous or malignant growth. More specific examples of cancers that demonstrate abnormal expression of the CD40 antigen include B lymphoblastoid cells, Burkitt's lymphoma, multiple myeloma, T cell lymphomas, Kaposi's sarcoma, osteosarcoma, epidermal and endothelial tumors, pancreatic cancer, lung cancer, breast cancer, ovarian cancer, colon cancer, prostate cancer, head and neck cancer, skin cancer (melanoma), bladder cancer, and kidney cancer. Such disorders include, but are not limited to, leukemias, lymphomas (including B cell lymphoma and non-Hodgkin lymphoma), multiple myeloma, Waldenstrom's macroglobulinemia; solid tumors, including sarcomas, such as osteosarcoma, Ewing's sarcoma, malignant melanoma, adenocarcinoma (including ovarian adenocarcinoma), Kaposi's sarcoma/Kaposi's tumor and squamous cell carcinoma.

"Proliferative disease" refers to a condition associated with a certain degree of abnormal cell proliferation. In one embodiment, the proliferative condition is cancer.

"Cancer", "cancerous", "proliferative condition" and "tumor" are not mutually exclusive when referred to in the present disclosure.

"Preventing cancer" or "prevention of cancer" refers to delaying, inhibiting or preventing the onset of cancer in a subject in which the onset of oncogenesis or tumorigenesis has not been evidenced but a predisposition for cancer has been identified, as determined by, for example, genetic screening or otherwise. The term also encompasses treating a subject having premalignant conditions to stop the progression of, or cause regression of, the premalignant conditions towards malignancy.

"Giving", "administering" and "treating", when applied to animals, humans, experimental subjects, cells, tissues, organs or biological fluid, refer to contact of an exogenous drug, a therapeutic agent, a diagnostic agent or a composition with the animals, humans, subjects, cells, tissues, organs or biological fluid, e.g., therapeutic, pharmacokinetic, diagnostic, research, and experimental methods. The treatment of cells comprises contacting the reagent with the cells and contacting the reagent with fluid, where the fluid is in contact with the cells. "Giving", "administering", and "treating" also refer to treating, e.g., a cell, by a reagent, diagnosis, a binding composition, or by another cell *in vitro* and *ex vivo.* When applied to humans, veterinary or research subjects, they refer to therapeutic treatment, preventive or prophylactic measures, and research and diagnostic applications.

"Treating" or "treatment" refers to administering a therapeutic agent, such as a therapeutic agent comprising any antibody of the present disclosure or a pharmaceutical composition thereof, either internally or externally, to a subject who has had, is suspected of having, or is predisposed to having one or more proliferative diseases or symptoms thereof on which the therapeutic agent is known to have a therapeutic effect. Typically, the therapeutic agent is administered in an amount effective to alleviate one or more symptoms of the disease in the subject or population being treated, whether by inducing regression of such symptoms or inhibiting the development of such symptoms into any clinically measurable degree. The amount of therapeutic agent effective to alleviate any particular symptom of the disease (also referred to as the "therapeutically effective amount") may vary depending on factors such as the disease state, age and weight of the subject, and the ability of the drug to produce a desired therapeutic effect in the subject. Whether a symptom of a disease has been alleviated can be evaluated by any clinical testing methods commonly used by doctors or other health care professionals to evaluate the severity or progression of the symptom. Although embodiments of the present disclosure (e.g., treatment methods or articles of manufacture) may be ineffective in alleviating symptoms of a disease of interest in a certain subject, they shall alleviate the symptoms of the disease of interest in a statistically significant number of subjects as determined by any statistical test method known in the art, such as the Student's t-test, Chi-square test, U-test by Mann and Whitney, Kruskal-Wallis test (H-test), Jonckheere-Terpstra test and Wilcoxon test.

"Effective amount" comprises an amount sufficient to ameliorate or prevent a symptom or sign of a medical condition. Effective amount also refers to an amount sufficient to allow or facilitate diagnosis. The effective amount for a subject may vary depending on factors such as the condition to be treated, the general health of the subject, the method and dose of administration, and the severity of side effects. Effective amount may be the maximum dose or administration regimen to avoid significant side effects or toxic effects. The subject of the present disclosure may be an animal or a human subject.

The "subject" or "patient" in the present disclosure refers to a mammal, particularly a primate, and particularly a human.

### BRIEF DESCRIPTION OF THE DRAWINGS

FIG. 1 shows the binding of the anti-FAP antibodies Ab9, Ab 10, Ab 14 and Ab 15 to cell surface human FAP, with 28H1 as a positive control and the IgG1 isotype as a negative control.
FIG. 2 shows the binding of the anti-FAP antibodies Ab9, Ab 10, Ab 14 and Ab 15 to cell surface mouse FAP, with 28H1 as a positive control and the IgG1 isotype as a negative control.
FIG. 3 shows the binding of the anti-CD40 antibodies A12 and A297 to Raji cell surface human CD40, with 9E5-SELFNS as a positive control and IgG1 as a negative control.
FIG. 4 shows the binding of the anti-CD40 antibodies A12 and A297 to HEK293 cell surface human CD40, with 9E5-SELFNS as a positive control and IgG1 as a negative control; the ordinate represents the percent agonist activity relative to 200 nM 9E5-SELFNS.
FIG. 5A shows the affinities of the humanized anti-CD40 antibodies A12_V1, A12_V2 and A12_V3 for HEK293 cells overexpressing human CD40, as measured by flow cytometry. FIG. 5B shows the affinities of the humanized anti-CD40 antibodies A297_V1, A297_V2, A297_V3 and A297_V4 for HEK293 cells overexpressing human CD40, as measured by flow cytometry. The analyses all used 9E5-SELFNS as a positive control and hIgG as a negative control.
FIG. 6A shows the FcγRIIb-mediated activation of HEK-Blue^{™}CD40L cells by the humanized anti-CD40 antibodies A12_V1, A12_V2 and A12_V3. FIG. 6B shows the FcyRIIb-mediated activation of HEK-Blue^{™}CD40L cells by the humanized anti-CD40 antibodies A297_V1, A297_V2, A297_V3 and A297_V4. The analyses all used 9E5-SELFNS as a positive control and hIgG as a negative control.
FIG. 7 shows a schematic diagram of the structures of anti-FAP/CD40 bispecific antibodies.
FIG. 8A shows the affinities of the anti-FAP/CD40 bispecific antibodies Ab10-A12V2-2, Ab10-A12V2-4, Ab10-A297V3-2 and Ab10-A297V3-4 for a stably transfected CHOK1/FAP strain highly expressing the human FAP antigen, as measured by flow cytometry. FIGs. 8B and 8C show the affinities of the aforementioned anti-FAP/CD40 bispecific antibodies for stably transfected CHOK1/FAP strains highly expressing the mouse FAP antigen and the cynomolgus monkey FAP antigen, as measured by flow cytometry.
FIG. 9A shows the affinities of the anti-FAP/CD40 bispecific antibodies Ab10-A12V2-2, Ab10-A12V2-4, Ab10-A297V3-2 and Ab10-A297V3-4 for a stably transfected HEK-BlueTMCD40L strain highly expressing the human CD40 antigen, as measured by flow cytometry. FIG. 9B shows the affinities of the aforementioned anti-FAP/CD40 bispecific antibodies for HEK293 cells highly expressing the cynomolgus monkey CD40 antigen, as measured by flow cytometry.
FIG. 10 shows the affinities of the anti-FAP/CD40 bispecific antibodies Ab10-A297V3-2 and Ab10-A297V3-4 for the CD40 on immature human DC, as measured by flow cytometry.
FIG. 11 shows the DC maturation-promoting effect of the anti-FAP/CD40 bispecific antibodies Ab10-A12V2-2, Ab10-A12V2-4, Ab10-A297V3-2 and Ab10-A297V3-4 of a concentration gradient in the absence of crosslinking of stably transfected CHOK1/FAP cells, with LPC, hIgG 1 and the vehicle as controls.
FIG. 12 shows the DC maturation-promoting effect of the anti-FAP/CD40 bispecific antibodies Ab10-A12V2-2, Ab10-A12V2-4, Ab10-A297V3-2 and Ab10-A297V3-4 of a concentration gradient in the presence of crosslinking of stably transfected CHOK1/FAP cells, with LPC, hIgG 1 and the vehicle as controls.
FIG. 13A shows tumor growth curves after single-dose injections of the antibody Ab10-A297V3-2 or Ab10-A297V3-4 into mFAP-MC38 tumor-bearing hCD40 humanized mice. FIG. 13B shows the corresponding peripheral blood B cell activation in the mice. FIG. 13C shows curves of the corresponding changes in the body weight of the mice. FIG. 13D shows the corresponding changes in the platelet counts of the mice.
FIG. 13E shows the corresponding influence on liver function in the mice.
FIG. 14A shows tumor growth curves after multiple-dose injections of the antibody Ab10-A297V3-2 or Ab10-A297V3-4 into mFAP-MC38 tumor-bearing hCD40 humanized mice. FIG. 14B shows the corresponding peripheral blood B cell activation in the mice. FIG. 14C shows curves of the corresponding changes in the body weight of the mice. FIG. 14D shows the corresponding changes in the platelet counts of the mice.
FIG. 14E shows the corresponding influence on liver function in the mice.
FIG. 15A shows tumor growth curves after multiple-dose injections of the antibody Ab10-A12V2-2 into mFAP-MC38 tumor-bearing hCD40 humanized mice. FIG. 15B shows curves of the corresponding changes in the body weight of the mice. FIG. 15C shows the corresponding influence on liver function in the mice. FIG. 15D shows the corresponding changes in the platelet counts of the mice.
FIG. 16A shows tumor growth curves after single-dose injections of Ab10-A12V2-4 into mFAP-MC38 tumor-bearing hCD40 humanized mice. FIG. 16B shows curves of the corresponding changes in the body weight of the mice. FIG. 16C shows the corresponding influence on liver function in the mice. FIG. 16D shows the corresponding influence on the platelet counts of the mice.
FIG. 17 shows a comparison of the pharmacokinetics of the bispecific antibodies Ab10-A12V2-2 and Ab10-A297V3-2.

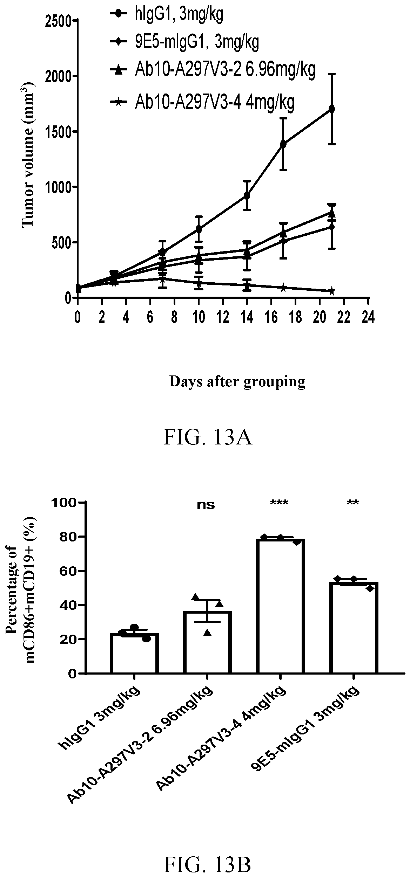

In FIGs. 13A to 17, the P values were calculated relative to the control hIgG 1, unless otherwise indicated. The statistical method Student' t-test was used. ns stands for no significant difference. * represents p < 0.05, ** represents p < 0.01, *** represents p < 0.001, and **** represents p < 0.0001.

### DETAILED DESCRIPTION

The present disclosure is further described below with reference to examples, but these examples are not intended to limit the scope of the present disclosure.

Experimental procedures without specific conditions indicated in the examples or test examples are generally conducted according to conventional conditions, or according to conditions recommended by the manufacturer of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY Reagents for which specific sources are not mentioned are commercially available conventional reagents.

### Example 1. Screening for and Preparation of Anti-FAP Monoclonal Antibodies

### 1.1. Sequences and preparation of screening and detection antigens

Human fibroblast activation protein (FAP; GeneBank accession No. AAC51668) is a serine oligopeptidase with a molecular weight of about 170 kDa and is a homodimer. The sequences, sources and uses of the recombinant FAP proteins used in the present disclosure are shown in Table 2.

**Table 2. The sources of the amino acid sequences of recombinant proteins**

| Name | Start and end of amino acid sequence | Sino Biological cat. No. | Use |
|---|---|---|---|
| h-FAP-biotin | Leu26-Asp760 (GeneBank accession No. AAC51668) | 10464-H07H-B | Phage display liquid-phase magnetic bead screening |
| h-FAP-His | | 10464-H07H | ELISA screening for positive clones |
| h-FAP-FITC | | 10464-H07H-F | CHO cell surface antibody display screening |

### 1.2. Screening for h-FAP-specific binding antibody fragments from human natural Fab phage display library

With the antigen h-FAP-biotin as the target molecule, antigen-specific phages were captured using avidin-coated magnetic beads, and phage enrichment was performed using a magnetic rack. The antigen-specific phages were eluted with a pH 2.2 glycine solution.

After two rounds of screening, 284 clones were randomly selected and screened by phage ELISA for positive clones. The specific steps are as follows: The growing colonies in the dish were counted-there were 284 colonies in total. The phages were inoculated onto a 96-well plate, each well of which contained 400 µL of culture medium (2YT + Amp + 0.2% glucose). The plate was shaken at 37 °C at 250 rpm for 6 h. The plate was coated with the antigen h-FAP-His at 100 ng/100 µL/well and incubated overnight at 4 °C. The next day, after the 96-well plate was washed and blocked, 100 µL of overnight-incubated bacterial liquid was added to each well, and the plate was incubated at 37 °C for 1 h. After a wash, a secondary antibody (an HRP-labeled anti-human IgG-Fab antibody) was added, and the plate was incubated at 37 °C for 40 min. After a wash, the substrate solution was added, and the plate was incubated in the dark for 30 min. Then the OD600 values were measured on a microplate reader. A total of 122 positive clones obtained from two rounds of screening were sequenced, yielding 74 unique VHs (constituting a VH-enriched library), 48 unique κ light chains (constituting a KLC-enriched library), and 10 unique λ light chains (constituting an LLC-enriched library).

### 1.3. Construction of CHO cell surface full-length antibody display library

1) Constructing a full-length antibody CHO cell display gene library, comprising the following steps: The VH-enriched library, the KLC-enriched library and the LLC-enriched library were subjected to PCR amplification with three sets of primers, respectively. The three enriched libraries were digested with enzymes and then inserted into corresponding component vectors to construct corresponding component libraries. The KLC, LLC and VH component libraries were digested with enzymes. After the digestion, the KLC, LLC and VH fragment libraries were analyzed and purified by electrophoresis. The full-length antibody cell display vector was digested with an enzyme, and the fragments of the vector were purified. The purified fragments of the vector and the KLC, LLC and VH fragment libraries were mixed and ligated. The ligation products were purified and transferred into *E. coli* cells by electroporation. The cells were plated on a dish and cultured overnight at 37 °C, and the colonies were counted. The library capacity measured 3.6 × 10E6, which is more than 800 times the theoretical diversity (74 × 58 = 4292). All the colonies were collected, and the vector DNA was extracted, yielding a full-length antibody cell display gene library.
2) Constructing an h-FAP antigen-specific full-length antibody cell display library, comprising the following steps: CHO cells were transformed with the vector DNA of the full-length antibody cell display gene library to construct a full-length antibody CHO cell display library. The cell library was screened under the pressure of hygromycin. A stably transformed cell library was obtained, and the cell library was double-stained with a PE-labeled mouse anti-human κ (or λ) light-chain antibody and an FITC-labeled h-FAP antigen and sorted by FACS for PE and FITC double-positive cells. Each well contained one cell. The κ light chain library sorting used 2 96-well plates, and the λ light chain library sorting used 1 96-well plate. The cells were cultured under the pressure of hygromycin.
3) FACS analysis to identify single-cell clones displaying h-FAP antigen-specific antibodies, comprising the following steps: The cells were cultured under the pressure of hygromycin for 14 days, yielding 153 stably transformed κ chain single-cell clones and 50 λ chain single-cell clones. The cells were digested with a 0.5 mM EDTA-PBS buffer solution, and the single-cell clones were double-stained with a PE-labeled mouse anti-human κ (or λ) light-chain antibody and an FITC-labeled h-FAP antigen. FACS analysis showed that 138 PE and FITC fluorescence double-positive single-cell clones were obtained.
4) Cloning antibody genes, comprising the following steps: The positive clones were assayed for affinity by FACS, and it was decided that 45 clones would be subjected to antibody gene PCR amplification. After centrifugation, the positive clones were collected, and the supernatant was discarded. The genomic DNA was extracted from the cells, and the VH and LC were amplified by PCR. The fragments obtained from the amplification were separated by electrophoresis and sequenced, and 6 unique VHs and 6 unique κ chains were identified. Their combinations can yield 12 clones with unique sequences. The VH and VL sequences of 4 of the clones are shown in Table 3.

**Table 3. The amino acid sequences of the VHs and VLs (κ of KLC) of h-FAP antibodies**

| No. | Amino acid sequence | |
|---|---|---|
| Ab9 | VH | QMQLVQSGAEVKKPGASVTVSCRASGYSFADHFIHWVRQAPGQGFQWM GWINPNRGVTHHAQDFQGRVAMTRDMSTDTVYMELTSLRSDDTAVYYC ARDESLTARPYYFYGFDVWGQGTLVTVSS (SEQ ID NO:1) |
| | VL | DIQMTQSPSSVSASVGDRVTITCRASQGISSWLAWYQQKPGKAPKLLIYA ASSLQSGVPSRFSGSGSGTDFTLTISSLQPEDFATYYCQQANSFPPAFGQGT KVEIK (SEQ ID NO:2) |
| Ab10 | VH | QVQLQQSGVEVKKPGASVTVSCRASGYSFADHFIHWVRQAPGQGFQWM GWINPNRGVTHHAQDFQGRVAMTRDMSTDTVYMELTSLRSDDTAVYYC ARDASLTARPYYFYGFDVWGQGTLVTVSS (SEQ ID NO:3) |
| | VL | DIQMTQSPSSVSASVGDRVTITCRASQGISSWLAWYQQKPGKAPKLLIYA ASSLQSGVPSRFSGSGSGTDFTLTISSLQPEDFATYYCQQANSFPPAFGQGT KVEIK (SEQ ID NO:4) |
| Ab14 | VH | QVQLQQSGVEVKKPGASVTVSCRASGYSFADHFIHWVRQAPGQGFQWM GWINPNRGVTHHAQDFQGRVAMTRDMSTDTVYMELTSLRSDDTAVYYC ARDASLTARPYYFYGFDVWGQGTLV TVSS (SEQ ID NO:5) |
| | VL | EIVLTQSPSSVSASVGDRVTITCRASQGISSWLAWYQQKPGKAPKLLIYAA SSLQSGVPSRFSGSGSGTDFTLTISSLQPEDFATYYCQQANSFPLTFGGGTK VEIK (SEQ ID NO:6) |
| Ab15 | VH | QMQLVQSGAEVKKPGASVTVSCRASGYSFADHFIHWVRQAPGQGFQWM GWINPNRGVTHHAQDFQGRVAMTRDMSTDTVYMELTSLRSDDTAVYYC ARDESLTARPYYFYGFDVWGQGTLVT VSS (SEQ ID NO:7) |
| | VL | EIVLTQSPSSVSASVGDRVTITCRASQGISSWLAWYQQKPGKAPKSLIYAA SSLQSGVPSRFSGSGSGTDFTLTISSLQPEDFATYYCQQANSFPPTFGGGTK VEIK (SEQ ID NO:8) |

| | | |
|---|---|---|
| Note: The CDRs defined by the Kabat numbering scheme are underlined. | | |

The present disclosure further provides the sequences of the full-length heavy and light chains of Ab9, Ab 10, Ab 14 and Ab 15.
> The full-length heavy chain of Ab9 
QMQLVQSGAEVKKPGASVTVSCRASGYSFADHFIHWVRQAPGQGFQWMGWI
NPNRGVTHHAQDFQGRVAMTRDMSTDTVYMELTSLRSDDTAVYYCARDESLTA
RPYYFYGFDVWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK (SEQ ID NO: 49)
> The full-length light chain of Ab9 
DIQMTQSPSSVSASVGDRVTITCRASQGISSWLAWYQQKPGKAPKLLIYAASSLQ
SGVPSRFSGSGSGTDFTLTISSLQPEDFATYYCQQANSFPPAFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC (SEQ
ID NO: 50)
> The full-length heavy chain of Ab10 
QVQLQQSGVEVKKPGASVTVSCRASGYSFADHFIHWVRQAPGQGFQWMGWIN
PNRGVTHHAQDFQGRVAMTRDMSTDTVYMELTSLRSDDTAVYYCARDASLTA
RPYYFYGFDVWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
 
PSNTKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK (SEQ ID NO: 51)
> The full-length light chain of Ab 10 
DIQMTQSPSSVSASVGDRVTITCRASQGISSWLAWYQQKPGKAPKLLIYAASSLQ
SGVPSRFSGSGSGTDFTLTISSLQPEDFATYYCQQANSFPPAFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC (SEQ
ID NO: 52)
> The full-length heavy chain of Ab14 
QVQLQQSGVEVKKPGASVTVSCRASGYSFADHFIHWVRQAPGQGFQWMGWIN
PNRGVTHHAQDFQGRVAMTRDMSTDTVYMELTSLRSDDTAVYYCARDASLTA
RPYYFYGFDVWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK (SEQ ID NO: 53)
> The full-length light chain of Ab 14 
EIVLTQSPSSVSASVGDRVTITCRASQGISSWLAWYQQKPGKAPKLLIYAASSLQ
SGVPSRFSGSGSGTDFTLTISSLQPEDFATYYCQQANSFPLTFGGGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC (SEQ
ID NO: 54)
> The full-length heavy chain of Ab15 
QMQLVQSGAEVKKPGASVTVSCRASGYSFADHFIHWVRQAPGQGFQWMGWI
NPNRGVTHHAQDFQGRVAMTRDMSTDTVYMELTSLRSDDTAVYYCARDESLTA
RPYYFYGFDVWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGK (SEQ ID NO: 55)
> The full-length light chain of Ab 15

EIVLTQSPSSVSASVGDRVTITCRASQGISSWLAWYQQKPGKAPKSLIYAASSLQS
GVPSRFSGSGSGTDFTLTISSLQPEDFATYYCQQANSFPPTFGGGTKVEIKRTVAA
PSVFIFPPSDEQLKSGTASVVCLLNNFYPREAKVQWKVDNALQSGNSQESVTEQ

DSKDSTYSLSSTLTLSKADYEKHKVYACEVTHQGLSSPVTKSFNRGEC (SEQ ID
NO: 56)

**Table 4. The CDRs of anti-FAP antibodies**

| No. | CDRs of VH | | CDRs of VL | |
|---|---|---|---|---|
| Ab9 | HCDR1 | DHFIH (SEQ ID NO:9) | LCDR1 | RASQGISSWLA (SEQ ID NO:12) |
| | HCDR2 | WINPNRGVTHRAQDFQG (SEQ ID NO:10) | LCDR2 | AASSLQ (SEQ ID NO:13) |
| | HCDR3 | DESLTARPYYFYGFDV (SEQ ID NO:11) | LCDR3 | QQANSFPPA (SEQ ID NO:14) |
| Ab10 | HCDR1 | SEQ ID NO:9 | LCDR1 | SEQ ID NO:12 |
| | HCDR2 | SEQ ID NO:10 | LCDR2 | SEQ ID NO:13 |
| | HCDR3 | DASLTARPYYFYGFDV (SEQ ID NO:15) | LCDR3 | SEQIDNO:14 |
| Ab14 | HCDR1 | SEQ ID NO:9 | LCDR1 | SEQ ID NO:12 |
| | HCDR2 | SEQ ID NO:10 | LCDR2 | SEQ ID NO:13 |
| | HCDR3 | SEQ ID NO:15 | LCDR3 | QQANSFPLT (SEQ ID NO:16) |
| Ab15 | HCDR1 | SEQ ID NO:9 | LCDR1 | SEQ ID NO:12 |
| | HCDR2 | SEQ ID NO:10 | LCDR2 | SEQ ID NO:13 |
| | HCDR3 | SEQ ID NO:11 | LCDR3 | QQANSFPPT (SEQ ID NO:17) |

That is, the above anti-FAP antibodies have the following general structure:
their HCDR1s are all SEQ ID NO: 9;
their HCDR2s are all SEQ ID NO: 10;
their HCDR3s are all DX₁SLTARPYYFYGFDV (SEQ ID NO: 18), where X₁ is E or A;
their LCDR1s are all SEQ ID NO: 12;
their LCDR2s are all SEQ ID NO: 13;
their LCDR3s are all QQANSFPX₂X₃ (SEQ ID NO: 19), where X₂ is P or L and X₃ is A or T.

### 1.4. Construction of CHO cell surface full-length antibody display library

1) Constructing a soluble antibody expression vector, comprising the following steps: Positive VH and LC fragments with unique sequences were digested with enzymes and purified. The VH fragments were inserted into a soluble heavy chain expression vector (IgG1), and the LC fragments were inserted into a soluble light chain expression vector. Colonies were sent for sequencing and DNA was extracted.
2) Expressing and purifying soluble antibodies, comprising the following steps: Expi293 cells were expanded by suspension culture. According to the light-heavy chain pairs determined above, 18 µg of light chain expression vector and 12 µg of heavy chain expression vector were mixed. The vector DNA and PEI were mixed in a ratio by weight of 1:2.5, and the mixture was used to transform Expi293 cells. On day 6, the supernatant of the culture was collected, and the antibodies were purified by the Protein-A method. SDS-PAGE denaturing gel electrophoresis analysis showed that the purity of the antibodies reached above 90%. The antibodies were stored at -80 °C.

### 1.5. ELISA binding assay

A FAP recombinant protein with a His tag was directly used for coating. After the antibodies were added, a secondary antibody (an HRP-conjugated anti-human IgG antibody) and the HRP substrate TMB were added to measure the binding activity of the antibodies to the antigen. The assay comprises the following steps: A 0.5 µg/mL solution of human FAP-His protein was used to coat a 96-well microplate at 100 µL/well, and the plate was incubated overnight at 4 °C. After a thorough wash, a blocking solution was added at 200 µL/well, and the plate was incubated at room temperature for 2 h. After a thorough wash, dilutions of test anti-FAP antibodies were added at 100 µL/well. The plate was incubated at room temperature for 1 h. After a thorough wash, a 1:20,000 dilution of an HRP-labeled goat anti-human IgG secondary antibody was added at 100 µL/well. The plate was incubated at room temperature for 1 h. After a thorough wash, TMB was added at 100 µL/well, and the plate was incubated in the dark for 15 min. 0.16 M sulfuric acid was added at 50 µL/well. The OD value at 450 nm was measured on a Thermo MultiSkanFc microplate reader, and the binding EC₅₀ values of the anti-FAP antibodies were calculated. The results are shown in Table 5.

**Table 5. The binding affinity EC₅₀ values of anti-FAP antibodies to the human FAP antigen**

| No. | EC₅₀ (mg/ mL) |
|---|---|
| Ab9 | 0.1014 |
| Ab10 | 0.0920 |
| Abl4 | 0.4168 |
| Abl5 | 0.3206 |

### 1.6. Surface plasmon resonance (SPR) binding assay

A CM5 sensor chip was used, and HBS-EP + buffer solution (10 mM HEPES, 150 mM NaCl, 3 mM EDTA, 0.05% surfactant P20) was used as the mobile phase. An anti-human IgG (Fc) antibody was made into a 30 µg/mL solution in a 10 mM sodium acetate buffer solution (pH 5.0) and immobilized by amino coupling. The test antibodies were diluted in HBS-EP + buffer solution and captured by the anti-human IgG (Fc) antibody on chip channels. h-FAP-His was dissolved in HBS-EP + buffer solution to prepare an analyte, and the analyte was serially diluted 2-fold. The diluted antibodies were passed through the experimental and reference channels at a flow rate of 30 µL/min, with 1 min of binding and 15 min of disassociation. 10 mM Glycine pH 1.5 was passed as a regeneration buffer solution for 30 s at a flow rate of 10 µL/min. Data were analyzed. The results in Table 6 show that the affinities of Ab9, Ab10, Ab14 and Ab15 for the antigen FAP are 3-6 times higher than that of the control anti-FAP antibody 28H1 (the sequences 219 and 233 of the patent US9266938B2), and the affinity of Ab 15 is the highest, with a K_{D} of 2.15 × 10⁻¹⁰. However, the binding affinities of the other anti-FAP antibodies obtained by screening at the same time in the present disclosure, such as Ab2, Ab4 and Ab5 (sequences not shown), for the antigen are lower than that of 28H1.

**Table 6. The SPR affinity Ka, Kd and K_{D} values of anti-FAP antibodies**

| No. | ka (1/Ms) | kd (1/s) | K_{D} (M) | Ratio of K_{D} of 28H1 to K_{D} of test antibody | Ratio of K_{d} of 28H1 to K_{d} of test antibody |
|---|---|---|---|---|---|
| 28H1 | 1.95E+05 | 2.55E-04 | 1.31E-09 | 1 | 1 |
| Ab2 | 1.62E+06 | 1.62E-02 | 9.98E-09 | 0.1 | 0.02 |
| Ab4 | 1.51E+06 | 6.17E-03 | 4.09E-09 | 0.3 | 0.04 |
| Ab5 | 1.08E+06 | 3.57E-03 | 3.32E-09 | 0.4 | 0.07 |
| Ab9 | 2.35E+05 | 7.19E-05 | 3.06E-10 | 4.3 | 3.54 |
| Ab10 | 2.97E+05 | 7.28E-05 | 2.45E-10 | 5.3 | 3.50 |
| Ab14 | 3.32E+05 | 1.21E-04 | 3.65E-10 | 3.6 | 2.10 |
| Ab15 | 4.88E+05 | 1.05E-04 | 2.15E-10 | 6.1 | 2.43 |

The heavy and light chain variable region sequences of the control antibody 28H1 are as follows:
> The VH of 28H1 
EVQLLESGGGLVQPGGSLRLSCAASGFTFSSHAMSWVRQAPGKGLEWVSAIWA
SGEQYYADSVKGRFTISRDNSKNTLYLQMNSLRAEDTAVYYCAKGWLGNFDY
WGQGTLVTVSS (SEQ ID NO: 20)
> The VL of 28H1

EIVLTQSPGTLSLSPGERATLSCRASQSVSRSYLAWYQQKPGQAPRLLIIGASTRA
TGIPDRFSGSGSGTDFTLTISRLEPEDFAVYYCQQGQVIPPTFGQGTKVEIK (SEQ
ID NO: 21)

### 1.7. FACS binding assay

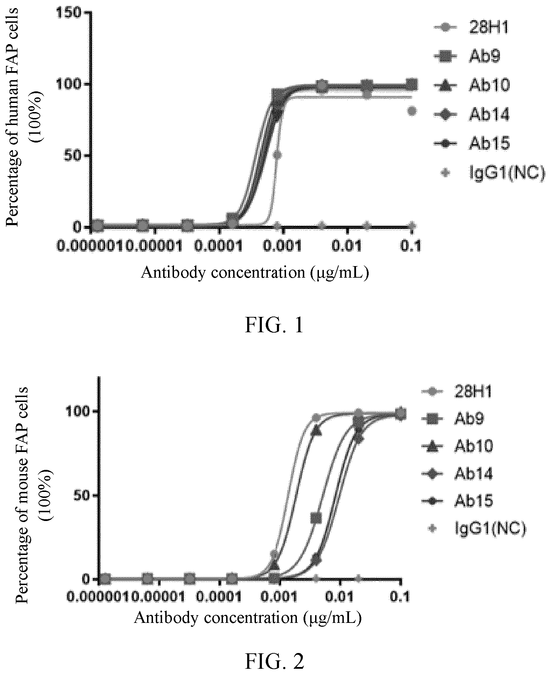

A FACS assay was used to measure the binding properties of anti-FAP antibodies on cells. The assay comprises the following steps: A CHO cell line overexpressing human FAP was constructed and plated (1E5/well). The test antibodies were added at 100 µL/well. The highest concentration was 100 nM. A 5-fold dilution was performed, yielding 8 concentrations in total. The plate was incubated at 4 °C for 1 h. Anti-hIgG Alexa Fluor-647 was added as a secondary antibody in the ratio of 1:500. The plate was incubated at 4 °C for 30 min, and a FACS assay was performed. Antibodies that bind to FAP can mark cells. The relation between the percentage of the marked cells that over-express human FAP among all cells and the antibody concentration is shown in FIG. 1. The binding EC₅₀ values of the antibodies to human FAP are shown in Table 7. The relation between the percentage of the marked cells that over-express mouse FAP among all cells and the antibody concentration is shown in FIG. 2.

**Table 7. The binding affinity EC₅₀ values of anti-FAP antibodies to the human FAP antigen**

| | |
|---|---|
| Antibody No. | EC₅₀ (µg/ mL) |
| 28H1 | 0.0007839 |
| Ab9 | 0.0003684 |
| Ab10 | 0.0004320 |
| Ab14 | 0.0004738 |
| Ab15 | 0.0004858 |
| Isotype (NC) | - |

### Example 2. Screening for and Preparation of Anti-CD40 Nanobodies (VHHs)

### 2.1. Sequences and preparation of immunizing and screening antigens

His-tagged human CD40 recombinant protein (h-CD40-His), C-terminally biotin-tagged human CD40 recombinant protein (h-CD40-biotin) and C-terminally His-tagged monkey CD40 recombinant protein (cyno-CD40-His) were selected. Their sequences and sources are shown in Table 8. The protein reagents can be used in the experiments of the following examples.

**Table 8. The amino acid sequences and sources of recombinant proteins**

| Name | Start and end of amino acid sequence | Acrobiosystems cat. No. |
|---|---|---|
| h-CD40-His | Glu21-Arg 193 | CD0-H5228 |
| h-CD40-biotin | | CD0-H82E8 |
| cyno-CD40-His | | CD0-C52H6 |

### 2.2. Alpaca immunization process and titer measurement

h-CD40-his was used to immunize an alpaca. Immunization was performed once every two weeks, and a total of four immunizations were performed. In the first immunization, 0.5 mg of antigen and 1 mL of complete Freund's adjuvant (CFA) were mixed and injected subcutaneously, and in the following three immunizations, 0.25 mg of antigen and 1 mL of incomplete Freund's adjuvant (IFA) were mixed and injected subcutaneously. Blank serum was collected before the immunizations. At one week after the third immunization and one week after the fourth immunization, 50 mL of peripheral blood was collected, and lymphocytes were separated. The serum titer was measured.

### 2.3. Titer measurement and phage library construction

After the four immunizations of the alpaca, the serum titer was measured. After the measurement showed that the titer was acceptable, PBMCs were separated, and total RNA was extracted. The purity was measured, and the RNA was reverse-transcribed into DNA. After two rounds of nested PCR, the purified vector and the VHH fragment of interest were digested with enzymes and ligated. Transfection was performed using electroporation, and clones were selected, yielding a phage library A and a phage library B.

### 2.4. Phage library affinity panning and ELISA identification

With the h-CD40 antigen as the target molecule, screening was performed by using the Gly-HCL acid elution method to elute specific phages. After two rounds of screening, 384 clones were randomly selected from the titer measurement plates of the first round and the second round and screened by phage ELISA for positive clones, and the optical density at 450 nm was measured. According to the sequencing results, the sequences were subjected to sequence alignment and evolutionary tree analysis, and 16 sequences were obtained by screening. Among them, the amino acid sequences of two better-functioning anti-CD40 antibodies are shown in Table 9 and Table 10.

**Table 9. The sequences of anti-CD40 single-domain antibodies**

| No. | Amino acid sequence |
|---|---|
| A12 | EVQVVESGGGLAQPGGSLRLSCAASGFTFSNYGMKWVRQAPGKGPEWVS TILSQGGSTYYADSVKGRFTISRDNAKNTLYLQMNSLKPEDTALYYCLRVD WSDYSPRGQGTQVTVSA (SEQ ID NO:22) |
| A297 | QVQLVESGGGMVEPGGSLRLSCAASGFTFSRYGMKWVRQAPGKGPEWVST INSHGDTTYYADSVKGRFTISRDNAQNTVYLQMNSLKPEDTALYYCLRIDYS DYSSRGQGTQVTVSS (SEQ ID NO:23) |

| | |
|---|---|
| Note: The CDRs defined by the Kabat numbering scheme are underlined. | |

**Table 10. The CDRs of anti-CD40 antibodies**

| No. | CDR sequences | |
|---|---|---|
| Ab12 | CDR1 | NYGMK (SEQ ID NO:24) |
| | CDR2 | TILSQGGSTYYADSVKG (SEQ ID NO:25) |
| | CDR3 | VDWSDYSP (SEQ ID NO:26) |
| Ab297 | CDR1 | RYGMK (SEQ ID NO:27) |
| | CDR2 | TINSHGDTTYYADSVKG (SEQ ID NO:28) |
| | CDR3 | IDYSDYSS (SEQ ID NO:29) |

### 2.5. Expression and purification of anti-CD40 single-domain antibody-Fc fusion proteins

The 2 VHHs above were separately ligated to human IgG1-Fc comprising the mutation N297A. The sequences of the resulting VHH-Fc fusion proteins are shown in Table 11. Human IgG1-Fc is underlined, and the mutation N297A is in bold type.

9E5-SELFNS was used as a positive control CD40 agonist (see the sequences 58 and 59 of WO2020108611A1).

**Table 11. The sequences of the fusion proteins obtained from the anti-CD40 single-domain antibodies and human IgG1-Fc**

| No. | Amino acid sequence |
|---|---|
| A12-Fc | EVQVVESGGGLAQPGGSLRLSCAASGFTFSNYGMKWVRQAPGKGPEW VSTILSQGGSTYYADSVKGRFTISRDNAKNTLYLQMNSLKPEDTALYYCL RVDWSDYSPRGQGTQVTVSAEPKSADKTHTCPPCPAPELLGGPSVFLFPP KPKDTLMISRTPEVTCVVVDVSHEDPEVKFNWYVDGVEVHNAKTKPRE EQYASTYRVVSVLTVLHQDWLNGKEYKCKVSNKALPAPIEKTISKAKGQ PREPQVYTLPPSREEMTKNQVSLTCLVKGFYPSDIAVEWESNGQPENNYK TTPPVLDSDGSFFLYSKLTVDKSRWQQGNVFSCSVMHEALHNHYTQKSL SLSPGK(SEQ ID NO: 30) |
| A297-Fc | QVQLVESGGGMVEPGGSLRLSCAASGFTFSRYGMKWVRQAPGKGPEW VSTINSHGDTTYYADSVKGRFTISRDNAQNTVYLQMNSLKPEDTALYYC LRIDYSDYSSRGQGTQVTVSSEPKSADKTHTCPPCPAPELLGGPSVFLFPP KPKDTLMISRTPEVTCVVVDVSHEDPEVKFNWYVDGVEVHNAKTKPRE EQYASTYRVVSVLTVLHQDWLNGKEYKCKVSNKALPAPIEKTISKAKGQ PREPQVYTLPPSREEMTKNQVSLTCLVKGFYPSDIAVEWESNGQPENNYK TTPPVLDSDGSFFLYSKLTVDKSRWQQGNVFSCSVMHEALHNHYTQKSL SLSPGK(SEQ ID NO: 31) |

Plasmids were constructed and used to transiently transfect cells, and antibodies were expressed and purified. Analysis showed that the antibodies of interest were obtained.

### 2.6. FACS binding assay

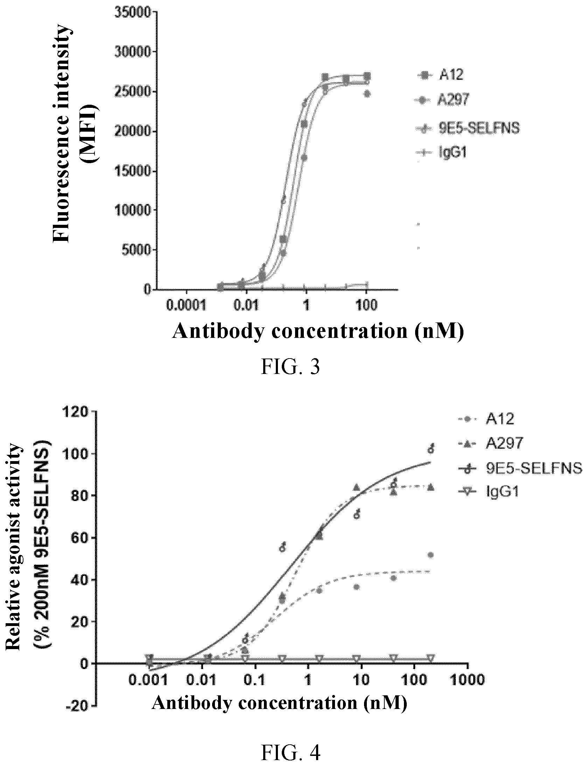

A FACS assay was used to measure the binding properties of anti-CD40 antibodies on cells. The assay comprises the following steps: Raji single cells were obtained and plated. The test antibodies were added at 100 µL/well. The highest concentration point was 100 nM. A 5-fold dilution was performed, yielding 8 concentration points in total. The plate was incubated at 4 °C for 1 h. Anti-hIgG Alexa Fluor-647 was added as a secondary antibody in the ratio of 1:500. The plate was incubated at 4 °C for 30 min. A FACS assay was performed. A CD40 agonist was used as a positive control. The EC₅₀ values of the antibodies are shown in FIG. 3 and Table 12.

**Table 12. The binding affinity EC₅₀ values of anti-CD40 antibodies to the human CD40 antigen**

| No. | EC₅₀(nM) |
|---|---|
| A12 | 0.3657 |
| A297 | 0.5293 |
| 9E5-SELFNS | 0.1961 |

For a CD40 agonist antibody used in a FAP/CD40 bispecific antibody, its affinity EC₅₀ for CD40 should not be too high; otherwise, the bispecific antibody will bind preferentially to CD40. It is more desirable for the bispecific antibody to bind preferentially to FAP, thereby playing a role. Therefore, A12 and A297 have advantages that are more in line with the above characteristics. This is also reflected in Table 13.

### 2.7. Measurement of activity of anti-CD40 nanobodies

The activation of CD40 reporter gene cells by the anti-CD40 antibodies was measured, and the agonist activity of the CD40 antibodies was assessed according to EC₅₀. The measurement comprises the following steps: HEK-Blue^{™}CD40L cells (purchased from Invivogen Cat#hkb-cd40, having been stably transfected with the human CD40 gene and the NF-xB-mediated SEAP genome; the level of activation of the CD40 signaling pathway can be characterized by measuring the amount of SEAP secreted in the supernatant using the SEAP substrate QUANTI-Blue) were prepared. The culture medium was DMEM containing 10% FBS, 100 µg/mL Normocin, 100 µg/mL Zeocin and 30 pg/mL Blasticidin. The cells were plated onto a 96-well plate at 5E4/well. The culture medium was DMEM containing 10% FBS and 100 µg/mL Normocin. The cells were cultured overnight. After cell adhesion, the serially diluted test antibodies were added at 100 µL/well, and the cells were cultured overnight at 37 °C. The cells were centrifuged. The cell supernatant was transferred to a new 96-well plate, and 180 µL of QUANTI-Blue substrate solution was added. The plate was incubated in the dark for 15 min. The absorbance at 620 nm was measured on an Envision microplate reader, and the EC₅₀ values were calculated. A CD40 agonist (9E5-SELFNS) was used as a positive control. The EC₅₀ for the relation between the relative agonist activity percentage (compared to 200 nM 9E5-SELFNS) and the antibody concentration is shown in FIG. 4 and Table 13.

**Table 13. The agonist activity EC₅₀ values of anti-CD40 antibodies and their activating activity percentages (%) relative to the positive control**

| No. | EC₅₀ (nM) | Maximum response (percentage relative to 200 nM 9E5-SELFNS) |
|---|---|---|
| A12 | 0.231 | 51.76 |
| A297 | 0.556 | 84.15 |
| 9E5-SELFNS | 0.466 | 102.55 |
| IgG1 | No activation | No activation |

### 2.8. Humanization of anti-CD40 nanobodies

According to the above results, A12 and A297 were humanized. On the basis of the typical VHH structures of the obtained nanobodies A12 and A297, the VHH variable region sequences were compared with an antibody germline database to obtain highly homologous human germline templates. The preferred human germline templates for the antibodies A12 and A297 in the present disclosure are both IGHV3-48*03. The CDRs were then grafted into human FRs, and the key amino acids that affect the structures and functions of the antibodies were back-mutated to restore binding and activity. The humanized sequences are shown in Table 14.

**Table 14. The sequences of anti-CD40 humanized antibodies**

| Name | | Amino acid sequence |
|---|---|---|
| A12 | V1 | EVQLVESGGGLVQPGGSLRLSCAASGFTFSNYGMKWVRQAPGKGLEW VSTILSQGGSTYYADSVKGRFTISRDNAKNSLYLQMNSLRAEDTALYYC ARVDWSDYSPRGQGTMVTVSS(SEQ ID NO: 32) |
| | V2 | EVQLVESGGGLVQPGGSLRLSCAASGFTFSNYGMKWVRQAPGKGLEW VSTILSQGGSTYYADSVKGRFTISRDNAKNSLYLQMNSLRAEDTALYYC LRVDWSDYSPRGQGTQVTVSS(SEQ ID NO: 33) |
| | V3: | EVQVVESGGGLVQPGGSLRLSCAASGFTFSNYGMKWVRQAPGKGLE WVSTILSQGGSTYYADSVKGRFTISRDNSKNTLYLQMNSLRAEDTAVY YCLRVDWSDYSPRGQGTQVTVSS(SEQ ID NO: 34) |
| A297 | V1 | QVQLVESGGGVVQPGGSLRLSCAASGFTFSRYGMKWVRQAPGKGLE WVSTINSHGDTTYYADSVKGRFTISRDNAKNSLYLQMNSLRAEDTALY YCARIDYSDYSSRGQGTMVTVSS(SEQ ID NO: 35) |
| | V2 | QVQLVESGGGVVQPGGSLRLSCAASGFTFSRYGMKWVRQAPGKGLE |
| | | WVSTINSHGDTTYYADSVKGRFTISRDNAKNSLYLQMNSLRAEDTALY YCLRIDYSDYSSRGQGTMVTVSS(SEQ ID NO: 36) |
| | V3 | EVQLVESGGGLVQPGGSLRLSCAASGFTFSRYGMKWVRQAPGKGLEW VSTINSHGDTTYYADSVKGRFTISRDNAKNSLYLQMNSLRAEDTAVYY CLRIDYSDYSSRGQGTLVTVSS(SEQ ID NO: 37) |
| | V4 | EVQLVESGGGLVQPGGSLRLSCAASGFTFSRYGMKWVRQAPGKGLEW VSTINSHGDSTYYADSVKGRFTISRDNSKNTLYLQMNSLRAEDTAVYYC LKIDYSDYSSRGQGTLVTVSS(SEQ ID NO: 38) |

That is, the anti-CD40 nanobodies of the present disclosure have the following general structure:
their CDR1s are X₄YGMK (SEQ ID NO: 39), where X₄ is N or R;
their CDR2s are TIX₅SX₆GX₇X₈TYYADSVKG (SEQ ID NO: 40), where X₅ is N or L; X₆ is Q or H; X₇ is G or D; X₈ is S or T;
their CDR3s are X₉D XᵢₒSDYSX₁₁ (SEQ ID NO: 41), where X₉ is V or I; X₁₀ is W or Y; X₁₁ is P or S.

The above 7 VHHs were separately ligated to human IgG1-Fc comprising the mutations S267E/L328F (numbered according to EU). The sequences of the resulting VHH-Fc fusion proteins are shown in Table 15. Human IgG1-Fc is underlined, and the mutations S267E/L328F are in bold type.

**Table 15. The sequences of the fusion proteins obtained from the humanized anti-CD40 single-domain antibodies and human IgG1-Fc (S267E/L328F)**

| No. | Amino acid sequence |
|---|---|
| A12V1-Fc | EVQLVESGGGLVQPGGSLRLSCAASGFTFSNYGMKWVRQAPGKGLE WVSTILSQGGSTYYADSVKGRFTISRDNAKNSLYLQMNSLRAEDTALY YCARVDWSDYSPRGQGTMVTVSSEPKSADKTHTCPPCPAPELLGGPS VFLFPPKPKDTLMISRTPEVTCVVVDVEHEDPEVKFNWYVDGVEVHN AKTKPREEQYNSTYRVVSVLTVLHQDWLNGKEYKCKVSSKAFPAPIE KTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMH EALHNHYTQKSLSLSPGK(SEQ ID NO: 57) |
| A12V2-Fc | EVQLVESGGGLVQPGGSLRLSCAASGFTFSNYGMKWVRQAPGKGLE WVSTILSQGGSTYYADSVKGRFTISRDNAKNSLYLQMNSLRAEDTALY YCLRVDWSDYSPRGQGTQVTVSSEPKSADKTHTCPPCPAPELLGGPSV FLFPPKPKDTLMISRTPEVTCVVVDVEHEDPEVKFNWYVDGVEVHNA KTKPREEQYNSTYRVVSVLTVLHQDWLNGKEYKCKVSSKAFPAPIEK TISKAKGQPREPQVYTLPPSRDELTKNQVSLTCLVKGFYPSDIAVEWES NGQPENNYKTTPPVLDSDGSFFLYSKLTVDKSRWQQGNVFSCSVMHE ALHNHYTQKSLSLSPGK(SEQ ID NO: 58) |
| A12V3-Fc | EVQVVESGGGLVQPGGSLRLSCAASGFTFSNYGMKWVRQAPGKGLE WVSTILSQGGSTYYADSVKGRFTISRDNSKNTLYLQMNSLRAEDTAVY YCLRVDWSDYSPRGQGTQVTVSSEPKSADKTHTCPPCPAPELLGGPSV FLFPPKPKDTLMISRTPEVTCVVVDVEHEDPEVKFNWYVDGVEVHNA KTKPREEQYNSTYRVVSVLTVLHQDWLNGKEYKCKVSSKAFPAPIEK |
| | TISKAKGQPREPQVYTLPPSRDELTKNQVSLTCLVKGFYPSDIAVEWES NGQPENNYKTTPPVLDSDGSFFLYSKLTVDKSRWQQGNVFSCSVMHE ALHNHYTQKSLSLSPGK(SEQ ID NO: 59) |
| A297V1-Fc | QVQLVESGGGVVQPGGSLRLSCAASGFTFSRYGMKWVRQAPGKGLE WVSTINSHGDTTYYADSVKGRFTISRDNAKNSLYLQMNSLRAEDTAL YYCARIDYSDYSSRGQGTMVTVSSEPKSADKTHTCPPCPAPELLGGPS VFLFPPKPKDTLMISRTPEVTCVVVDVEHEDPEVKFNWYVDGVEVHN AKTKPREEQYNSTYRVVSVLTVLHQDWLNGKEYKCKVSSKAFPAPIE KTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMH EALHNHYTQKSLSLSPGK(SEQ ID NO: 60) |
| A297V2-Fc | QVQLVESGGGVVQPGGSLRLSCAASGFTFSRYGMKWVRQAPGKGLE WVSTINSHGDTTYYADSVKGRFTISRDNAKNSLYLQMNSLRAEDTAL YYCLRIDYSDYSSRGQGTMVTVSSEPKSADKTHTCPPCPAPELLGGPS VFLFPPKPKDTLMISRTPEVTCVVVDVEHEDPEVKFNWYVDGVEVHN AKTKPREEQYNSTYRVVSVLTVLHQDWLNGKEYKCKVSSKAFPAPIE KTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMH EALHNHYTQKSLSLSPGK(SEQ ID NO: 61) |
| A297V3-Fc | EVQLVESGGGLVQPGGSLRLSCAASGFTFSRYGMKWVRQAPGKGLE WVSTINSHGDTTYYADSVKGRFTISRDNAKNSLYLQMNSLRAEDTAV YYCLRIDYSDYSSRGQGTLVTVSSEPKSADKTHTCPPCPAPELLGGPSV FLFPPKPKDTLMISRTPEVTCVVVDVEHEDPEVKFNWYVDGVEVHNA KTKPREEQYNSTYRVVSVLTVLHQDWLNGKEYKCKVSSKAFPAPIEK TISKAKGQPREPQVYTLPPSRDELTKNQVSLTCLVKGFYPSDIAVEWES NGQPENNYKTTPPVLDSDGSFFLYSKLTVDKSRWQQGNVFSCSVMHE ALHNHYTQKSLSLSPGK(SEQ ID NO: 62) |
| A297V4-Fc | EVQLVESGGGLVQPGGSLRLSCAASGFTFSRYGMKWVRQAPGKGLE WVSTINSHGDSTYYADSVKGRFTISRDNSKNTLYLQMNSLRAEDTAVY YCLKIDYSDYSSRGQGTLVTVSSEPKSADKTHTCPPCPAPELLGGPSVF LFPPKPKDTLMISRTPEVTCVVVDVEHEDPEVKFNWYVDGVEVHNAK TKPREEQYNSTYRVVSVLTVLHQDWLNGKEYKCKVSSKAFPAPIEKTI SKAKGQPREPQVYTLPPSRDELTKNQVSLTCLVKGFYPSDIAVEWESN GQPENNYKTTPPVLDSDGSFFLYSKLTVDKSRWQQGNVFSCSVMHEA LHNHYTQKSLSLSPGK(SEQ ID NO: 63) |

The above 7 VHH-Fc were used to transiently transfect cells, and antibodies were expressed and purified. Analysis showed that the antibody proteins of interest were obtained.

### 2.9. Affinities of humanized CD40 antibodies for cell strain highly expressing CD40

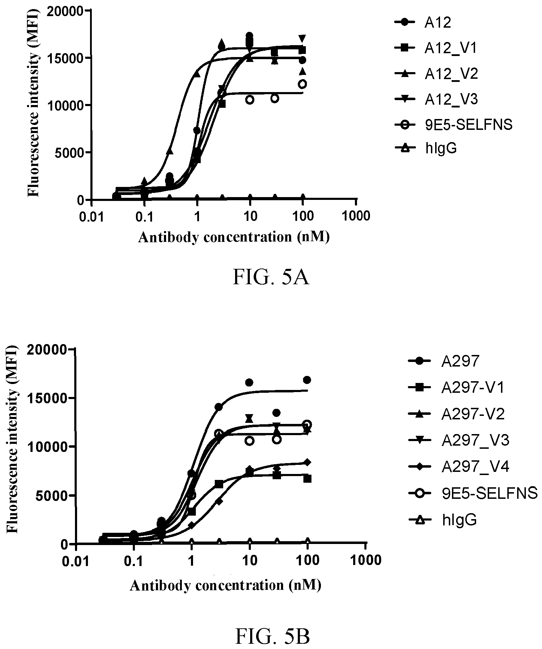

The ability of the humanized antibodies to bind to cells highly expressing the human antigen protein CD40 was measured by FACS. HEK293 cells were transiently transfected with CD40 plasmids (CD40 cDNA ORF Clone, Human, C-DYKDDDDK (Flag^{®}) Tag; Sino Biological; HG10774-CF), yielding HEK293 cells highly expressing CD40. The resulting cells were resuspended in the flow cytometry staining buffer (PBS + 2% FBS), and the test antibodies that had been serially diluted to different concentrations were added. After 1 h of incubation on ice, the cells were washed with PBS and centrifuged at 400 g for 5 min. A goat anti-human Fc antibody labeled with the fluorescent group Alexa Fluor 647 was added as a secondary antibody, and the cells were stained in an ice bath for 1 h. After two washes with PBS, the fluorescent signals on the cell surface were detected by FACS. The results are shown in FIGs. 5A and 5B, and the EC₅₀ values are shown in Table 16. The humanized antibodies of the present disclosure all have relatively high affinities for the cell strain highly expressing CD40.

**Table 16. The affinity EC₅₀ values of the humanized anti-CD40 antibodies for the cell strain highly expressing human CD40**

| Antibody | EC₅₀ (nM) | Emax MFI |
|---|---|---|
| A12 | 1.09 | 17256 |
| A12_V1 | 2.13 | 16608 |
| A12_V2 | 0.42 | 16609 |
| A12_V3 | 1.74 | 16909 |
| A297 | 1.12 | 16728 |
| A297_V1 | 1.11 | 7300 |
| A297_V2 | 1.07 | 12764 |
| A297_V3 | 1.25 | 12827 |
| A297_V4 | 2.70 | 8255 |
| 9E5-SELFNS | 1.12 | 12120 |

### 2.10. Activation of HEK-Blue^{™} CD40L cells by humanized CD40 antibodies

The *in-vitro* agonist activity of the CD40 antibodies was assessed by measuring the activation of HEK-Blue^{™}CD40L cells by the CD40 antibodies when crosslinked by FcyRIIb. The Fc of the humanized CD40 antibodies comprise the mutations S267E/L328F. The mutations enhance the affinity of IgG1 Fc for FcyRIIb, thereby enhancing the FcyRIIb's crosslinking of the antibodies. The FcyRIIb-mediated activation of HEK-Blue^{™} CD40L cells by the CD40 antibodies simulates the agonist activity of the CD40 antibodies when fully crosslinked. HEK293 cells were transiently transfected with FcyRIIb plasmids (CD32B/Fcgr2b cDNA ORF Clone, Human, N-His tag; Sino Biological; HG10259-NH), yielding HEK293 cells highly expressing FcyRIIb. HEK-Blue^{™}CD40L cells were plated onto a 96-well cell culture plate at 5E4/well (the culture medium was DMEM, 10% FBS, 100 µg/mL Normocin), and the HEK293 cells expressing FcyRIIb were added at 5E4/well. The serially diluted test antibodies were added at 100 µL/well, and the plate was incubated overnight at 37 °C. The cells were centrifuged. 20 µL of the cell supernatant was transferred to a new 96-well plate, and 180 µL of QUANTI-Blue substrate solution was added. The plate was incubated in the dark for 30 min. The absorbance at 620 nm was measured on an Envision microplate reader, and the EC₅₀ values and the Emax values (relative to the fluorescence intensity of the antibody-free group) were calculated. The EC₅₀ values were used to assess the *in-vitro* cell agonist activity of the CD40 antibodies.

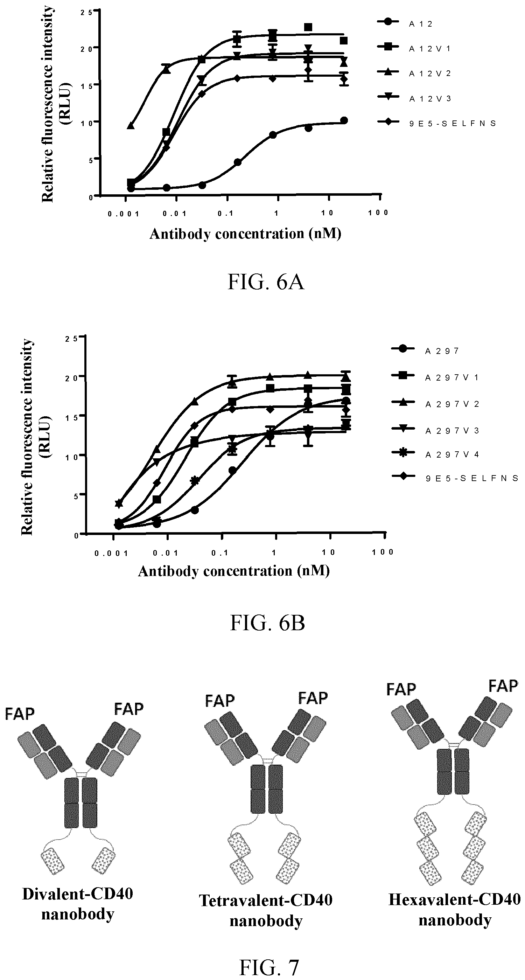

The FcyRIIb-mediated activation of HEK-Blue^{™}CD40L cells by the CD40 antibodies is shown in FIGs. 6A and 6B. The EC₅₀ values and the Emax values (relative fluorescence intensity) are shown in Table 17.

The results show that where the humanized CD40 antibodies were crosslinked by FcyRIIb, the humanized CD40 antibodies all have relatively strong agonist activity in the reporter gene cell system described above.

**Table 17. The activation of HEK-Blue^{™}CD40L cells by the humanized anti-CD40 antibodies**

| Antibody | FcyRIIb-mediated activation of HEK-Blue^{™}CD40L cells | |
|---|---|---|
| | EC₅₀ (nM) | Emax (relative fluorescence intensity) |
| A12 | 0.231 | 9.68 |
| A12_V1 | 0.009 | 21.61 |
| A12_V2 | 0.002 | 18.57 |
| A12_V3 | 0.011 | 19.08 |
| A297 | 0.243 | 17.34 |
| A297-V1 | 0.021 | 18.37 |
| A297-V2 | 0.005 | 19.99 |
| A297_V3 | 0.006 | 12.84 |
| A297_V4 | 0.038 | 13.31 |
| 9E5-SELFNS | 0.009 | 16.05 |

### Example 3. Design and Preparation of Anti-FAP/CD40 Bispecific Antibodies

### 3.1. Design, expression and purification of anti-FAP/CD40 bispecific antibodies

According to the humanized anti-CD40 antibody screening results, A12V2 and A297V3 were selected. According to the anti-FAP antibody screening results, Ab10 was selected. The selected antibodies were used to construct anti-FAP/CD40 bispecific antibodies. Ab10 was used as the IgG framework for the bispecific antibodies, and each of the C-termini of the two heavy chains of Ab10 was connected with 1 anti-CD40 nanobody, or 2 or 3 anti-CD40 nanobodies in tandem. The linkers used between the CD40 nanobodies and the linkers used for linking to the C-termini of the heavy chains of Ab10 were "GGGGSGGGGS". Each of the bispecific antibodies contains divalent, tetravalent and hexavalent CD40 nanobodies, as shown in FIG. 7. In the names of the antibodies, for example, Ab10-A12V2-2, Ab10 represents that the anti-FAP antibody was used, A12V2 represents that the humanized anti-CD40 antibody was used, and the "2" at the end represents that CD40 was divalent. The other antibodies are all named in this fashion. Transient transfection and antibody expression and purification were performed using the methods in section 2.5 of Example 2. Analysis showed that the bispecific antibody molecules of interest were obtained. The amino acid sequences of the bispecific antibody molecules are as follows:
> The heavy chain of Ab 10-A12V2-2 
QVQLQQSGVEVKKPGASVTVSCRASGYSFADHFIHWVRQAPGQGFQWMGWIN
PNRGVTHHAQDFQGRVAMTRDMSTDTVYMELTSLRSDDTAVYYCARDASLTA
RPYYFYGFDVWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGKGGGGSGGGGSEVQLVESGGGLVQPGG
SLRLSCAASGFTFSNYGMKWVRQAPGKGLEWVSTILSQGGSTYYADSVKGRFT
ISRDNAKNSLYLQMNSLRAEDTALYYCLRVDWSDYSPRGQGTQVTVSS (SEQ
ID NO: 42)
> The light chain of Ab 10-A12V2-2 
DIQMTQSPSSVSASVGDRVTITCRASQGISSWLAWYQQKPGKAPKLLIYAASSLQ
SGVPSRFSGSGSGTDFTLTISSLQPEDFATYYCQQANSFPPAFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC (SEQ
ID NO: 43)
> The heavy chain of Ab 10-A12V2-4 
QVQLQQSGVEVKKPGASVTVSCRASGYSFADHFIHWVRQAPGQGFQWMGWIN
PNRGVTHHAQDFQGRVAMTRDMSTDTVYMELTSLRSDDTAVYYCARDASLTA
RPYYFYGFDVWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGKGGGGSGGGGSEVQLVESGGGLVQPGG
SLRLSCAASGFTFSNYGMKWVRQAPGKGLEWVSTILSQGGSTYYADSVKGRFT
ISRDNAKNSLYLQMNSLRAEDTALYYCLRVDWSDYSPRGQGTQVTVSSGGGGS
GGGGSEVQLVESGGGLVQPGGSLRLSCAASGFTFSNYGMKWVRQAPGKGLEW
VSTILSQGGSTYYADSVKGRFTISRDNAKNSLYLQMNSLRAEDTALYYCLRVDW
SDYSPRGQGTQVTVSS (SEQ ID NO: 44)
> The heavy chain of Ab 10-A12V2-6 
QVQLQQSGVEVKKPGASVTVSCRASGYSFADHFIHWVRQAPGQGFQWMGWIN
PNRGVTHHAQDFQGRVAMTRDMSTDTVYMELTSLRSDDTAVYYCARDASLTA
RPYYFYGFDVWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
 
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGKGGGGSGGGGSEVQLVESGGGLVQPGG
SLRLSCAASGFTFSNYGMKWVRQAPGKGLEWVSTILSQGGSTYYADSVKGRFT
ISRDNAKNSLYLQMNSLRAEDTALYYCLRVDWSDYSPRGQGTQVTVSSGGGGS
GGGGSEVQLVESGGGLVQPGGSLRLSCAASGFTFSNYGMKWVRQAPGKGLEW
VSTILSQGGSTYYADSVKGRFTISRDNAKNSLYLQMNSLRAEDTALYYCLRVDW
SDYSPRGQGTQVTVSSGGGGSGGGGSEVQLVESGGGLVQPGGSLRLSCAASGFT
FSNYGMKWVRQAPGKGLEWVSTILSQGGSTYYADSVKGRFTISRDNAKNSLYL
QMNSLRAEDTALYYCLRVDWSDYSPRGQGTQVTVSS (SEQ ID NO: 45)
> The heavy chain of Ab 10-A297V3-2 
QVQLQQSGVEVKKPGASVTVSCRASGYSFADHFIHWVRQAPGQGFQWMGWIN
PNRGVTHHAQDFQGRVAMTRDMSTDTVYMELTSLRSDDTAVYYCARDASLTA
RPYYFYGFDVWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGKGGGGSGGGGSEVQLVESGGGLVQPGG
SLRLSCAASGFTFSRYGMKWVRQAPGKGLEWVSTINSHGDTTYYADSVKGRFT
ISRDNAKNSLYLQMNSLRAEDTAVYYCLRIDYSDYSSRGQGTLVTVSS (SEQ ID
NO: 46)
> The heavy chain of Ab 10-A297V3-4 
QVQLQQSGVEVKKPGASVTVSCRASGYSFADHFIHWVRQAPGQGFQWMGWIN
PNRGVTHHAQDFQGRVAMTRDMSTDTVYMELTSLRSDDTAVYYCARDASLTA
RPYYFYGFDVWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGKGGGGSGGGGSEVQLVESGGGLVQPGG
SLRLSCAASGFTFSRYGMKWVRQAPGKGLEWVSTINSHGDTTYYADSVKGRFT
ISRDNAKNSLYLQMNSLRAEDTAVYYCLRIDYSDYSSRGQGTLVTVSSGGGGSG
GGGSEVQLVESGGGLVQPGGSLRLSCAASGFTFSRYGMKWVRQAPGKGLEWV
STINSHGDTTYYADSVKGRFTISRDNAKNSLYLQMNSLRAEDTAVYYCLRIDYS
DYSSRGQGTLVTVSS (SEQ ID NO: 47)
> The heavy chain of Ab 10-A297V3-6

QVQLQQSGVEVKKPGASVTVSCRASGYSFADHFIHWVRQAPGQGFQWMGWIN
PNRGVTHHAQDFQGRVAMTRDMSTDTVYMELTSLRSDDTAVYYCARDASLTA

RPYYFYGFDVWGQGTLVTVSSASTKGPSVFPLAPSSKSTSGGTAALGCLVKDYF
PEPVTVSWNSGALTSGVHTFPAVLQSSGLYSLSSVVTVPSSSLGTQTYICNVNHK
PSNTKVDKKVEPKSCDKTHTCPPCPAPEAAGGPSVFLFPPKPKDTLMISRTPEVT
CVVVDVSHEDPEVKFNWYVDGVEVHNAKTKPREEQYNSTYRVVSVLTVLHQD
WLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQG
NVFSCSVMHEALHNHYTQKSLSLSPGKGGGGSGGGGSEVQLVESGGGLVQPGG
SLRLSCAASGFTFSRYGMKWVRQAPGKGLEWVSTINSHGDTTYYADSVKGRFT
ISRDNAKNSLYLQMNSLRAEDTAVYYCLRIDYSDYSSRGQGTLVTVSSGGGGSG
GGGSEVQLVESGGGLVQPGGSLRLSCAASGFTFSRYGMKWVRQAPGKGLEWV
STINSHGDTTYYADSVKGRFTISRDNAKNSLYLQMNSLRAEDTAVYYCLRIDYS
DYSSRGQGTLVTVSSGGGGSGGGGSEVQLVESGGGLVQPGGSLRLSCAASGFTF
SRYGMKWVRQAPGKGLEWVSTINSHGDTTYYADSVKGRFTISRDNAKNSLYLQ
MNSLRAEDTAVYYCLRIDYSDYSSRGQGTLVTVSS (SEQ ID NO: 48)

The light chains of Ab10-A12V2-4, Ab10-A12V2-6, Ab10-A297V3-2, Ab10-A297V3-4 and Ab10-A297V3-6 are all SEQ ID NO: 43.

Note: The CH1 and Fc regions in the heavy chains are underlined; the CL regions in the light chains are underlined; the linkers are in italic type; LALA (the mutations L234A and L235A) is in bold type.

### 3.2. Measurement of antigen-binding affinities of anti-FAP/CD40 bispecific antibodies

The affinities of the anti-FAP/CD40 bispecific antibodies for their antigens, the human FAP protein (Aero, FAP-H5244) and the human CD40 protein (Aero, CD0-H5228), were measured by SPR (GE Healthcare; Biacore 8K). A CM5 sensor chip was used, and HBS-EP + buffer solution (10 mM HEPES, 150 mM NaCl, 3 mM EDTA, 0.05% surfactant P20) was used as the mobile phase. An anti-human IgG (Fc) antibody was immobilized by amino coupling. The test antibodies were separately diluted in HBS-EP + buffer solution as ligands and captured by the anti-human IgG (Fc) antibody on chip channels. Antigenic proteins of different species were used as analytes. The results are shown in Tables 18 and 19. The results show that the prepared bispecific antibodies can normally bind to FAP, and because their affinities for CD40 are lower, they bind preferentially to FAP, thereby playing a role.

**Table 18. The affinities of anti-FAP/CD40 bispecific antibodies for human FAP**

| Antibody | Ka (1/Ms) | Kd (1/s) | KD (M) |
|---|---|---|---|
| Ab10 | 2.17E+05 | 7.39E-05 | 3.40E-10 |
| Ab10-A12V2-4 | 1.75E+05 | 5.14E-05 | 2.94E-10 |
| Ab10-A297V3-2 | 2.03E+05 | 8.22E-05 | 4.05E-10 |
| Ab10-A297V3-4 | 1.84E+05 | 6.96E-05 | 3.78E-10 |

**Table 19. The affinities of anti-FAP/CD40 bispecific antibodies for human CD40**

| Antibody | Ka (1/Ms) | Kd (1/s) | KD (M) |
|---|---|---|---|
| 9E5-SELFNS | 6.04E+06 | 6.70E-03 | 1.11E-09 |
| Ab10-A12V2-4 | 6.90E+04 | 2.00E-03 | 2.90E-08 |
| Ab10-A297V3-2 | 2.09E+05 | 2.94E-02 | 1.41E-07 |
| Ab10-A297V3-4 | 5.24E+05 | 1.37E-01 | 2.62E-07 |

### 3.3. Measurement of affinities of anti-FAP/CD40 bispecific antibodies for cell strains highly expressing FAP

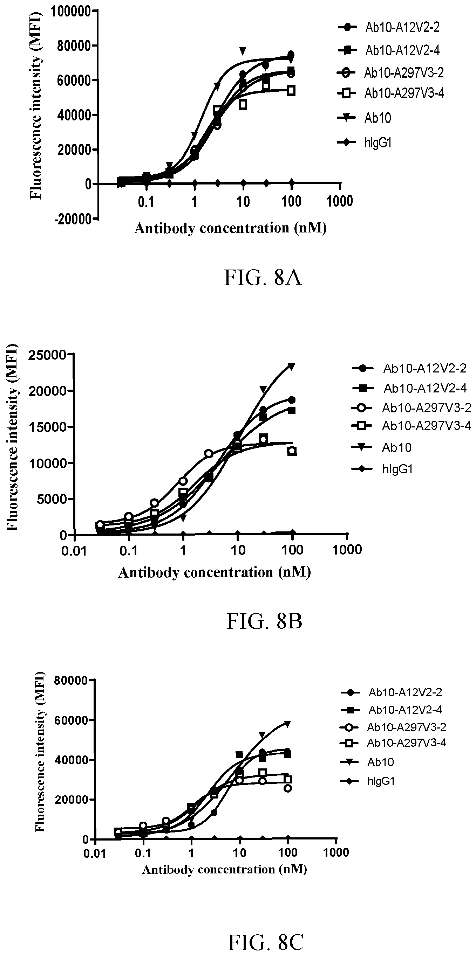

In this experiment, the binding of the anti-FAP/CD40 bispecific antibodies to stably transfected strains of CHOK1 cells that highly express human FAP, cynomolgus monkey FAP and mouse FAP was measured by FACS. The bispecific antibodies were shown to bind to the human, mouse and cynomolgus monkey cell surface FAP antigens, and their binding abilities are similar to those of the mAbs. See FIGs. 8A-8C. The EC₅₀ values are shown in Table 20.

**Table 20. The EC₅₀ values for the binding of the anti-FAP/CD40 bispecific antibodies to the human and mouse cell surface FAP antigens**

| Antibody | Human FAP | | Mouse FAP | | Cynomolgus monkey FAP | |
|---|---|---|---|---|---|---|
| | EC₅₀ (nM) | Emax (MFI) | EC₅₀ (nM) | Emax (MFI) | EC₅₀ (nM) | Emax (MFI) |
| Ab10-A12V2-2 | 2.72 | 74010 | 4.32 | 19541 | 5.65 | 44985 |
| Ab10-A12V2-4 | 2.57 | 64905 | 4.43 | 18967 | 2.26 | 43150 |
| Ab10-A297V3-2 | 2.51 | 65204 | 0.79 | 12633 | 1.09 | 28178 |
| Ab10-A297V3-4 | 1.57 | 53835 | 1.65 | 12747 | 1.35 | 32480 |
| Ab10 | 1.41 | 71968 | 10.41 | 26381 | 7.34 | 64618 |

### 3.4. Measurement of affinities of anti-FAP/CD40 bispecific antibodies for cell strains highly expressing CD40

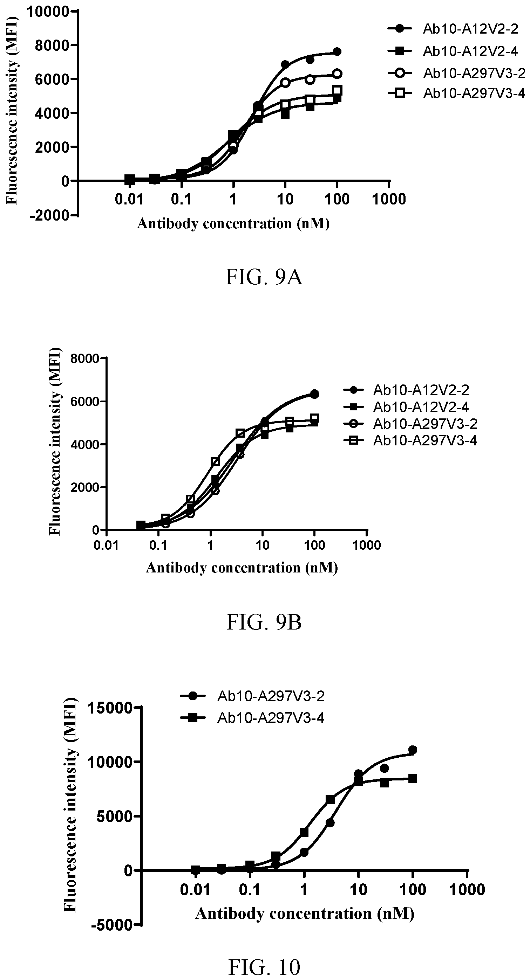

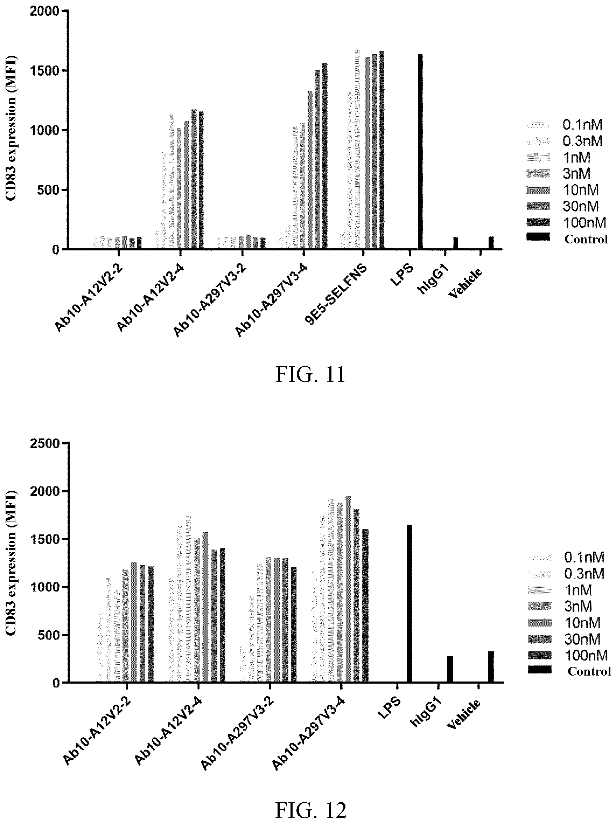

The binding of the bispecific antibodies to cell surface CD40 was measured by FACS, including HEK-BlueTMCD40L cells highly expressing human CD40 (Invivogen, Cat#hkb-cd40), and HEK293 cells that were transiently transfected with cynomolgus monkey CD40 plasmids (Sino Biological, cat# CG90970-UT) and thus made to highly express cynomolgus monkey CD40. The results show that the bispecific antibodies can bind to the CD40 on the surfaces of human and cynomolgus monkey cell membranes. See FIGs. 9A, 9B and 11 and Table 21 for details.

**Table 21. The EC₅₀ values for the binding of the bispecific antibodies to the human cell surface CD40 antigen**

| Antibody | Human CD40 | | Cynomolgus monkey CD40 | |
|---|---|---|---|---|
| | EC₅₀ (nM) | Emax (MFI) | EC₅₀ (nM) | Emax (MFI) |
| Ab10-A12V2-2 | 2.40 | 7570 | 2.76 | 6614 |
| Ab10-A12V2-4 | 0.79 | 4635 | 3.24 | 6545 |
| Ab10-A297V3-2 | 1.66 | 6254 | 1.31 | 4927 |
| Ab10-A297V3-4 | 0.94 | 5085 | 0.88 | 5124 |

### 3.5. Measurement of FAP-dependent DC maturation-promoting activity of humanized anti-FAP/CD40 bispecific antibodies

To validate the effect of the anti-FAP/CD40 bispecific antibodies on dendritic cell maturation, monocytes were isolated from fresh PBMCs using CD14 positive magnetic beads and cultured in 1640 medium containing 50 ng/mL GM-CSF and 50 ng/mL hIL-4 for 5 days. A half-medium change was performed every 2-3 days. On day 5, the dendritic cells obtained by induction were added to a 96-well plate at the density of 1E5 cells/well, and CHOK1 cells in the log phase of growth and CHOK1 cells overexpressing human FAP were separately added. Meanwhile, the serially diluted test antibodies were added, with 1 µg/mL LPS as a positive control, and 100 nM hIgG 1 and an antibody-free group as negative controls. After 48 h of culture, the expression of CD83 molecules on the surfaces of the dendritic cells was measured by FACS.

The results in FIG. 11 (in the absence of CHOK1/FAP) and FIG. 12 (in the presence of CHOK1/FAP) show that the divalent molecules Ab10-A12V2-2 and Ab10-A297V3-2 cannot activate DCs in the absence of CHOK1/FAP: their CD40 agonist activity is completely dependent on FAP-mediated crosslinking. However, the tetravalent molecules Ab10-A12V2-4 and Ab10-A297V3-4 can induce DC cell maturation in the absence of CHOK1/FAP cells, and the activation of DC cells can be further enhanced in the presence of CHOK1/FAP cells. Thus, the FAP expressed on the surfaces of dendritic cells can provide a window of activation for the bispecific antibodies, whether divalent or tetravalent.

### Example 4. In-Vivo Efficacy of Anti-FAP/CD40 Bispecific Antibodies Ab10-A297V3-2/4 in Mice

B-hCD40 humanized mice were from Biocytogen Jiangsu Co., Ltd. (species: Mus muse µLus; strain: C57BL/6; female). Mouse colorectal carcinoma MC38 cells were transfected with full-length mouse FAP plasmids to construct a stably transfected strain. Cells of the strain were subcutaneously inoculated into the right axillae of the mice at 5 × 10⁵ cells/0.1 mL/mouse. When the mean tumor volume grew to 80-100 mm³, mice with proper individual tumor volume were selected and randomized into groups of 6. Mice were intraperitoneally injected twice a week. 48 h after the first administration, peripheral blood samples were taken and assayed for B cell counts and cell surface CD86 expression. On day 8 after the grouping, the concentrations of alanine aminotransferase (ALT) and aspartate aminotransferase (AST) were measured. The administration was stopped after four administrations. When there were mice with a tumor volume of more than 2000 mm³ in the hIgG1 control group, the last administration was performed. 24 h after the last administration, a complete blood count was performed.

### 4.1. Single-dose in-vivo efficacy and toxicity of Ab10-A297V3-2 or Ab10-A297V3-4 in mice

The anti-tumor activity of Ab10-A297V3-2 or Ab10-A297V3-4 was compared with that of the CD40 agonist mAb 9E5-mIgG1 under single-dose conditions. 9E5-mIgG1 and 9E5-SELFNS have the same variable region but use the mouse mIgG1 heavy chain constant region and the mouse kappa chain constant region as their heavy and light chain constant regions. As the activity of CD40 agonist mAbs is dependent on the FcyRIIb's crosslinking of their Fc, 9E5-mIgG1, which uses the mouse IgG1 heavy chain constant region, can better exhibit CD40 agonist activity in mice. In the experiment on *in-vivo* efficacy in mice, 9E5-mIgG1 was a better control antibody than 9E5-SELFNS. Ab10-A297V3-4 has stronger anti-tumor activity than the CD40 mAb 9E5-mIgG1 at the same molar dose in terms of whether TGI (tumor growth inhibition) or CR (complete response). The sequences of 9E5-mIgG1 are as follows:
> The heavy chain of 9E5-mIgG1 
QVQLVQSGAEVKKPGASVKVSCKASGYILTTYWITWVRQAPGQGLEWMGDIH
PGSGSTKYNEKFKSRVTLTVDTSISTAYMELSRLRSEDTAVYYCARRDYWGQGT
TVTVSSAKTTPPSVYPLAPGSAAQTNSMVTLGCLVKGYFPEPVTVTWNSGSLSS
GVHTFPAVLQSDLYTLSSSVTVPSSTWPSETVTCNVAHPASSTKVDKKIVPRDCG
CKPCICTVPEVSSVFIFPPKPKDVLTITLTPKVTCVVVDISKDDPEVQFSWFVDDV
EVHTAQTQPREEQFNSTFRSVSELPIMHQDWLNGKEFKCRVNSAAFPAPIEKTIS
KTKGRPKAPQVYTIPPPKEQMAKDKVSLTCMITDFFPEDITVEWQWNGQPAEN
YKNTQPIMDTDGSYFVYSKLNVQKSNWEAGNTFTCSVLHEGLHNHHTEKSLSH
SPGK
(SEQ ID NO: 64)
> The light chain of 9E5-mIgG1

DIVMTQSPLSLPVTPGEPASISCRSSQNIVNSQGNTYLEWYLQKPGQSPQLLIYK
VTNRFSGVPDRFSGSGSGTDFTLKISRVEAEDVGVYYCFQASLVPWTFGGGTKV
EIKRADAAPTVSIFPPSSEQLTSGGASVVCFLNNFYPKDINVKWKIDGSERQNGV
LNSWTDQDSKDSTYSMSSTLTLTKDEYERHNSYTCEATHKTSTSPIVKSFNRNEC
(SEQ ID NO: 65)

The anti-tumor activity of Ab10-A297V3-2 in a two-fold molar concentration is still weaker than that of Ab10-A297V3-4 but is similar to that of 9E5-mIgG1.

The activation of peripheral CD40 by the bispecific antibody molecules was measured by measuring the activation of mouse peripheral blood B lymphocytes. The results agree with the *in-vitro* experiment. Ab10-A297V3-4 was able to partially activate peripheral B lymphocytes due to its CD40 activation activity partially independent of FAP, while Ab10-A297V3-2 did not cause detectably significant peripheral B cell activation (One-Way ANOVA) because its CD40 activation is completely dependent on FAP. Information about the tested antibodies is shown in Table 22. The *in-vivo* efficacy results are shown in FIG. 13A (P < 0.0001 for all the antibody groups relative to the hIgG 1 control group) and FIG. 13B.

**Table 22. Information about the tested antibodies**

| Drug | Concentration | Molecular weight (kDa) | Dose |
|---|---|---|---|
| hIgG 1 | 14.17 mg/ mL | 150 | 3 mg/kg |
| Ab10-A297V3-2 | 2.95 mg/ mL | 174 | 6.96 mg/kg |
| Ab10-A297V3-4 | 3 mg/ mL | 200.03 | 4 mg/kg |
| 9E5-mIgG1 | 4.4 mg/ mL | 150 | 3 mg/kg |

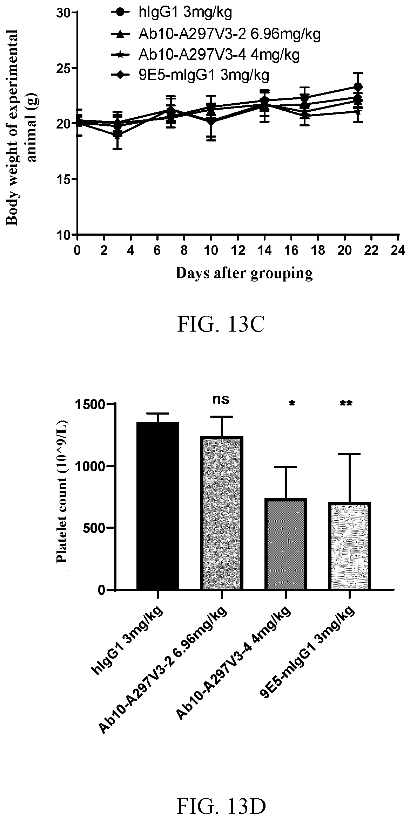

In terms of mouse toxicity, neither Ab10-A297V3-2 nor Ab10-A297V3-4 caused changes in mouse body weight. See FIG. 13C.

The complete blood count results show that compared to the hIgG1 control group at the same molar dose, 9E5-mIgG1 caused a certain platelet reduction, similar to hepatotoxicity; Ab10-A297V3-2 did not cause platelet reductions; Ab10-A297V3-4 caused the same platelet reduction as 9E5-mIgG1. See FIG. 13D. The dose of Ab10-A297V3-2/4 administered to mice and the statistics about tumor growth inhibition rates are detailed in Table 23.

The formula for calculating TGI is: TGI = (day's tumor volume of blank group - day's tumor volume of administration group) / (day's tumor volume of blank group) × 100%

**Table 23. The dose of Ab10-A297V3-2 or 4 administered to mice and the tumor growth inhibition rates**

| Antibody No. | Dose (mpk) | TGI |
|---|---|---|
| hIgG1 | 3 | - |
| Ab10-A297V3-2 | 6.96 (two-fold molar dose) | 54.6% |
| Ab10-A297V3-4 | 4 (equimolar dose) | 96.4% |
| 9E5-mIgG1 | 3 | 59.2% |

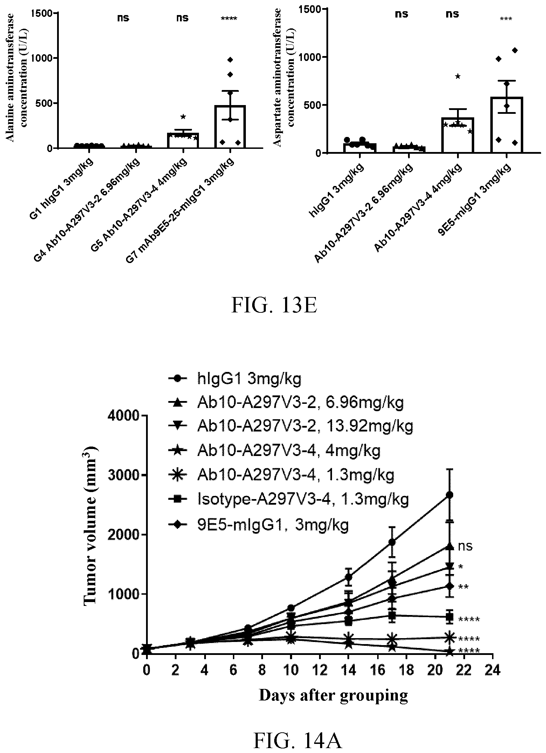

As can be seen from the ALT and AST results, 9E5-mIgG1 showed some hepatotoxicity on day 8 post-dose through CD32B (i.e., FcγRIIB)-mediated crosslinking. Ab10-A297V3-4 has some hepatotoxicity due to a certain level of background activation, but because its activation is independent of CD32B, the hepatotoxicity is lower than that of 9E5-mIgG1. Ab10-A297V3-2 does not have hepatotoxicity at all because its CD40 activation is dependent on FAP. See FIG. 13E.

### 4.2. Multiple-dose in-vivo efficacy and toxicity of Ab10-A297V3-2 or Ab10-A297V3-4 in mice

To explore the therapeutic window of Ab10-A297V3-2 or 4, the dose of Ab10-A297V3-2 was further increased, and the dose of the molecule Ab10-A297V3-4 was decreased. See Table 24 for details. When the dose of the divalent molecule Ab10-A297V3-2 was further increased, its anti-tumor activity was not further improved, and peripheral B lymphocyte activation was not observed either. When the dose of Ab10-A297V3-4 was reduced to 1.3 mg/kg (i.e., the 1/3 molar dose of 9E5-mIgG1), it still had a TGI of 92.6%, which was far better than that of 9E5-mIgG1. To assess the role of the FAP antibody in the tetravalent CD40 bispecific antibody in *in-vivo* experiments, the anti-tumor activity of Ab 10-A297V3-4 was compared with that of another tetravalent CD40 bispecific antibody without FAP binding function (isotype-A297V3-4). Isotype-A297V3-4 is identical to Ab10-A297V3-4 in structural terms, except that it contains another isotype antibody that did not bind to any murine proteins instead of the anti-FAP moiety Ab10.

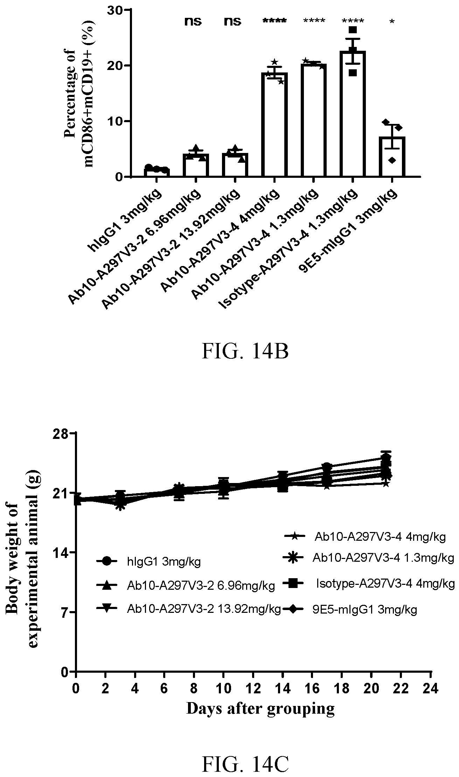

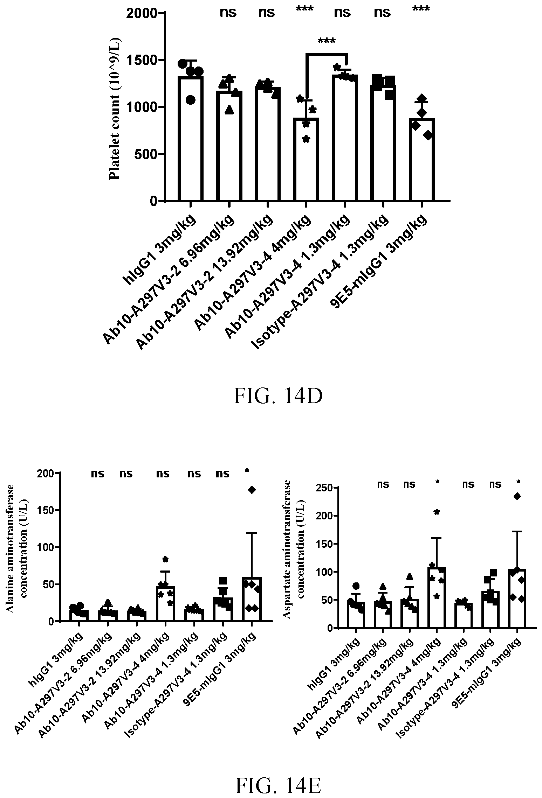

The results in FIGs. 14A-14E show that the tetravalent CD40 bispecific antibody can exhibit relatively good anti-tumor activity even without binding to FAP; furthermore, its anti-tumor activity can be stronger if it binds to FAP. As for toxicity, the increased dose of Ab10-A297V3-2 still did not cause abnormal body weight, ALT/AST levels or platelet counts, and the reduced dose of Ab10-A297V3-4 did not cause the abnormal ALT/AST levels or platelet counts which were observed with a higher dose previously, while still maintaining a very strong anti-tumor effect (P < 0.0001). This indicates that both Ab10-A297V3-2 and Ab10-A297V3-4 have wider therapeutic windows than the CD40 mAb 9E5-mIgG1. Information about the antibodies is shown in Table 24. The statistics about the multiple-dose *in-vivo* efficacy of Ab10-A297V3-2 or Ab10-A297V3-4 are shown in Table 25.

**Table 24. Information about the tested antibodies**

| Drug | Concentration | Molecular weight (kDa) | Dose |
|---|---|---|---|
| hIgG1 | 14.17 mg/ mL | 150 | 3 mg/kg |
| Ab10-A297V3-2 | 2 mg/ mL | 174 | 6.96 mg/kg |
| Ab10-A297V3-2 | | | 13.92 mg/kg |
| Ab10-A297V3-4 | 2 mg/ mL | 200.03 | 4 mg/kg |
| Ab10-A297V3-4 | | | 1.3 mg/kg |
| Isotype-A297V3-4 | 3.44 mg/ mL | 200.03 | 1.3 mg/kg |
| 9E5-mIgG1 | 4.79 mg/ mL | 150 | 3 mg/kg |

**Table 25. The statistics about the multiple-dose in-vivo efficacy of Ab10-A297V3-2/4**

| Drug | Dose (mg/kg) | TGI | CR ratio (<100 mm³) |
|---|---|---|---|
| hIgG 1 | 3 | - | 0% |
| Ab10-A297V3-2 | 6.96 (two-fold molar dose) | 32.8% | 0% |
| Ab10-A297V3-2 | 13.92 (four-fold molar dose) | 46.9% | 0% |
| Ab10-A297V3-4 | 4 (equimolar dose) | 101.6% | 100% |
| Ab10-A297V3-4 | 1.3 (1/3 molar dose) | 92.6% | 33% |
| Isotype-A297V3-4 | 1.3 (1/3 molar dose) | 79.3% | 0% |
| 9E5-mIgG1 | 3 | 59.2% | 0% |

### 4.3. Multiple-dose in-vivo efficacy and toxicity of Ab10-A12V2-2 in mice

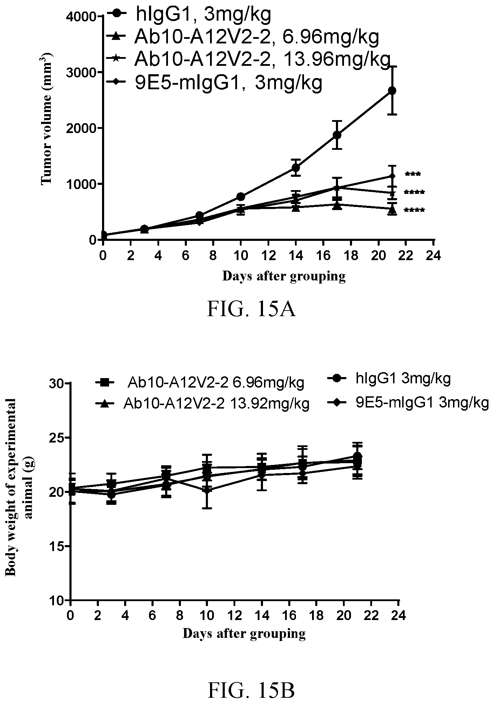

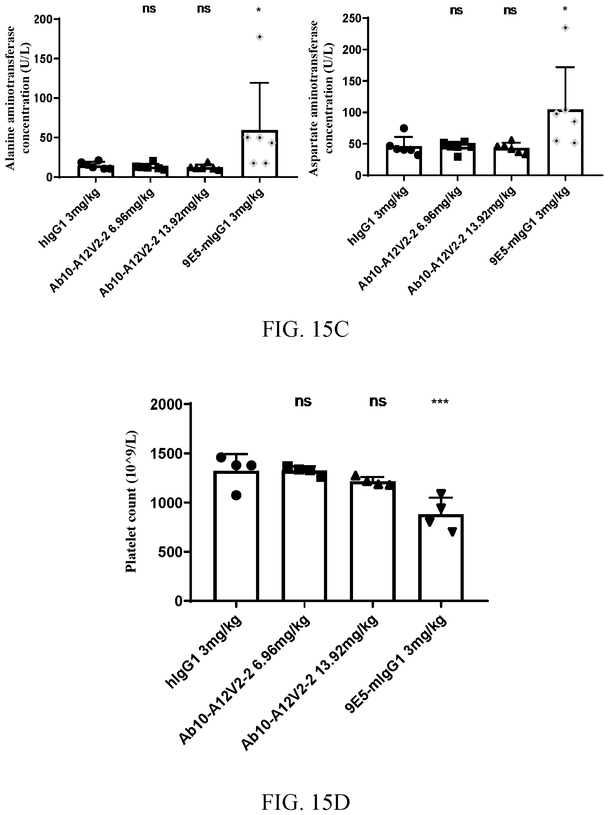

See FIGs. 15A-15D and Table 26. Ab10-A12V2-2 has similar pharmacological characteristics to Ab10-A297V3-2 in mice (P < 0.0001 for both doses compared to the hIgG1 control group) and is superior in anti-tumor activity to 9E5-mIgG1. The anti-tumor activity cannot be further improved by increasing the dose, but no ALT/AST increase or platelet reduction was caused. This suggests a wider therapeutic window and lower side effects.

**Table 26. Information about the tested antibodies and TGI**

| Drug | Concentration | Molecular weight (kDa) | Dose | TGI |
|---|---|---|---|---|
| hIgG1 | 14.17 mg/ mL | 150 | 3 mg/kg | - |
| Ab10-A12V2-2 | 2 mg/ mL | 174 | 6.96 mg/kg | 81.8% |
| Ab10-A12V2-2 | | | 13.92 mg/kg | 70.9% |
| 9E5-mIgG1 | 4.79 mg/ mL | 150 | 3 mg/kg | 59.2% |

### 4.4. Single-dose in-vivo efficacy and toxicity of Ab10-A12V2-4 in mice

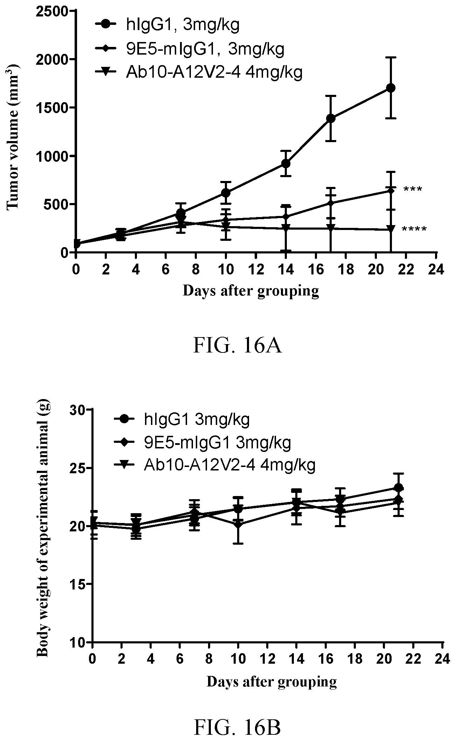

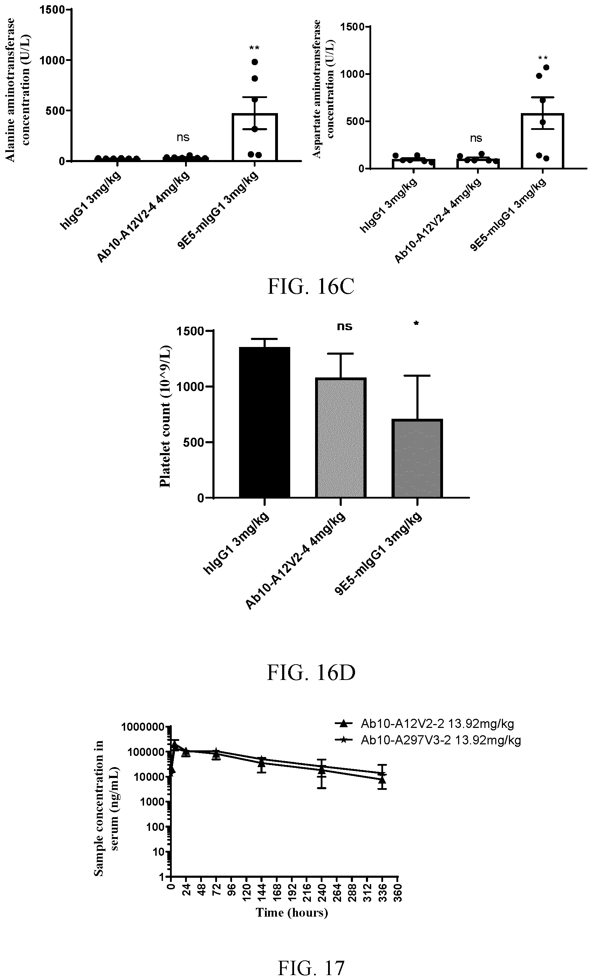

Similarly, this example explores the single-dose efficacy and toxicity of Ab10-A12V2-4 in mice. Compared to the hIgG1 control group, the Ab10-A12V2-4 group (P < 0.0001) is superior in anti-tumor activity to 9E5-mIgG1 and did not cause ALT/AST increases or platelet reductions. See Table 27 and FIGs. 16A-16D.

**Table 27. Information about the tested antibodies and TGI**

| Drug | Concentration | Molecular weight (kDa) | Dose | TGI |
|---|---|---|---|---|
| hIgG1 | 14.17 mg/ mL | 150 | 3 mg/kg | - |
| Ab10-A12V2-4 | 3 mg/ mL | 200.03 | 4 mg/kg | 88.3% |
| 9E5-mIgG1 | 4.4 mg/ mL | 150 | 3 mg/kg | 59.2% |

### 4.5. Measurement of pharmacokinetics of anti-FAP/CD40 bispecific antibodies

The present disclosure compares the pharmacokinetic characteristics of anti-FAP/CD40 bispecific antibodies in mFAP-MC38 tumor-bearing hCD40 humanized mice from three different perspectives. The antibodies were administered by intravenous injection in different concentrations, and then blood samples were taken at different time points. The plasma concentration/time curve of each of the antibody molecules was analyzed by measuring the human Fc content in mouse blood.

An anti-human IgG-Fc antibody was diluted to 1.5 µg/mL with PBS and used to coat a plate at 100 µL/well, and the plate was left overnight at 4 °C. The solution was removed, and the plate was washed with PBST (200 µL/well) three times. After PBST was removed, the plate was blocked with 1% BSA (100 µL/well) at 37 °C for 2 h. The solution was removed. After the plate was washed with PBST (200 µL/well) three times, the diluted antibodies or serum samples (all were diluted with 1% BSAPBS; serum was diluted 10-fold, 100-fold, 1000-fold and 10,000-fold) were added at 50 µL/well, and the plate was incubated at 37 °C for 1 h. The solution was removed. After the plate was washed with PBST (200 µL/well) three times, an HRP-labeled goat anti-human IgG-Fc secondary antibody (diluted with 1% BSAPBS, 1:20,000) was added at 50 µL/well, and the plate was incubated at 37 °C for 20 min. The solution was removed. After the plate was washed with PBST (200 µL/well) five times, TMB was added at 50 µL/well for color development. After gradient color reactions of the antibodies were observed, ELISA stop solution was added at 50 µL/well. OD450 values were measured on Envision. After serum OD450 readings that were within the linear range of the antibody standard curve were converted into concentrations, PK curves were plotted and analyzed.

The results are shown in FIG. 17, which compares the pharmacokinetic characteristics of the molecules Ab10-A297V3-2 and Ab10-A12V2-2 at a dose of 13.92 mg/kg. The results show that the two molecules have similar pharmacokinetic characteristics. Thus, the pharmacokinetic characteristics of the bispecific antibodies of the present disclosure do not vary with the different sequences of the CD40 antibodies.

## Claims

1. A FAP/CD40-binding molecule, comprising a first antigen-binding domain that specifically binds to FAP and a second antigen-binding domain that specifically binds to CD40, wherein the first antigen-binding domain that specifically binds to FAP comprises a heavy chain variable region and a light chain variable region; the heavy chain variable region comprises a HCDR1, a HCDR2 and a HCDR3, and the light chain variable region comprises a LCDR1, a LCDR2 and a LCDR3, wherein:
the HCDR1, HCDR2 and HCDR3 comprise the amino acid sequences set forth in SEQ ID NOs: 9, 10 and 18, respectively; the LCDR1, LCDR2 and LCDR3 comprise the amino acid sequences set forth in SEQ ID NOs: 12, 13 and 19, respectively;
preferably,
the HCDR1, HCDR2 and HCDR3 comprise the amino acid sequences set forth in SEQ ID NOs: 9, 10 and 15, respectively; the LCDR1, LCDR2 and LCDR3 comprise the amino acid sequences set forth in SEQ ID NOs: 12, 13 and 14, respectively;
the HCDR1, HCDR2 and HCDR3 comprise the amino acid sequences set forth in SEQ ID NOs: 9, 10 and 11, respectively; the LCDR1, LCDR2 and LCDR3 comprise the amino acid sequences set forth in SEQ ID NOs: 12, 13 and 14, respectively;
the HCDR1, HCDR2 and HCDR3 comprise the amino acid sequences set forth in SEQ ID NOs: 9, 10 and 15, respectively; the LCDR1, LCDR2 and LCDR3 comprise the amino acid sequences set forth in SEQ ID NOs: 12, 13 and 16, respectively; or
the HCDR1, HCDR2 and HCDR3 comprise the amino acid sequences set forth in SEQ ID NOs: 9, 10 and 11, respectively; the LCDR1, LCDR2 and LCDR3 comprise the amino acid sequences set forth in SEQ ID NOs: 12, 13 and 17, respectively.

2. The FAP/CD40-binding molecule according to claim 1, wherein the second antigen-binding domain that specifically binds to CD40 comprises at least one immunoglobulin single variable domain, and the immunoglobulin single variable domain comprises three complementarity-determining regions CDR1, CDR2 and CDR3, wherein the CDR1, CDR2 and CDR3 comprise the amino acid sequences set forth in SEQ ID NOs: 39, 40 and 41, respectively;
preferably, the CDR1, CDR2 and CDR3 comprise the amino acid sequences set forth in SEQ ID NOs: 24, 25 and 26, respectively; or the CDR1, CDR2 and CDR3 comprise the amino acid sequences set forth in SEQ ID NOs: 27, 28 and 29, respectively.

3. The FAP/CD40-binding molecule according to claim 1 or 2, wherein in the first antigen-binding domain that specifically binds to FAP:
the heavy chain variable region comprises the amino acid sequence set forth in SEQ ID NO: 3 or an amino acid sequence having at least 90% identity thereto, and the light chain variable region comprises the amino acid sequence set forth in SEQ ID NO: 4 or an amino acid sequence having at least 90% identity thereto;
the heavy chain variable region comprises the amino acid sequence set forth in SEQ ID NO: 1 or an amino acid sequence having at least 90% identity thereto, and the light chain variable region comprises the amino acid sequence set forth in SEQ ID NO: 2 or an amino acid sequence having at least 90% identity thereto;
the heavy chain variable region comprises the amino acid sequence set forth in SEQ ID NO: 5 or an amino acid sequence having at least 90% identity thereto, and the light chain variable region comprises the amino acid sequence set forth in SEQ ID NO: 6 or an amino acid sequence having at least 90% identity thereto; or
the heavy chain variable region comprises the amino acid sequence set forth in SEQ ID NO: 7 or an amino acid sequence having at least 90% identity thereto, and the light chain variable region comprises the amino acid sequence set forth in SEQ ID NO: 8 or an amino acid sequence having at least 90% identity thereto.

4. The FAP/CD40-binding molecule according to claim 2 or 3, wherein the immunoglobulin single variable domain in the second antigen-binding domain that specifically binds to CD40 comprises any of the amino acid sequences set forth in SEQ ID NOs: 22, 23, and 32 to 38 or an amino acid sequence having at least 90% sequence identity to any of SEQ ID NOs: 22, 23, and 32 to 38.

5. The FAP/CD40-binding molecule according to any of claims 2 to 4, wherein the second antigen-binding domain that specifically binds to CD40 comprises 2, 3, 4, 5 or 6 said immunoglobulin single variable domains.

6. The FAP/CD40-binding molecule according to any of claims 2 to 5, wherein the first antigen-binding domain that specifically binds to FAP comprises a heavy chain variable region and a light chain variable region, wherein:
the immunoglobulin single variable domain of the second antigen-binding domain that specifically binds to CD40 is located at the N-terminus of the heavy chain variable region of the first antigen-binding domain that specifically binds to FAP;
the immunoglobulin single variable domain of the second antigen-binding domain that specifically binds to CD40 is located at the C-terminus of the heavy chain variable region of the first antigen-binding domain that specifically binds to FAP;
the immunoglobulin single variable domain of the second antigen-binding domain that specifically binds to CD40 is located at the N-terminus of the light chain variable region of the first antigen-binding domain that specifically binds to FAP; and/or
the immunoglobulin single variable domain of the second antigen-binding domain that specifically binds to CD40 is located at the C-terminus of the light chain variable region of the first antigen-binding domain that specifically binds to FAP.

7. The FAP/CD40-binding molecule according to any of claims 2 to 6, wherein the immunoglobulin single variable domain of the second antigen-binding domain that specifically binds to CD40 is linked, either directly or by a linker, to the first antigen-binding domain that specifically binds to FAP;
preferably, the linker is an amino acid sequence represented by (G₄S)ₓ, wherein x is independently selected from an integer from 1 to 20;
more preferably, the linker is an amino acid sequence represented by (G₄S)₂, (G₄S)₃ or (G₄S)₄.

8. The FAP/CD40-binding molecule according to any of claims 1 to 7, further comprising a human immunoglobulin Fc region, wherein:
preferably, the Fc region is the Fc region of human IgG1 or IgG4;
more preferably, the human IgG1 has a mutation that removes or reduces Fc effector function;
most preferably, the human IgG1 has a mutation selected from the group consisting of N297A, D265A/N297A, L234A/L235A, L234A/L235A/P329G, L234E, L234F, L234E/L235F and L234E/L235F/P329G.

9. The FAP/CD40-binding molecule according to any of claims 1 to 8, wherein the first antigen-binding domain that specifically binds to FAP comprises a heavy chain and a light chain, wherein:
preferably, the heavy chain is of the IgG1 or IgG4 isotype, and the light chain is of the Kappa isotype;
more preferably, the heavy chain is the amino acid sequence set forth in SEQ ID NO: 51 or an amino acid sequence having at least 90% identity thereto, and the light chain is the amino acid sequence set forth in SEQ ID NO: 52 or an amino acid sequence having at least 90% identity thereto;
the heavy chain is the amino acid sequence set forth in SEQ ID NO: 49 or an amino acid sequence having at least 90% identity thereto, and the light chain is the amino acid sequence set forth in SEQ ID NO: 50 or an amino acid sequence having at least 90% identity thereto;
the heavy chain is the amino acid sequence set forth in SEQ ID NO: 53 or an amino acid sequence having at least 90% identity thereto, and the light chain is the amino acid sequence set forth in SEQ ID NO: 54 or an amino acid sequence having at least 90% identity thereto; or
the heavy chain is the amino acid sequence set forth in SEQ ID NO: 55 or an amino acid sequence having at least 90% identity thereto, and the light chain is the amino acid sequence set forth in SEQ ID NO: 56 or an amino acid sequence having at least 90% identity thereto.

10. The FAP/CD40-binding molecule according to any of claims 1 to 9, comprising a first polypeptide chain and a second polypeptide chain, wherein:
the first polypeptide chain comprises any of the amino acid sequences set forth in SEQ ID NOs: 42 and 44-48 or an amino acid sequence having at least 90% identity thereto, and the second polypeptide chain comprises the amino acid sequence set forth in SEQ ID NO: 43 or an amino acid sequence having at least 90% identity thereto;
preferably, the FAP/CD40-binding molecule comprises two identical first polypeptide chains and two identical second polypeptide chains.

11. A FAP-binding molecule, comprising a heavy chain variable region and a light chain variable region, wherein the heavy chain variable region comprises a HCDR1, a HCDR2 and a HCDR3, and the light chain variable region comprises a LCDR1, a LCDR2 and a LCDR3, wherein:
the HCDR1, HCDR2 and HCDR3 comprise the amino acid sequences set forth in SEQ ID NOs: 9, 10 and 18, respectively; the LCDR1, LCDR2 and LCDR3 comprise the amino acid sequences set forth in SEQ ID NOs: 12, 13 and 19, respectively;
preferably,
the HCDR1, HCDR2 and HCDR3 comprise the amino acid sequences set forth in SEQ ID NOs: 9, 10 and 15, respectively; the LCDR1, LCDR2 and LCDR3 comprise the amino acid sequences set forth in SEQ ID NOs: 12, 13 and 14, respectively;
the HCDR1, HCDR2 and HCDR3 comprise the amino acid sequences set forth in SEQ ID NOs: 9, 10 and 11, respectively; the LCDR1, LCDR2 and LCDR3 comprise the amino acid sequences set forth in SEQ ID NOs: 12, 13 and 14, respectively;
the HCDR1, HCDR2 and HCDR3 comprise the amino acid sequences set forth in SEQ ID NOs: 9, 10 and 15, respectively; the LCDR1, LCDR2 and LCDR3 comprise the amino acid sequences set forth in SEQ ID NOs: 12, 13 and 16, respectively; or
the HCDR1, HCDR2 and HCDR3 comprise the amino acid sequences set forth in SEQ ID NOs: 9, 10 and 11, respectively; the LCDR1, LCDR2 and LCDR3 comprise the amino acid sequences set forth in SEQ ID NOs: 12, 13 and 17, respectively.

12. The FAP-binding molecule according to claim 11, wherein:
the heavy chain variable region comprises the amino acid sequence set forth in SEQ ID NO: 3 or an amino acid sequence having at least 90% identity thereto, and the light chain variable region comprises the amino acid sequence set forth in SEQ ID NO: 4 or an amino acid sequence having at least 90% identity thereto;
the heavy chain variable region comprises the amino acid sequence set forth in SEQ ID NO: 1 or an amino acid sequence having at least 90% identity thereto, and the light chain variable region comprises the amino acid sequence set forth in SEQ ID NO: 2 or an amino acid sequence having at least 90% identity thereto;
the heavy chain variable region comprises the amino acid sequence set forth in SEQ ID NO: 5 or an amino acid sequence having at least 90% identity thereto, and the light chain variable region comprises the amino acid sequence set forth in SEQ ID NO: 6 or an amino acid sequence having at least 90% identity thereto; or
the heavy chain variable region comprises the amino acid sequence set forth in SEQ ID NO: 7 or an amino acid sequence having at least 90% identity thereto, and the light chain variable region comprises the amino acid sequence set forth in SEQ ID NO: 8 or an amino acid sequence having at least 90% identity thereto.

13. The FAP-binding molecule according to claim 11 or 12, further comprising a human immunoglobulin Fc region, wherein:
preferably, the Fc region is the Fc region of human IgG1 or IgG4;
more preferably, the human IgG1 has a mutation that removes or reduces Fc effector function;
most preferably, the human IgG1 has a mutation selected from the group consisting of N297A, D265A/N297A, L234A/L235A, L234A/L235A/P329G, L234E, L234F, L234E/L235F and L234E/L235F/P329G.

14. The FAP-binding molecule according to any of claims 11 to 13, comprising a heavy chain and a light chain, wherein:
the heavy chain is set forth in SEQ ID NO: 51 or an amino acid sequence having at least 90% identity thereto, and the light chain is set forth in SEQ ID NO: 52 or an amino acid sequence having at least 90% identity thereto;
the heavy chain is set forth in SEQ ID NO: 49 or an amino acid sequence having at least 90% identity thereto, and the light chain is set forth in SEQ ID NO: 50 or an amino acid sequence having at least 90% identity thereto;
the heavy chain is set forth in SEQ ID NO: 53 or an amino acid sequence having at least 90% identity thereto, and the light chain is set forth in SEQ ID NO: 54 or an amino acid sequence having at least 90% identity thereto; or
the heavy chain is set forth in SEQ ID NO: 55 or an amino acid sequence having at least 90% identity thereto, and the light chain is set forth in SEQ ID NO: 56 or an amino acid sequence having at least 90% identity thereto.

15. The FAP-binding molecule according to any of claims 11 to 14, being an anti-FAP antibody or an antigen-binding fragment thereof, wherein:
preferably, the anti-FAP antibody or the antigen-binding fragment thereof is selected from the group consisting of bi- or multi-specific antibodies, scFv, scFv dimers, dsFv, (dsFv)2, dsFv-dsFv', Fv fragments, Fab, Fab' and F(ab')2;
preferably, the anti-FAP antibody or the antigen-binding fragment thereof is a chimeric antibody, a humanized antibody or a fully human antibody, or an antigen-binding fragment thereof.

16. A CD40-binding molecule, comprising an immunoglobulin single variable domain that binds to CD40, wherein the immunoglobulin single variable domain comprises three complementarity-determining regions CDR1, CDR2 and CDR3, and the CDR1, CDR2 and CDR3 comprise the amino acid sequences set forth in SEQ ID NOs: 39, 40 and 41, respectively;
preferably, the CDR1, CDR2 and CDR3 comprise the amino acid sequences set forth in SEQ ID NOs: 24, 25 and 26, respectively; or the CDR1, CDR2 and CDR3 comprise the amino acid sequences set forth in SEQ ID NOs: 27, 28 and 29, respectively.

17. The CD40-binding molecule according to claim 16, comprising any of the amino acid sequences set forth in SEQ ID NOs: 22, 23, and 32 to 38 or an amino acid sequence having at least 90% identity thereto.

18. The CD40-binding molecule according to claim 16 or 17, further comprising a human immunoglobulin Fc region, wherein:
preferably, the Fc region is the Fc region of human IgG1 or IgG4;
more preferably, the Fc region of human IgG1 has a mutation that increases affinity for FcyRIIb and/or reduces ADCC effects;
most preferably, the Fc region of human IgG1 has a mutation selected from the group consisting of S267E/L328F, L234A/L235A, N297A, L234F, L235E, L234F/L235E/D265A, L234A/L235A/G237A/P238S/H268A/A330S/P331S, and E233D/G237D/P238D/H268D/P271G/A330R.

19. The CD40-binding molecule according to any of claims 16 to 18, comprising any of the amino acid sequences set forth in SEQ ID NOs: 57 to 63 or an amino acid sequence having at least 90% identity thereto.

20. The CD40-binding molecule according to any of claims 16 to 19, being an anti-CD40 antibody or an antigen-binding fragment thereof, wherein:
preferably, the anti-CD40 antibody or the antigen-binding fragment thereof is selected from the group consisting of linear antibodies, single-chain antibodies, nanobodies, peptibodies, domain antibodies, and multispecific antibodies;
preferably, the anti-CD40 antibody or the antigen-binding fragment thereof is a camelid antibody, a chimeric antibody, a humanized antibody or a fully human antibody, or an antigen-binding fragment thereof.

21. A FAP/CD40-binding molecule, comprising a first antigen-binding domain that specifically binds to FAP and a second antigen-binding domain that specifically binds to CD40, wherein the second antigen-binding domain that specifically binds to CD40 comprises at least one immunoglobulin single variable domain, and the immunoglobulin single variable domain comprises three complementarity-determining regions CDR1, CDR2 and CDR3, wherein the CDR1, CDR2 and CDR3 comprise the amino acid sequences set forth in SEQ ID NOs: 39, 40 and 41, respectively;
preferably, the CDR1, CDR2 and CDR3 comprise the amino acid sequences set forth in SEQ ID NOs: 24, 25 and 26, respectively; or the CDR1, CDR2 and CDR3 comprise the amino acid sequences set forth in SEQ ID NOs: 27, 28 and 29, respectively.

22. The FAP/CD40-binding molecule according to claim 21, wherein the immunoglobulin single variable domain in the second antigen-binding domain that specifically binds to CD40 comprises any of the amino acid sequences set forth in SEQ ID NOs: 22, 23, and 32 to 38 or an amino acid sequence having at least 90% sequence identity to any of SEQ ID NOs: 22, 23, and 32 to 38.

23. The FAP/CD40-binding molecule according to any of claims 1 to 10 and 21 to 22, being an anti-FAP/CD40 bispecific antibody.

24. A polynucleotide, encoding the FAP/CD40-binding molecule according to any of claims 1 to 10 and 21 to 23, the FAP-binding molecule according to any of claims 11 to 15, or the CD40-binding molecule according to any of claims 16 to 20.

25. A vector, comprising the polynucleotide according to claim 24.

26. A host cell, comprising or expressing the polynucleotide according to claim 24 or the vector according to claim 25.

27. A pharmaceutical composition, comprising the FAP/CD40-binding molecule according to any of claims 1 to 10 and 21 to 23, the FAP-binding molecule according to any of claims 11 to 15, or the CD40-binding molecule according to any of claims 16 to 20, and at least one pharmaceutically acceptable excipient, diluent or carrier.

28. Use of the FAP/CD40-binding molecule according to any of claims 1 to 10 and 21 to 23, the FAP-binding molecule according to any of claims 11 to 15, the CD40-binding molecule according to any of claims 16 to 20, the polynucleotide according to claim 24, or the pharmaceutical composition according to claim 27 in the preparation of a medicament for treating or ameliorating a disease or condition, wherein:
the disease or condition is preferably a tumor or cancer;
the disease or condition is more preferably lung cancer, prostate cancer, breast cancer, head and neck cancer, esophageal cancer, gastric cancer, colorectal cancer, bladder cancer, cervical cancer, uterine cancer, ovarian cancer, liver cancer, melanoma, kidney cancer, squamous cell cancer, or hematological cancer.

29. A method for treating or ameliorating a tumor or cancer, wherein the method comprises:
administering to a subject in need thereof a therapeutically effective amount of the FAP/CD40-binding molecule according to any of claims 1 to 10 and 21 to 23, the FAP-binding molecule according to any of claims 11 to 15, the CD40-binding molecule according to any of claims 16 to 20, the polynucleotide according to claim 24, or the pharmaceutical composition according to claim 27;
the tumor or cancer is preferably lung cancer, prostate cancer, breast cancer, head and neck cancer, esophageal cancer, gastric cancer, colorectal cancer, bladder cancer, cervical cancer, uterine cancer, ovarian cancer, liver cancer, melanoma, kidney cancer, squamous cell cancer, or hematological cancer.
